(19) Europäisches Patentamt
European Patent Office
Office européen des brevets

(11) EP 4 755 402 A1

(12) EUROPEAN PATENT APPLICATION
published in accordance with Art. 153(4) EPC

(43) Date of publication:
**10.06.2026 Bulletin 2026/24**

(21) Application number: **24848293.7**

(22) Date of filing: **31.07.2024**

(51) International Patent Classification (IPC):
***A61K 47/68*** (2017.01) ***A61K 47/65*** (2017.01)
***C07K 16/00*** (2006.01) ***C07K 16/28*** (2006.01)
***C07K 16/30*** (2006.01) ***A61K 39/395*** (2006.01)
***A61P 35/00*** (2006.01)

(52) Cooperative Patent Classification (CPC):
**A61K 39/395; A61K 47/65; A61K 47/68;
A61P 35/00; C07K 16/00; C07K 16/28; C07K 16/30**

(86) International application number:
**PCT/CN2024/108728**

(87) International publication number:
**WO 2025/026338 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 CN 202310943337**

(71) Applicant: **CSPC Megalith Biopharmaceutical Co., Ltd.**
**Shijiazhuang, Hebei 050025 (CN)**

(72) Inventors:
- **HUI, Xiwu**
  **Shijiazhuang, Hebei 050025 (CN)**
- **SHEN, Mingyue**
  **Shijiazhuang, Hebei 050025 (CN)**
- **SUN, Zhaopeng**
  **Shijiazhuang, Hebei 050025 (CN)**
- **DAN, Mo**
  **Shijiazhuang, Hebei 050025 (CN)**
- **ZHANG, Di**
  **Shijiazhuang, Hebei 050025 (CN)**
- **YUAN, Can**
  **Shijiazhuang, Hebei 050025 (CN)**
- **SUN, Yufei**
  **Shijiazhuang, Hebei 050025 (CN)**
- **XIE, Ru**
  **Shijiazhuang, Hebei 050025 (CN)**
- **WANG, Pengfei**
  **Shijiazhuang, Hebei 050025 (CN)**
- **YAO, Bing**
  **Shijiazhuang, Hebei 050025 (CN)**

(74) Representative: **Lavoix**
**Bayerstraße 83**
**80335 München (DE)**

Remarks:
The complete document including Reference Table(s) and the Sequence Listing(s) can be downloaded from the EPO website

# (54) ANTIBODY-DRUG CONJUGATE HAVING HIGH DRUG LOAD AND USE THEREOF

(57) An antibody-drug conjugate having a high drug load and the use thereof. The antibody-drug conjugate has a high drug-antibody ratio (DAR) and comprises an antibody and a drug conjugate moiety, wherein a constant region of the antibody comprises reactive glutamine. Also provided are the preparation method for and the use of the antibody-drug conjugate having a high drug load.

EP 4 755 402 A1

## Description

### CROSS-REFERENCE TO RELATED APPLICATIONS

The application claims priority to, and the benefit of Application No. CN202310943337.5, filed on July 31, 2023, and PCT Application No. PCT/CN2024/108728, filed on July 31, 2024, the contents of which are incorporated herein by reference in their entirety.

### TECHNICAL FIELD

This application relates to an antibody-drug conjugate with high drug load, and a preparation method and use thereof.

### BACKGROUND ART

Antibody-drug conjugates (ADCs) are composed of monoclonal antibodies targeting antigens and small molecule cytotoxic drugs connected by linkers, combining the killing effect of traditional small molecule chemotherapy with the tumor-targeting properties of antibody drugs. An ADC consists of three parts: an antibody that selectively recognizes antigens on the surface of cancer cells, a payload that kills cancer cells, and a linker that connects the antibody and the payload. After recognizing antigens on the surface of tumor cells, the ADC enters the cell via endocytosis. The linker is hydrolyzed by proteases in lysosomes, releasing the payload in a biologically active form, leading to cancer cell death. The amount of payload entering the cell is determined by the number of antigens on each cell surface, the drug-antibody ratio (DAR) of the ADC, and the time required for the antigens to return to the cell surface. The payload permeates through the cytoplasm and exerts an inhibitory effect on surrounding tumor cells; this is known as the bystander effect.

The research and development technology of ADC has been constantly updated. The research on ADC can be traced back to 1980, but it was not until 2000 that the first generation of ADC drugs, represented by Mylotarg, was successfully launched. Because the drug was not highly targeted to tumors, had a low localization rate, and used an unstable linker, it was highly toxic. Ultimately, due to severe liver damage and poor efficacy, Pfizer voluntarily withdrew the drug from the market in 2010, and it was not relaunched until 2017. Second-generation ADC drugs, represented by Kadcyla, were approved by the FDA in February 2013 for the treatment of HER2-positive breast cancer patients who had previously failed Trastuzumab and Taxane chemotherapy. Kadcyla is a HER2-targeting antibody-drug conjugate containing a humanized anti-HER2 IgG1 antibody, Trastuzumab, and a microtubule inhibitor, DM1 (a maytansine derivative), covalently connected by a stable thioether linker (MCC). Second-generation ADC drugs have improved antibody technology, enhancing their targeting of tumor cells and reducing cross-reactivity with healthy cells. However, the coupling sites of the payloads in ADCs remain nonspecific, leading to differences in pharmacokinetics between ADC drugs with different numbers of payloads. Third-generation ADC drugs are developing towards specific conjugation sites and uniform DAR values. Although no ADC has been successfully launched yet, many site-conjugated ADCs have shown advantages in clinical practice. For example, ADC drugs have reduced toxicity, no unbound monoclonal antibodies, and greatly improved stability and pharmacokinetics, thus improving efficacy.

Among the site-specific coupling methods of antibodies and small molecule drugs, enzyme-catalyzed coupling is a type of site-directed coupling that can achieve site-directed coupling of ADCs. Enzyme-catalyzed coupling refers to the use of genetic engineering technology to generate specific amino acid sequences in antibodies that can be specifically recognized by a certain type of enzyme. Then, by utilizing the specific action of this enzyme on the amino acid sequence, specific amino acid residues are modified to carry active reaction sites, thereby achieving site-directed coupling by reacting with linkers. The main enzymes include transglutaminase, glycosyltransferase, and sorting enzyme A. Microbial transglutaminase (mTGase) can also catalyze transpeptidation reactions. It mainly catalyzes the acyl transfer reaction between the amide on glutamine in proteins or polypeptides and other primary amines, and participates in the cross-linking of proteins or small molecules to form heteropeptide bonds. Therefore, mTGase can covalently couple the linker-payload to a specific glutamine (Q295) in the antibody to form an ADC drug with a specific coupling site and a DAR (drug-to-antibody ratio) of 2.0. For example, the antibody-drug conjugate DP303c prepared in Chinese patent application CN114901308A. DP303c is an antibody-drug conjugate containing a monoclonal IgG1 antibody targeting HER2 (DP001) and a cleavable LND1002 (linker-toxin) site specifically conjugated to glutamine 295 in the constant region of each heavy chain of DP001. DP303c has a DAR (drug-antibody ratio) of about 1.8 to about 2.2, such as about 1.8, 1.9, 2.0, 2.1 or 2.2.

The antibody-drug conjugate DS-8201a (trade name: Enhertu, generic name: Trastuzumab Deruxtecan), jointly developed and commercialized by Daiichi Sankyo and AstraZeneca, has shown breakthrough therapeutic potential in patients resistant to multiple HER2-ADCs, making DS-8201a a blockbuster drug in recent years. The success of DS-8201a has led to an increasing number of ADC drugs being developed towards smaller molecule toxins with lower

toxicity and higher DAR values. Currently, the DAR of ADC drugs prepared by mTGase enzymatic conjugation is 2.0, and the number of small molecule toxins that can be conjugated is limited. Currently, most high DAR value molecules utilize non-site-specific chemical coupling methods, which also present certain drawbacks in terms of molecular stability.

## SUMMARY OF THE INVENTION

In a first aspect, this application relates to antibody-drug conjugates with high drug load, comprising an antibody or an antigen-binding fragment thereof and a drug conjugated moiety, wherein a constant region of the antibody or an antigen-binding fragment thereof comprises one or more reactive glutamine, and the reactive glutamine, based on EU numbering rules, is selected from the group consisting of: (i) a glutamine in a Q-containing tag selected from the group consisting of: GGLQSGA, LQSGA, EEQYGA and EEQYQSG, which is located at the carboxyl terminus of the light chain, the heavy chain, or both the light chain and the heavy chain; (ii) a reactive glutamine obtained by amino acid modification, which is located in an amino acid sequence selected from the group consisting of: heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q; (iii) a reactive glutamine obtained by amino acid modification, which is located in an amino acid sequence selected from the group consisting of: light chain 126L-127Q-G128-129G, light chain 205L-206Q-207G-208G, light chain 109L-110Q-111G-112G and light chain 199L-200Q-201S-202G; (iv) an endogenous glutamine in the heavy chain Q295; or (v) glutamine in Q-containing tag LQSG that replaces the light chain G200-L201-S202, and glutamine in the Q-containing tag LQSG that inserts after the heavy chain T135.

In some embodiments, the antibody-drug conjugate comprises an antibody or an antigen-binding fragment thereof and a drug conjugated moiety, wherein the constant region of the antibody comprises two or more reactive glutamine, wherein the reactive glutamine, based on EU numbering rules, is a glutamine in a Q-containing tag selected from the group consisting of: GGLQSGA, LQSGA, EEQYGA, EEQYQSG and LLQGA, which is located at the carboxyl terminus of the light chain or the heavy chain; glutamine in the Q-containing tag LQSG that replaces the light chain G200-L201-S202; glutamine in the Q-containing tag LQSG that inserts after the heavy chain T135; a reactive glutamine obtained by amino acid modification, which is located in an amino acid sequence selected from the group consisting of: heavy chain S354L-R355Q-E356G (Q438), heavy chain P445Q-G446G, heavy chain H268L-E269Q-D270G, heavy chain M252Q-I253G, heavy chain S444Q-P445G-G446A, heavy chain V282Q, heavy chain E283L-V284V-H285Q-N286G, heavy chain H285-N286Q, heavy chain K340L-G341L-Q342Q-P343G, heavy chain N384L-G385Q-Q386G-P387G, heavy chain H268L-E269Q-D270G-P271G, heavy chain Q438I, heavy chain Q295A, heavy chain T109L-V110Q-A111G-A112G, and light chain K126L-S127Q-G128G-T129G; or an endogenous glutamine in the heavy chain Q295-V296-N297, wherein the drug conjugated moiety is conjugated to the antibody via the reactive glutamine site.

In the second aspect, this application relates to an engineered antibody or an antigen-binding fragment thereof, wherein the constant region of the antibody or an antigen-binding fragment thereof comprises one or more reactive glutamine, the reactive glutamine, based on EU numbering rules, is selected from the group consisting of: (i) a glutamine in a Q-containing tag selected from the group consisting of: GGLQSGA, LQSGA, EEQYGA, EEQYQSG and LLQGA, which is located at carboxyl terminus of light chain, heavy chain, or both the light chain and the heavy chain; (ii) a reactive glutamine obtained by amino acid modification, which is located in an amino acid sequence selected from the group consisting of: heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q; (iii) a reactive glutamine obtained by amino acid modification, which is located in an amino acid sequence selected from the group consisting of: light chain 126L-127Q-G128-129G, light chain 205L-206Q-207G-208G, light chain 109L-110Q-111G-112G and light chain 199L-200Q-201S-202G; (iv) an endogenous glutamine in the heavy chain Q295; or (v) glutamine in the Q-containing tag LQSG that replaces the light chain G200-L201-S202, and glutamine in the Q-containing tag LQSG that inserts after the heavy chain T135.

In the first and second aspects above:

In some embodiments, the reactive glutamine is selected from the group consisting of: (i) a glutamine in a Q-containing tag selected from the group consisting of: GGLQSGA, LQSGA, EEQYGA, EEQYQSG and LLQGA, which is located at carboxyl terminus of light chain, heavy chain, or both the light chain and the heavy chain; (ii) a reactive glutamine obtained by amino acid modification, which is located in an amino acid sequence selected from the group consisting of: heavy chain S354L-R355Q-E356G (Q438), heavy chain P445Q-G446 (also represented as P445Q-G446G), heavy chain S117L-A118Q-S119G-T120G, heavy chain H310L-Q311-D312G (also represented as H310L-Q311Q-D312G), heavy chain D399L-S400Q-D401G, heavy chain H268L-E269Q-D270G, heavy chain M252Q-I253G, heavy chain S444Q-P445G-G446A, heavy chain E283L-V284-H285Q-N286G (also represented as E283L-V284V-H285Q-N286G), heavy chain H285L-N286Q, heavy chain K340L-G341L-Q342-P343G (also represented as K340L-G341L-Q342Q-P343G), heavy chain N384L-G385Q-Q386G-P387G, heavy chain H268L-E269Q-D270G-P271G, heavy chain N325L-K326Q, heavy chain P329Q-A330G, heavy chain K360L-N361Q-Q362G, heavy chain N384Q-G385-Q386G (also represented as N384Q-G385G-Q386G), heavy chain S134L-T135Q-S136G, and heavy chain V282Q; (iii) an endogenous glutamine in the heavy chain Q295 (also represented as Q295Q); (iv) a reactive glutamine obtained by amino acid modification, which is located in an amino acid sequence selected from the group consisting of: light chain K126L-S127Q-G128-T129G (also represented as K126L-S127Q-G128G-T129G), light chain V205L-T206Q-K207G-S208G, light chain T109L-V110Q-A111G-A112G, and light chain Q199L-G200Q-L201S-S202G; and (v) a glutamine in a Q-containing tag LQSG that replaces the light chain G200-L201-S202, and a glutamine in a Q-containing tag LQSG that inserts after the heavy chain T135.

In some embodiments, the heavy chain Q295 endogenous glutamine is the endogenous glutamine in the heavy chain Q295-V296-N297.

In some embodiments, the constant region of the antibody or an antigen-binding fragment thereof comprises one or more reactive glutamine, for example, two, three, four, five, six, seven, eight, nine, or ten reactive glutamine. In some embodiments, the constant region of the antibody or an antigen-binding fragment thereof comprises one or more reactive glutamine, for example, two, four, six, eight, ten, twelve, fourteen, or sixteen reactive glutamine.

In some embodiments, the reactive glutamine comprises heavy chain Q295 endogenous glutamine and one or more reactive glutamine selected from the group consisting of: (1) a glutamine in a Q-containing tag selected from the group consisting of: GGLQSGA, LQSGA, EEQYGA, EEQYQSG and LLQGA, which is located at the carboxyl terminus of the light chain; (2) a glutamine in a Q-containing tag LQSG that replaces the light chain G200-L201-S202; (3) a glutamine in a Q-containing tag LQSG that inserts after the heavy chain T135; and (4) a reactive glutamine obtained by amino acid modification, which is located in an amino acid sequence selected from the group consisting of: heavy chain 354L-355Q-356G (Q438), heavy chain 445Q-G446, and heavy chain 268L-269Q-270G-271G. In a further embodiment, the heavy chain Q295 endogenous glutamine is the endogenous glutamine in the heavy chain Q295-V296-N297. In a further embodiment, the Q-containing tag located at the carboxyl terminus of the light chain is GGLQSGA.

In some embodiments, the reactive glutamine comprises heavy chain Q295 endogenous glutamine and one or more reactive glutamine selected from the group consisting of: (1) a glutamine in a Q-containing tag selected from the group consisting of: GGLQSGA, LQSGA, EEQYGA, EEQYQSG and LLQGA, which is located at the carboxyl terminus of the light chain; (2) a glutamine in a Q-containing tag LQSG that replaces the light chain G200-L201-S202; (3) a glutamine in a Q-containing tag LQSG that inserts after the heavy chain T135; and (4) a reactive glutamine obtained by amino acid modification, which is located in an amino acid sequence selected from the group consisting of: heavy chain S354L-R355Q-E356G (Q438), heavy chain P445Q-G446G, and heavy chain H268L-E269Q-D270G-P271G. In a further embodiment, the heavy chain Q295 endogenous glutamine is the endogenous glutamine in the heavy chain Q295-V296-N297. In a further embodiment, the Q-containing tag located at the carboxyl terminus of the light chain is GGLQSGA.

In some embodiments, the heavy chain Q295 endogenous glutamine is the endogenous glutamine in the heavy chain Q295-V296-N297.

In some embodiments, the Q-containing tag located at the carboxyl terminus of the light chain is GGLQSGA.

In some embodiments, the reactive glutamine comprises an endogenous glutamine in the heavy chain Q295-V296-N297 and one or more reactive glutamine selected from the group consisting of: (1) a glutamine in a Q-containing tag selected from the group consisting of: GGLQSGA, LQSGA, EEQYGA, EEQYQSG and LLQGA, which is located at the carboxyl terminus of the light chain; (2) a glutamine in a Q-containing tag LQSG that replaces the light chain G200-L201-S202; (3) a glutamine in a Q-containing tag LQSG that inserts after the heavy chain T135; and (4) a reactive glutamine obtained by amino acid modification, which is located in an amino acid sequence selected from the group consisting of: heavy chain S354L-R355Q-E356G (Q438), heavy chain P445Q-G446G, and heavy chain H268L-E269Q-D270G-P271G. In a further embodiment, the Q-containing tag located at the carboxyl terminus of the light chain is GGLQSGA.

In some embodiments, the reactive glutamine comprises (or is) the glutamine in a Q-containing tag selected from the group consisting of: GGLQSGA, LQSGA, EEQYGA, EEQYQSG and LLQGA, which is located at the carboxyl terminus of the light chain, and the heavy chain Q295 endogenous glutamine. In some embodiments, the reactive glutamine comprises (or is) the glutamine in a Q-containing tag selected from the group consisting of: GGLQSGA, LQSGA, EEQYGA, EEQYQSG and LLQGA, which is located at the carboxyl terminus of the light chain, and endogenous glutamine in the heavy chain Q295-V296-N297. In some embodiments, the reactive glutamine comprises (or is) the glutamine in a Q-containing tag GGLQSGA located at the carboxyl terminus of the light chain and endogenous glutamine in the heavy chain Q295-V296-N297.

In some embodiments, the reactive glutamine comprises (or is) the reactive glutamine obtained by amino acid modification in the heavy chain 354L-355Q-356G (Q438), and heavy chain Q295 endogenous glutamine. In some

embodiments, the reactive glutamine comprises (or is) reactive glutamine obtained by amino acid modification in the heavy chain S354L-R355Q-E356G (Q438) and heavy chain Q295 endogenous glutamine. In some embodiments, the reactive glutamine comprises (or is) reactive glutamine obtained by amino acid modification in the heavy chain S354L-R355Q-E356G (Q438) and endogenous glutamine in the heavy chain Q295-V296-N297.

In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, heavy chain Q295 endogenous glutamine, and reactive glutamine obtained by amino acid modification in the heavy chain 354L-355Q-356G (Q438). In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295-V296-N297, and a reactive glutamine obtained by amino acid modification in the heavy chain 354L-355Q-356G (Q438). In some embodiments, the reactive glutamine comprises (or is) a glutamine in a Q-containing tag GGLQSGA located at the carboxyl terminus of the light chain, an endogenous glutamine in the heavy chain Q295-V296-N297, and a reactive glutamine obtained by amino acid modification in the heavy chain S354L-R355Q-E356G (Q438).

In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295, and a reactive glutamine obtained by amino acid modification in the heavy chain 354L-355Q-356G (Q438) and heavy chain 445Q-G446. In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295-V296-N297, and a reactive glutamine obtained by amino acid modification in the heavy chain 354L-355Q-356G (Q438) and heavy chain 445Q-G446. In some embodiments, the reactive glutamine comprises (or is) a glutamine in a Q-containing tag GGLQSGA located at the carboxyl terminus of the light chain, an endogenous glutamine in the heavy chain Q295-V296-N297, and a reactive glutamine obtained by amino acid modification in the heavy chain S354L-R355Q-E356G (Q438) an heavy chain P445Q-G446G. In some embodiments, the amino acid modification in the heavy chain Q438I is further comprised.

In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295, a reactive glutamine obtained by amino acid modification in the heavy chain 354L-355Q-356G (Q438), and a glutamine in a Q-containing tag LQSG that inserts after the heavy chain T135. In some embodiments, the reactive glutamine comprises (or is) glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295-V296-N297, a glutamine in the heavy chain 354L-355Q-356G (Q438) and a glutamine in a Q-containing tag LQSG that inserts after the heavy chain T135. In some embodiments, the reactive glutamine comprises (or is) a glutamine in a Q-containing tag GGLQSGA located at the carboxyl terminus of the light chain, an endogenous glutamine in the heavy chain Q295-V296-N297, a reactive glutamine obtained by amino acid modification in the heavy chain S354L-R355Q-E356G (Q438), and a glutamine in a Q-containing tag LQSG that inserts after the heavy chain T135.

In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295, a reactive glutamine obtained by amino acid modification in the heavy chain 354L-355Q-356G (Q438), and a glutamine in a Q-containing tag LQSG that replaces the light chain G200-L201-S202. In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the light chain, an endogenous glutamine in the heavy chain Q295-V296-N297, a reactive glutamine obtained by amino acid modification in the heavy chain 354L-355Q-356G (Q438), and a glutamine in a Q-containing tag LQSG that replaces the light chain G200-L201-S202. In some embodiments, the reactive glutamine comprises (or is) a glutamine in a Q-containing tag GGLQSGA located at the carboxyl terminus of the light chain, an endogenous glutamine in the heavy chain Q295-V296-N297, a reactive glutamine obtained by amino acid modification in the heavy chain S354L-R355Q-E356G (Q438), and a glutamine in the Q-containing tag LQSG that replaces the light chain G200-L201-S202.

In some embodiments, the reactive glutamine comprises (or is) reactive glutamine obtained by amino acid modification in the heavy chain 354L-355Q-356G (Q438) and the heavy chain 445Q-G446, and the constant region of the antibody or an antigen-binding fragment thereof further comprises an amino acid modification of 438I. In some embodiments, the reactive glutamine comprises (or is) a reactive glutamine obtained by amino acid modification in the heavy chain S354L-R355Q-E356G (Q438) and the heavy chain P445Q-G446G, and the constant region of the antibody or an antigen-binding fragment thereof further comprises an amino acid modification of Q438I.

In some embodiments, the reactive glutamine comprises (or is) a reactive glutamine obtained by amino acid modification in the heavy chain 354L-355Q-356G (Q438) and the heavy chain 445Q-G446, and the antibody or an antigen-binding fragment thereof further comprises an amino acid modification of 295A. In some embodiments, the reactive glutamine comprises (or is) a reactive glutamine obtained by amino acid modification in the heavy chain S354L-R355Q-E356G (Q438) and the heavy chain P445Q-G446G, and the antibody or an antigen-binding fragment thereof further comprises an amino acid modification of Q295A.

In some embodiments, the reactive glutamine comprises a reactive glutamine obtained by amino acid modification in the heavy chain 445Q-G446, and the K is removed from the C-terminus of the heavy chain.

In some embodiments, the reactive glutamine comprises (or is) a glutamine in a Q-containing tag selected from the group consisting of: GGLQSGA, LQSGA, EEQYGA, EEQYQSG and LLQGA, which is located at the carboxyl terminus of the heavy chain and/or light chain. In some embodiments, the K is removed from the C-terminus of the heavy chain before the Q-containing tag is attached.

In some embodiments, the specific amino acid mutation schemes of the reactive glutamine are shown in Table 1.

In some embodiments, the specific amino acid mutation schemes of the antibody or an antigen-binding fragment thereof are shown in Table 1.

In some embodiments, both heavy chains and/or both light chains of the antibody are inserted into the Q-containing tag or both contain the amino acid modification. In some embodiments, the Q-containing tag or the amino acid modification is the same in both heavy chains and/or both light chains. In some embodiments, the Q-containing tag and the amino acid modification are identical in both heavy chains. In some embodiments, the Q-containing tag and the amino acid modification are identical in both light chains. In some embodiments, the Q-containing tag and the amino acid modification are identical in both heavy chains and both light chains.

In some embodiments, the antibody moiety is connected to the drug conjugated moiety via a linker containing an amino group, which is covalently connected to reactive glutamine in the antibody.

In some embodiments, the antibody-drug conjugate has the following structure: A-(NH-L-X)d, where A is the antibody or antigen-binding fragment described in this application, -NH-L- is the linker, X is the drug molecule, and d is 1-16, preferably 1-12, more preferably 1-8, such as an integer or decimal of 1, 2, 3, 4, 5, 6, 7, 8.

In some embodiments, -L- comprises an extension unit, and/or a reaction unit, and/or a cleavable unit, and/or a self-cleaving unit, and/or a branching unit, and/or a a hydrophilic unit.

In some embodiments, -L- has the following structure: -L1-RG-B-(L2-L3)s-, where L1 is an extension unit, RG is a reaction unit or bond, B is a branching unit or bond, L2 is a cleavable unit or bond, L3 is a self-cleaving unit or bond, and when B is a bond, s is 1, and when B is a branching unit, s is selected from 2 or 3, wherein L1, B, L2, and L3 are optionally connected with hydrophilic units.

In some embodiments,

wherein, -L1- is selected from the group consisting of:

- $(CH_2CH_2O)_nCH2C$ (O)-, - $(CH_2CH_2O)_nCH_2CH_2C$ (O)-, or - $(CH_2CH_2O)_nCH_2CH_2$-, n is an integer selected from 1 to 24, preferably an integer from 3 to 12, more preferably an integer from 3 to 8, and most preferably an integer from 3 to 6.

RG is selected from bond or:

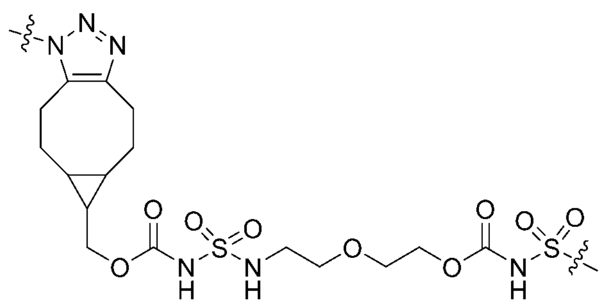

;

B is selected from bond or:

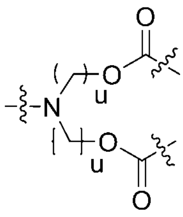, 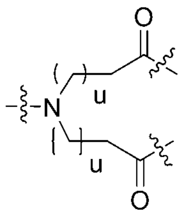, 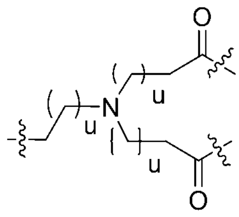, or 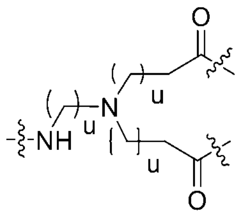,

wherein each u is independently selected from an integer from 1 to 8, such as 1, 2, 3, 4, 5, 6, 7, 8.
L2 is selected from bond or:

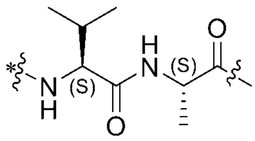, 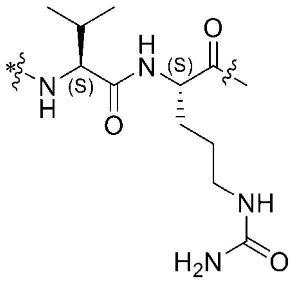, 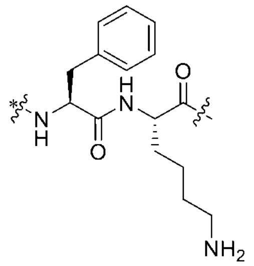,

or

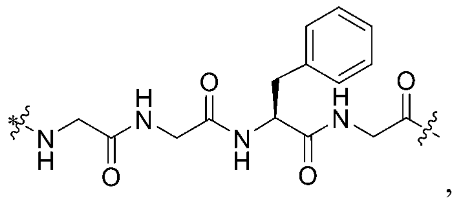,

wherein the * terminal is connected to B;
L3 is selected from bond or:

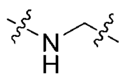, or 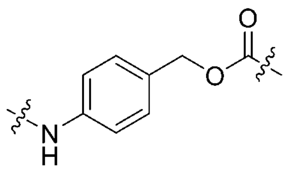,

wherein the N terminal is connected to L2.

In some embodiments, X is selected from::

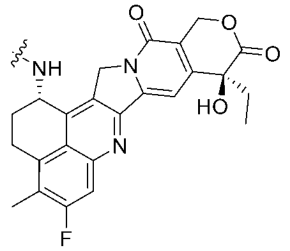, 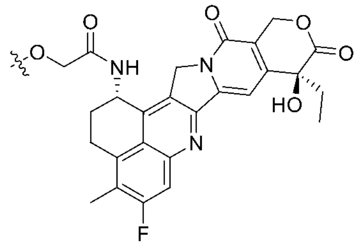, 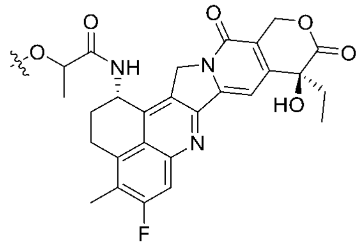,

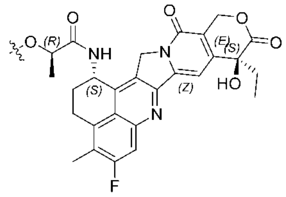, 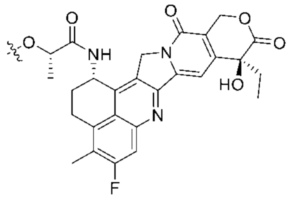, or 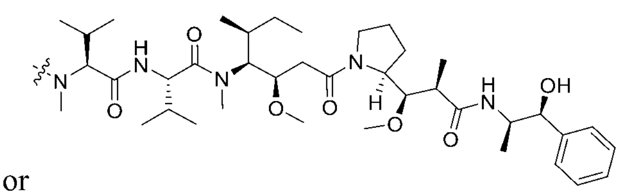.

In some embodiments, the antibody-drug conjugate has the following structure:

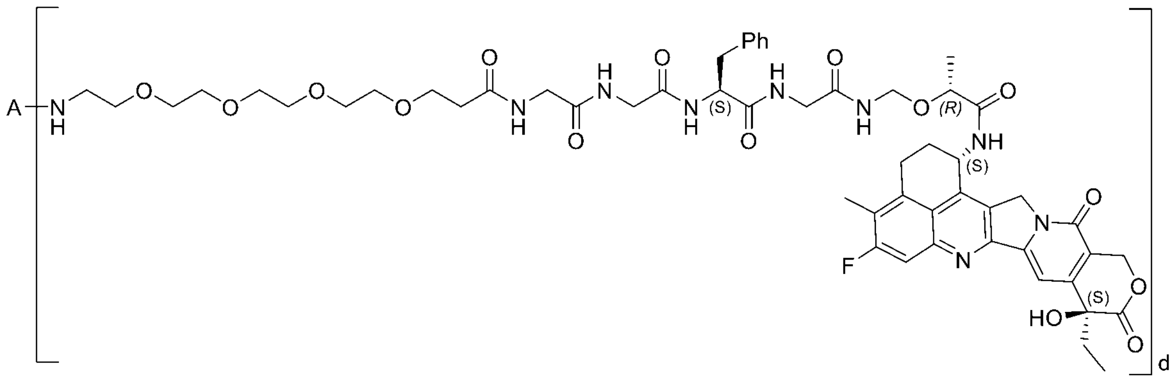,

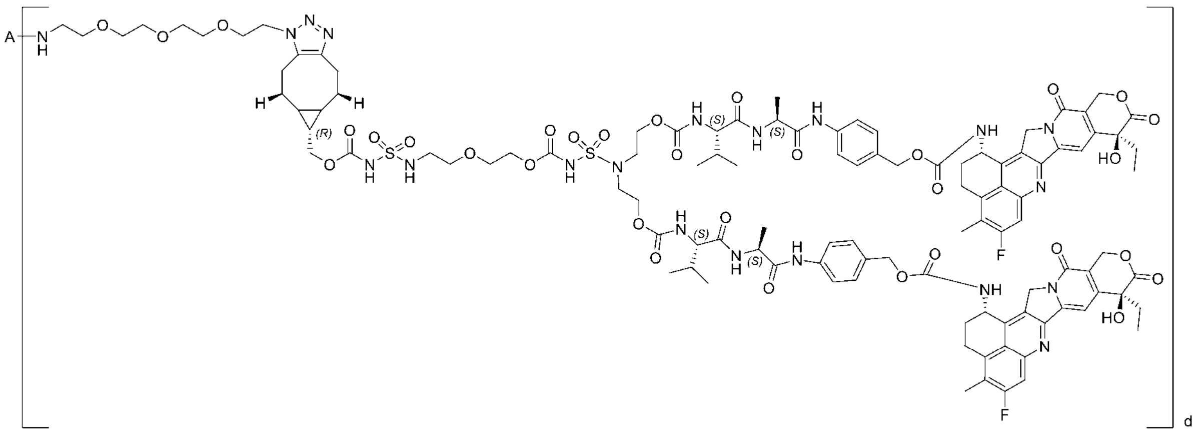,

or

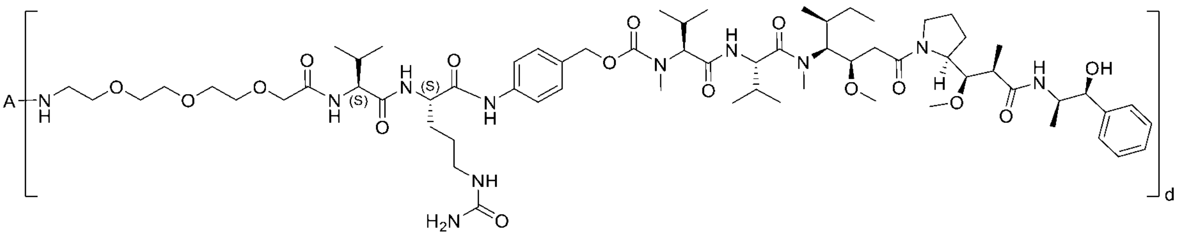.

In some embodiments, in the antibody-drug conjugate, the antibody moiety and the drug conjugated moiety are connected by a linker, which may be a cleavable linker or a non-cleavable linker. In some embodiments, when the linker is a cleavable linker, the cleavable linker includes, but is not limited to, $NH_2$-$(CH_2CH_2O)_n$-Val-Cit-PABC-X and $NH_2$-$(CH_2CH_2O)_n$-(Val-Cit-PABC-X$)_2$, wherein X is a drug molecule, and n is an integer of at least 1 (such as any one of 2, 3, 4, 6, 8, 10, 12, 16, 20 or 24), PABC refers to p-aminobenzyloxycarbonyl, Val refers to valine, and Cit refers to citrulline. In some embodiments, when the linker is an non-cleavable linker, the non-cleavable linker includes, but is not limited to, but is not limited to, $NH_2$-R-X, $NH_2NH$-R-X and $NH_2$-O-R-X, wherein R is an alkyl or polyethylene glycol (PEG) group, X is a drug molecule, and the polyethylene glycol group can have the general formula - $(CH_2CH_2O)_n$-, wherein n is an integer of at least 1 (such as any one of 2, 4, 6, 8, 10, 12, 16, 20 or 24).

In some embodiments, the linker is selected from: acetyl-lysine-glycine, aminocaproic acid, acetyl-lysine-β-alanine, amino-$PEG_2$-$C_2$, amino-$PEG_3$-$C_2$, amino-$PEG_6$-$C_2$, acetyl-lysine-valine-citrulline-p-aminobenzyloxycarbonyl, amino-$PEG_6$-$C_2$-Val-Cit-PABC, amino-$PEG_3$-$C_2$-Val-Cit-PABC, aminohexanoyl-Val-Cit-PABC, [(3R,5R)-1-{3-[2-(2-aminoethoxy)ethoxy]propionyl}piperidine-3,5-diyl]bis-Val-Cit-PABC, [(3S,5S)-1-{3-[2-(2-aminoethoxy)ethoxy]propionyl}piperidine-3,5-di]bis-Val-Cit-PABC, putrescine, or Ac-Lys-putrescine.

In some embodiments, the cleavable linker is of the structure shown in Formulas I-IV:

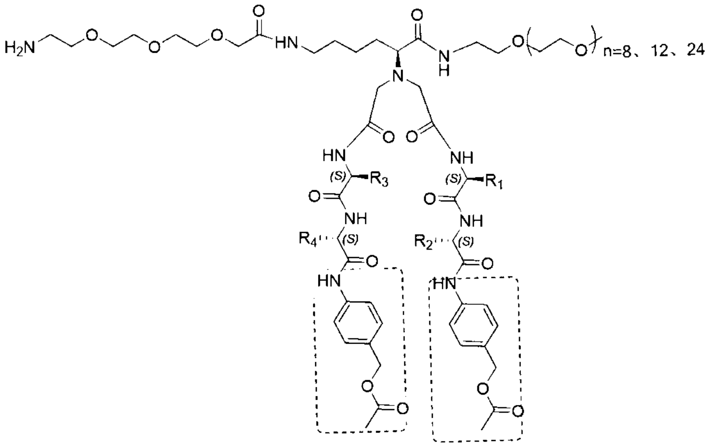

Formulas I

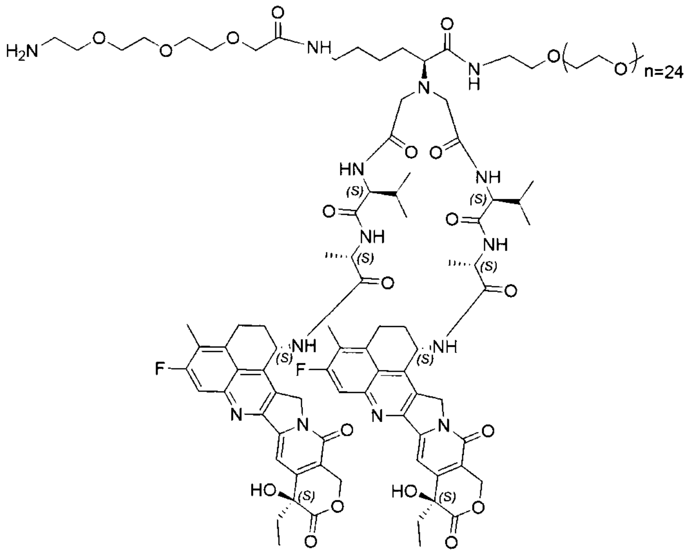

Formula II

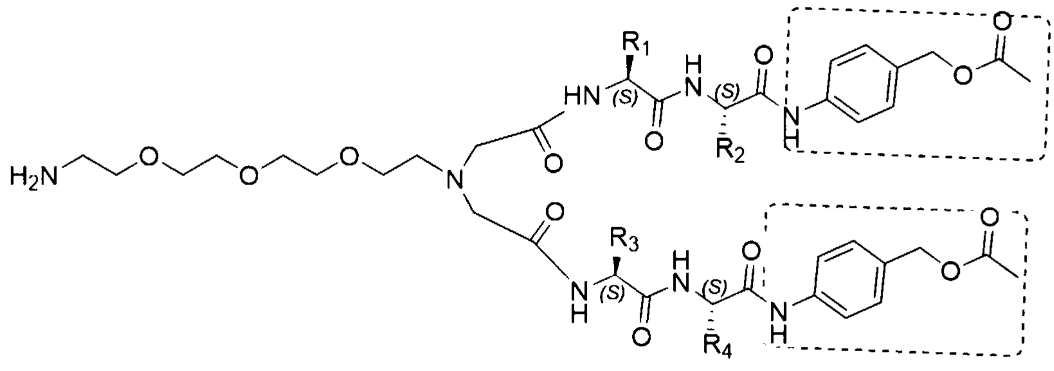

Formula III

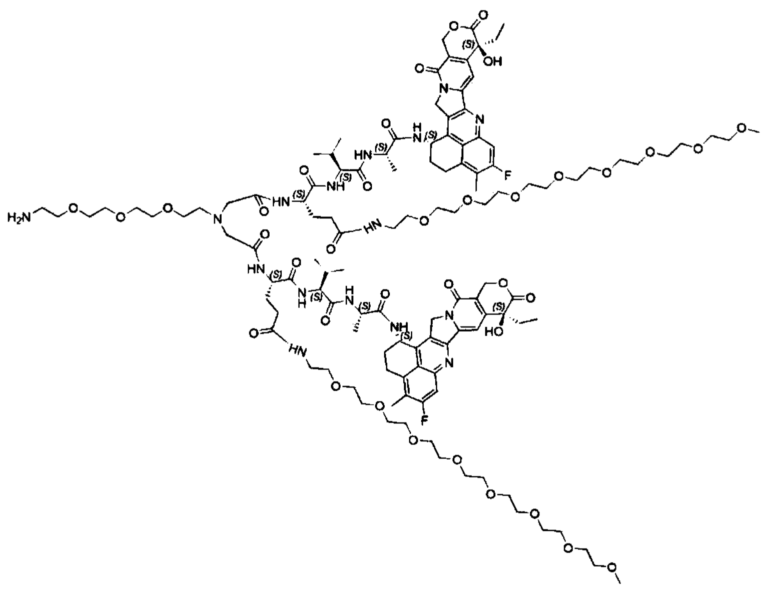

Formula IV

In some embodiments, the antibody in the antibody-drug conjugate is a monoclonal antibody, polyclonal antibody, human antibody, humanized antibody, chimeric antibody, microantibody, or bispecific antibody.

In some embodiments, the ratio of the drug molecule to the antibody is about 2-16:1, for example, about 2:1, 4:1, 6:1, 8:1, 10:1, 12:1, 14:1, or 16:1.

In some embodiments, the drug molecule is an anticancer drug, e.g., a cytotoxic drug, an immune enhancer, or a radioactive isotope.

In some embodiments, the cytotoxic drug is selected from: microtubule inhibitors, DNA topoisomerase inhibitors, DNA damaging agents, immune enhancers, antimetabolites, or antitumor antibiotics.

In some embodiments, the microtubule inhibitor is selected from: Auristatin derivatives (e.g., MMAE (Monomethyl Auristatin E), MMAF (Monomethyl Auristatin F)) or Mertansine alkaloid derivatives (e.g., DM1, DM4, Ansamitocin, Mertansine, Dolastatin, or derivatives thereof).

In some embodiments, the DNA topoisomerase inhibitor is selected from: camptothecin analogs, other DNA topoisomerase I inhibitors or derivatives thereof, e.g., DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyeclipticine, topotecan, letopotecan, Belotecan, Exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenyl methyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]amin o]-5H-indolo[2,3-a]pyrrolo[3,4-c]carbazole-5,7(6H)-dione, N-[2-(dimethylamino)ethyl]-4-acridinecarboxamide dihydrochloride, N-[2-(dimethylamino)ethyl]-4-acridinecarboxamide or derivatives thereof.

In some embodiments, the DNA damaging agent is selected from: calicheamicin, duocarmycin, or anthramycin derivative PBD (pyrrolobenzodiazepine).

In some embodiments, the immune enhancer is selected from: levamisole, pidotimod, imiquimod, isoproinosine, polyinosinic-polycytidylic acid, or polyinosinic-uridine acid.

In some embodiments, the antimetabolite is selected from: methotrexate, 6-mercaptopurine, or 5-fluorouracil.

In some embodiments, the antitumor antibiotic is selected from: polypeptide antibiotics (e.g., actinomycin D or bleomycin) or anthraquinone drugs (e.g., adriamycin or mitoxantrone hydrochloride).

In some embodiments, the radioisotope is selected from: $^{211}$At, $^{131}$I, $^{125}$I, $^{90}$Y, $^{186}$Re, $^{188}$Re, $^{153}$Sm, $^{212}$Bi, $^{32}$P, $^{60}$Co or $^{177}$Lu.

In some embodiments, the antibody is an anti-HER2, anti-Nectin 4, or anti-ROR1 antibody.

In some embodiments, the antibody encompasses monoclonal antibodies, polyclonal antibodies, antibody fragments (e.g., Fab, Fab', F(ab')2, Fv, Fc, etc.), chimeric antibodies, humanized antibodies, human antibodies (e.g., fully human antibodies), single-chain (ScFv), bispecific antibodies, multispecific antibodies, mutants thereof, fusion proteins containing antibody moieties, and any other modified configurations of immunoglobulin molecules containing antigen recognition sites of desired specificity.

In some embodiments, the anti-HER2 antibody comprises a heavy chain variable region and a light chain variable region, wherein the heavy chain variable region comprises heavy chain CDR1 as shown in SEQ ID NO:1, heavy chain CDR2 as shown in SEQ ID NO:2, and heavy chain CDR3 as shown in SEQ ID NO:3, and the light chain variable region comprises light chain CDR1 as shown in SEQ ID NO:4, light chain CDR2 as shown in SEQ ID NO:5, and light chain CDR3 as shown in SEQ ID NO:6.

In some embodiments, the anti-HER2 antibody comprises a heavy chain variable region as shown in SEQ ID NO:7, and/or comprises a light chain variable region sequence as shown in SEQ ID NO:8.

In some embodiments, the anti-HER2 antibody comprises a heavy chain constant region and a light chain constant region, wherein the heavy chain constant region is subjected to reactive glutamine mutation based on the heavy chain constant region as shown in SEQ ID NO:9, and/or the light chain constant region is subjected to reactive glutamine mutation based on the light chain constant region as shown in SEQ ID NO: 10.

In some embodiments, the anti-HER2 antibody is subjected to reactive glutamine mutation based on the light and heavy chain amino acid sequences of DP001 or trastuzumab.

In some embodiments, the heavy chain constant region of the anti-HER2 antibody comprises one or more reactive glutamine selected from the group consisting of: (i) a glutamine in a Q-containing tag selected from the group consisting of: GGLQSGA, LQSGA, EEQYGA, EEQYQSG and LLQGA, which is located at carboxyl terminus of light chain, heavy chain, or both the light chain and the heavy chain; (ii) a reactive glutamine obtained by amino acid modification, which is located in an amino acid sequence selected from the group consisting of: heavy chain S354L-R355Q-E356G (Q438), heavy chain P445Q-G446, heavy chain S117L-A118Q-S119G-T120G, heavy chain H310L-Q311-D312G, heavy chain D399L-S400Q-D401G, heavy chain H268L-E269Q-D270G, heavy chain M252Q-1253G, heavy chain S444Q-P445G-G446A, heavy chain E283L-V284-H285Q-N286G, heavy chain H285L-N286Q, heavy chain K340L-G341L-Q342-P343G, heavy chain N384L-G385Q-Q386G-P387G, heavy chain H268L-E269Q-D270G-P271G, heavy chain N325L-K326Q, heavy chain P329Q-A330G, heavy chain K360L-N361Q-Q362G, heavy chain N384Q-G385-Q386G, heavy chain S134L-T135Q-S136G, and heavy chain V282Q; (iii) endogenous glutamine in Q295; (iv) a reactive glutamine obtained by amino acid modification, which is located in an amino acid sequence selected from the group consisting of: light chain K126L-S127Q-G128-T129G, light chain V205L-T206Q-K207G-S208G, light chain T109L-V110Q-A111G-A112G or light chain Q199L-G200Q-L201S-S202G; and (v) glutamine in the Q-containing tag LQSG that replaces the light chain G200-L201-S202, and glutamine in the Q-containing tag LQSG that inserts after the heavy chain T135.

In some embodiments, the anti-Nectin 4 antibody comprises a heavy chain variable region and a light chain variable region, wherein the heavy chain variable region comprises heavy chain CDR1 as shown in SEQ ID NO:11, heavy chain CDR2 as shown in SEQ ID NO:12, and heavy chain CDR3 as shown in SEQ ID NO:13, and the light chain variable region comprises light chain CDR1 as shown in SEQ ID NO:14, light chain CDR2 as shown in SEQ ID NO:15, and light chain CDR3 as shown in SEQ ID NO:16.

In some embodiments, the anti-Nectin 4 antibody comprises a heavy chain variable region as shown in SEQ ID NO:17, and/or comprises a light chain variable region sequence as shown in SEQ ID NO:18.

In some embodiments, the anti-Nectin 4 antibody comprises a heavy chain constant region and a light chain constant region, wherein the heavy chain constant region is subjected to reactive glutamine mutation based on the heavy chain constant region shown in SEQ ID NO:19, and/or the light chain constant region is subjected to reactive glutamine mutation based on the light chain constant region shown in SEQ ID NO:20.

In some embodiments, the anti-Nectin4 antibody is subjected to reactive glutamine mutation based on the light and heavy chain amino acid sequences of Enfortumab.

In some embodiments, the heavy chain constant region of the anti-Nectin 4 antibody comprises one or more reactive glutamine selected from the group consisting of: (i) a glutamine in a Q-containing tag selected from the group consisting of: GGLQSGA, LQSGA, EEQYGA, EEQYQSG and LLQGA, which is located at carboxyl terminus of light chain, heavy chain, or both the light chain and the heavy chain; (ii) a reactive glutamine obtained by amino acid modification,

which is located in an amino acid sequence selected from the group consisting of: heavy chain S354L-R355Q-E356G (Q438), heavy chain P445Q-G446, heavy chain S117L-A118Q-S119G-T120G, heavy chain H310L-Q311-D312G, heavy chain D399L-S400Q-D401G, heavy chain H268L-E269Q-D270G, heavy chain M252Q-1253G, heavy chain S444Q-P445G-G446A, heavy chain E283L-V284-H285Q-N286G, heavy chain H285L-N286Q, heavy chain K340L-G341L-Q342-P343G, heavy chain N384L-G385Q-Q386G-P387G, heavy chain H268L-E269Q-D270G-P271G, heavy chain N325L-K326Q, heavy chain P329Q-A330G, heavy chain K360L-N361Q-Q362G, heavy chain N384Q-G385-Q386G, heavy chain S134L-T135Q-S136G, and heavy chain V282Q; (iii) endogenous glutamine in Q295; (iv) a reactive glutamine obtained by amino acid modification, which is selected from the group consisting of: light chain K126L-S127Q-G128-T129G, light chain V205L-T206Q-K207G-S208G, light chain T109L-V110Q-A111G-A112G and light chain Q199L-G200Q-L201S-S202G; and (v) glutamine in the Q-containing tag LQSG that replaces the light chain G200-L201-S202, and glutamine in the Q-containing tag LQSG that inserts after the heavy chain T135.

In some embodiments, the anti-ROR1 antibody comprises a heavy chain variable region and a light chain variable region, wherein the heavy chain variable region comprises heavy chain CDR1 as shown in SEQ ID NO:21, heavy chain CDR2 as shown in SEQ ID NO:22, and heavy chain CDR3 as shown in SEQ ID NO:23, and the light chain variable region comprises light chain CDR1 as shown in SEQ ID NO:24, light chain CDR2 as shown in SEQ ID NO:25, and light chain CDR3 as shown in SEQ ID NO:26.

In some embodiments, the anti-ROR1 antibody comprises a heavy chain variable region and a light chain variable region, wherein the heavy chain variable region comprises heavy chain CDR1 as shown in SEQ ID NO:21, heavy chain CDR2 as shown in SEQ ID NO:29, and heavy chain CDR3 as shown in SEQ ID NO:23, and the light chain variable region comprises light chain CDR1 as shown in SEQ ID NO:24, light chain CDR2 as shown in SEQ ID NO:25, and light chain CDR3 as shown in SEQ ID NO:26.

In some embodiments, the anti-ROR1 antibody comprises a heavy chain variable region and a light chain variable region, wherein the heavy chain variable region comprises heavy chain CDR1 as shown in SEQ ID NO:21, heavy chain CDR2 as shown in SEQ ID NO:31, and heavy chain CDR3 as shown in SEQ ID NO:23, and the light chain variable region comprises light chain CDR1 as shown in SEQ ID NO:24, light chain CDR2 as shown in SEQ ID NO:25, and light chain CDR3 as shown in SEQ ID NO:26.

In some embodiments, the anti-ROR1 antibody comprises a heavy chain variable region as shown in SEQ ID NO:27, and/or comprises a light chain variable region sequence as shown in SEQ ID NO:28.

In some embodiments, the anti-ROR1 antibody comprises a heavy chain variable region as shown in SEQ ID NO:30, and/or comprises a light chain variable region sequence as shown in SEQ ID NO:28.

In some embodiments, the anti-ROR1 antibody heavy chain comprises a heavy chain variable region as shown in SEQ ID NO:32, and/or comprises a light chain variable region sequence as shown in SEQ ID NO:28.

In some embodiments, the anti-ROR1 antibody comprises a heavy chain and a light chain, wherein the heavy chain comprises a heavy chain constant region as shown in SEQ ID NO:33, and the light chain comprises a light chain constant region as shown in SEQ ID NO:34.

In some embodiments, the heavy chain constant region of the anti-ROR1 antibody comprises one or more reactive glutamine selected from the group consisting of: (i) a glutamine in a Q-containing tag selected from the group consisting of: GGLQSGA, LQSGA, EEQYGA, EEQYQSG and LLQGA, which is located at carboxyl terminus of light chain, heavy chain, or both the light chain and the heavy chain; (ii) a reactive glutamine obtained by amino acid modification, which is located in an amino acid sequence selected from the group consisting of: heavy chain S354L-R355Q-E356G (Q438), heavy chain P445Q-G446, heavy chain S117L-A118Q-S119G-T120G, heavy chain H310L-Q311-D312G, heavy chain D399L-S400Q-D401G, heavy chain H268L-E269Q-D270G, heavy chain M252Q-I253G, heavy chain S444Q-P445G-G446A, heavy chain E283L-V284-H285Q-N286G, heavy chain H285L-N286Q, heavy chain K340L-G341L-Q342-P343G, heavy chain N384L-G385Q-Q386G-P387G, heavy chain H268L-E269Q-D270G-P271G, heavy chain N325L-K326Q, heavy chain P329Q-A330G, heavy chain K360L-N361Q-Q362G, heavy chain N384Q-G385-Q386G, heavy chain S134L-T135Q-S136G, and heavy chain V282Q; (iii) endogenous glutamine in Q295; (iv) a reactive glutamine obtained by amino acid modification, which is located in an amino acid sequence selected from the group consisting of: light chain K126L-S127Q-G128-T129G, light chain V205L-T206Q-K207G-S208G, light chain T109L-V110Q-A111G-A112G and light chain Q199L-G200Q-L201S-S202G; and (v) glutamine in the Q-containing tag LQSG that replaces the light chain G200-L201-S202, and glutamine in the Q-containing tag LQSG that inserts after the heavy chain T135. The variable area and CDR numbering in this application adopt the Kabat numbering rule.

The constant regions in this application adopt the EU numbering rule.

In some embodiments, the drug molecule is DXD or a derivative thereof, or MMAE or a derivative thereof.

In some embodiments, the linker is $NH_2$-$(CH_2CH_2O)_n$-Val-Cit-PABC-X, wherein X is a drug molecule; preferably, n is 3.

In some embodiments, the linker is $NH_2$-$(CH_2CH_2O)_n$-Val-Cit-PABC-X, wherein X is DXD or a derivative thereof, or MMAE or a derivative thereof; preferably, n is 3.

In some embodiments, the linker can be directly conjugated to the antibody in a one-step process using mTGase

enzymatic conjugation, with hydrophilicity adjusted using PEG.

In some embodiments, the drug conjugated moiety is LND1002, with a structure as shown in Formula V: Formula:

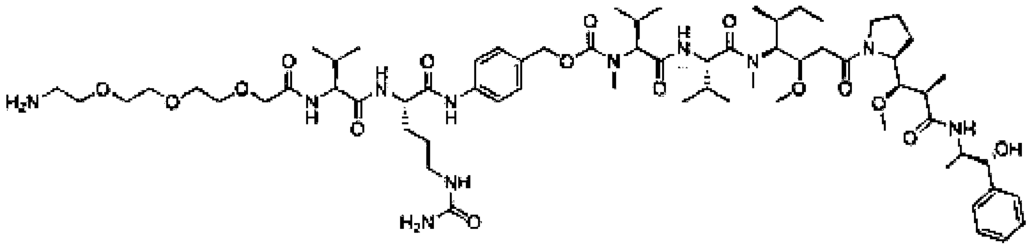

molecular formula: C66H109N11O16

molecular weight: 1312.64

Formula V

In some embodiments, the drug conjugated moiety is JSSW038, the structure of which is shown in Formula VI:

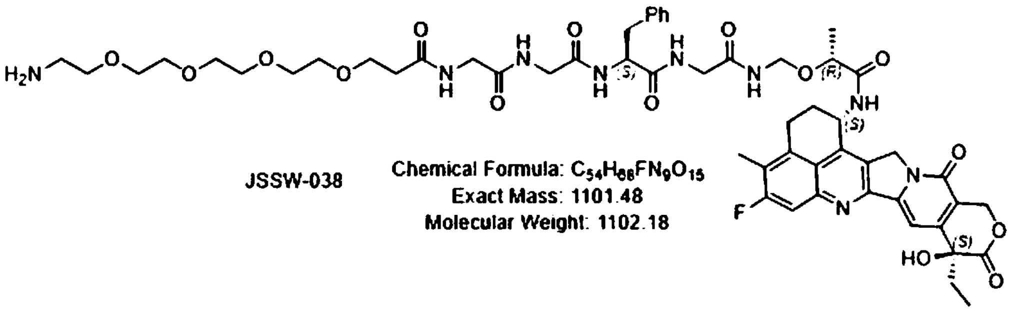

JSSW-038

Chemical Formula: $C_{54}H_{68}FN_9O_{15}$

Exact Mass: 1101.48

Molecular Weight: 1102.18

Formula VI

In some embodiments, the structure of the drug conjugated moiety is as shown in Formula VII:

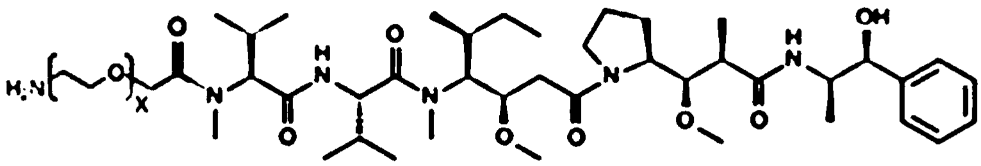

Formula VII.

In some embodiments, the antibody-drug conjugate is AB5-ADC, MFC8-ADC, MFC10-ADC, DP28-ADC, DP35-ADC, DP36-ADC, DP35-JSSW038, DP36-JSSW038, MFC8-JSSW038, DP28-JSSW038, Hu17-H2L1-4LND1002, Hu17-H3L1-4LND1002, or Hu17-H4L1-4LND1002.

The structure of the exemplary drug conjugated moiety can also be found in Figures 2A-2B.

In the third aspect, this application relates to a method for preparing the above-mentioned antibody-drug conjugate, including the step of performing one or more insertion or substitution mutations in a constant region of the antibody to obtain reactive glutamine.

In some embodiments, the reactive glutamine obtained by insertion or substitution mutation is as described in the first aspect above.

In some embodiments, the method for the above-mentioned antibody-drug conjugate further includes the step of preparing the antibody and conjugating it to the drug conjugated moiety via mTGase enzymatic conjugation to obtain the ADC.

This application also relates to a method for preparing antibody-drug conjugates with high drug load, the method

comprising the step of firstly preparing the antibody-drug conjugate by the aforementioned method, and then further coupling the drug conjugated moiety by a chemical method to obtain an ADC with a higher DAR value.

In some embodiments, the chemical coupling comprises one or more of click chemistry, amide condensation, and Michael addition. In one embodiment, the chemical method is a click chemistry method achieved by an azide-cycloyne addition reaction.

In some embodiments, an ADC may be prepared first by the aforementioned chemical method, and then the drug conjugated moiety may be coupled by the aforementioned mTGase enzymatic conjugation to obtain an ADC with a higher DAR value.

In some embodiments, the drug conjugated moiety is JSSW-SY.

In some embodiments, the steps of preparing monoclonal antibodies include constructing the mutated sequence into a eukaryotic cell protein expression plasmid vector, extracting the plasmid and transiently transfecting CHO cells to express monoclonal antibodies, and conjugating the obtained antibodies using the mTGase enzymatic conjugation to obtain ADCs.

In the fourth aspect, this application relates to the use of the aforementioned antibody-drug conjugates or antibodies in the preparation of medicaments for treating cancer.

In the fifthly aspect, this application relates to methods for using the aforementioned antibody-drug conjugates or antibodies to treat cancer.

In the sixthly aspect, this application relates to a method for treating cancer, the method comprises administering a therapeutically effective amount of the aforementioned antibody-drug conjugate or antibody.

In the seventhly aspect, this application relates to an acyl-donor substrate Q-containing tag for transglutaminase, wherein the Q-containing tag is selected from the group consisting of: (i) GGLQSGA, LQSGA, EEQYGA, and EEQYQSG located at the carboxyl terminus of the light chain, heavy chain, or both the light chain and the heavy chain; (ii) heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q; (iii) light chain 126L-127Q-G128-129G, light chain 205L-206Q-207G-208G, light chain 109L-110Q-111G-112G and light chain 199L-200Q-201S-202G; and (iv) a Q-containing tag LQSG that replaces the light chain G200-L201-S202, and a Q-containing tag LQSG that inserts after the heavy chain T135.

In some embodiments, the Q-containing tag is selected from the group consisting of: (i) GGLQSGA, LQSGA, EEQYGA, and EEQYQSG located at the carboxyl terminus of the light chain, heavy chain, or both the light chain and the heavy chain; (ii) heavy chain S354L-R355Q-E356G (Q438), heavy chain P445Q-G446, heavy chain S117L-A118Q-S119G-T120G, heavy chain H310L-Q311-D312G, heavy chain D399L-S400Q-D401G, heavy chain H268L-E269Q-D270G, heavy chain M252Q-1253G, heavy chain S444Q-P445G-G446A, heavy chain E283L-V284-H285Q-N286G, heavy chain H285L-N286Q, heavy chain K340L-G3410L-Q342-P343G, heavy chain N384L-G385Q-Q386G-P387G, heavy chain H268L-E269Q-D270G-P271G, heavy chain N325L-K326Q, heavy chain P329Q-A330G, heavy chain K360L-N361Q-Q362G, heavy chain N384Q-G385-Q386G, heavy chain S134L-T135Q-S136G, and heavy chain V282Q; (iii) endogenous glutamine in Q295; (iv) light chain K126L-S127Q-G128-T129G, light chain V205L-T206Q-K207G-S208G, light chain T109L-V110Q-A111G-A112G, light chain Q199L-G200Q-L201S-S202G; and (v) a Q-containing tag LQSG that replaces the light chain G200-L201-S202, and a Q-containing tag LQSG that inserts after the heavy chain T135.

In aspects four to seven:

In some embodiments, the cancer is a solid tumor or a hematoma.

In some embodiments, the solid tumor is a HER-2-positive cancer, e.g., breast cancer, colorectal cancer (also known as colon and rectal cancer, and including colon and rectal cancer), ovarian cancer, gastric cancer (also known as gastric adenocarcinoma), urethral cancer, or lung cancer (including small cell lung cancer and non-small cell lung cancer), preferably breast cancer.

In some embodiments, the solid tumor is a Nectin4-positive cancer, e.g., breast cancer, pancreatic cancer, lung cancer, ovarian cancer, or gastric cancer.

In some embodiments, the solid tumor or hematoma is a ROR1-positive cancer, e.g., hematologic malignancies, such as chronic lymphocytic leukemia (CLL), mantle cell lymphoma (MCL), or solid tumors (ovarian cancer, breast cancer, prostate cancer, lung cancer, melanoma, and colorectal cancer, etc.).

In some embodiments, the antibody-drug conjugate or antibody of this application may be used alone.

In some embodiments, the antibody-drug conjugate or antibody of this application can be used in combination with other therapies, and can be combined with surgery, radiotherapy, hormone therapy, etc.

In some embodiments of various aspects of the present application, the antibody or functional fragment thereof of this application has ADCC activity.

In some embodiments of various aspects of the present application, the antibody or its functional fragment thereof of this application has CDC activity.

In some embodiments of various aspects of the present application, the antibody or an antigen-binding fragment thereof of this application is an IgG1 antibody.

This application uses the amino acid sequence of the antibody as a backbone. For example, based on the glutamine at position 295 (EU numbering) of the antibody Fc region as a conjugation site for mTGase with a DAR of 2, amino acid mutations are introduced into the constant region of IgG1 to increase the number of mTGase-catalyzed conjugation sites, thereby obtaining an antibody-drug conjugate with high drug load.

Computer-aided drug design is employed to model the constant region of the antibody. Mutations to glutamine are introduced at positions with high solvent accessibility and exposed spatial structure, or the amino acids surrounding native glutamine residues are replaced with amino acids with smaller spatial structures to enhance glutamine exposure, thereby enabling these glutamine to serve as conjugation sites for mTGase catalysis and consequently increase the DAR of the resulting ADC. The mutated sequence is constructed into a eukaryotic protein expression plasmid vector, and the plasmid is transiently transfected into expression cells (e.g., CHO cells) for monoclonal antibody production. The obtained antibody is then conjugated via mTGase enzymatic coupling to generate the ADC. The antibody-drug conjugate with high drug load obtained in this application exhibits favorable homogeneity, enhanced tumor-killing efficacy, and good safety. An exemplary schematic diagram of the ADC conjugation reaction via the mTGase enzymatic method is shown in Figure 1.

## BRIEF DESCRIPTION OF THE DRAWINGS

Figure 1: schematic diagram of the ADC conjugation reaction via the mTGase enzymatic conjugation.
Figures 2A-2B: exemplary structure diagram of the drug conjugated moiety.
Figures 3A-3E: graphs of the results of in vitro plasma stability of enzymatically conjugated samples.
Figure 4: graph of the results of ADC endocytosis assays.
Figure 5: graph of in vivo efficacy results of ADC on H1975 human lung adenocarcinoma xenografts.
Figure 6: graph of in vivo efficacy results of ADC on HCC1187 human breast cancer xenografts.
Figure 7: concentration-time profile of ADC in cynomolgus monkeys after three administrations.
figure 8: concentration-time profile of free small molecule drug in cynomolgus monkeys after three administrations.
Figure 9: concentration-time profile of total antibody.
Figure 10: concentration-time profile of MMAE.

## DETAIL DESCRIPTION OF THE INVENTION

It is understood that the examples and embodiments described herein are for illustrative purposes only, and various modifications or variations thereto are shown to those skilled in the art and will be included within the spirit and scope of this application.

This application uses only a limited number of antibodies and a limited number of drug conjugates as examples to verify the experimental data of the ADC molecules obtained by conjugating the antibody with reactive glutamine to the drug conjugated moiety designed in this application. This application aims to modify the constant region of an antibody to increase reactive glutamine, thereby increasing the drug conjugated moiety that can be conjugated to the antibody. The proposed scheme for increasing the reactive glutamine in the constant region of an antibody is applicable to any antibody targeting any target and any drug conjugated moiety.

### Definition

To make the application easier to understand, certain technical and scientific terms are defined below. Unless otherwise expressly defined herein, all other technical and scientific terms used herein have the meanings commonly understood by one of ordinary skill in the art to which this application pertains. Before describing the application, it should be understood that the application is not limited to the specific methods and experimental conditions described, as such methods and conditions can be varied. It should also be understood that the terminology used herein is intended only to describe particular embodiments and is not intended to be restrictive; the scope of the application will be limited only by the appended claims.

The antibody-drug conjugate (ADC) described in this application refers to a drug conjugated with an antibody that has cytotoxic effects, wherein the antigen of the antibody is expressed on the surface of cancer cells and the antibody also binds to the antigen on the cell surface that enables it to be internalized, and thus can selectively deliver the drug to cancer cells, thereby causing the drug to accumulate in the cancer cells and kill the cancer cells. Targeting ligands, such as

antibodies (e.g., monoclonal antibodies), and antigen-binding fragments are connected to biologically active molecules via chemical linker compounds.

The antibody-drug conjugate (ADC) with high drug load described in this application refers to: modeling the constant region of the antibody through computer-aided drug design; mutating glutamine at sites of solvent accessibility and spatial structure exposure; or changing the amino acids surrounding glutamine in the original antibody to amino acids with smaller spatial structures, thereby increasing glutamine exposure so that glutamine can become a coupling site catalyzed by mTGase, thus obtaining an ADC drug with an increased DAR value; further, the antibody and drug conjugated moiety can be coupled through chemical methods, such as click chemistry achieved by azide-cycloalkyne addition reaction, to further increase the DAR value of the ADC drug; its DAR can be increased to 2 or more, for example, 4, 6, 8, 10, 12, 14 or 16.

The term "linker-drug" refers to the partial structure of an antibody-drug conjugate consisting of a linker compound and a bioactive compound.

As used herein, the terms "comprising," "including," and "containing" are used interchangeably and include not only closed definitions but also semi-closed and open definitions. In other words, the term includes "consisting of ...... " and "substantially composed of ...... ".

As used herein, "HER2" refers to human epidermal growth factor receptor 2. "HER2-positive cancer" refers to cancer in which cancer cells overexpress HER2 compared to non-cancerous normal cells. HER2 status can be determined using known HER2 tests, including immunohistochemistry (IHC), fluorescence in situ hybridization (FISH), subtractive probe technique chromogenic in situ hybridization (SPoT-LightHER2 CISH), and Inform HER2 Dual ISH. HER2-positive cancers include cancers that test 2+ (borderline) or 3+ (positive) on an IHC test. HER2-positive cancers also include cancers that test positive in the HER2FISH test, the SPoT-Light HER2 CISH test, and/or the Inform HER2 Dual ISH test. "HER2 2+ cancer" refers to cancer that tests as 2+ in an IHC test. "HER2 3+ cancer" refers to cancer that tests as 3+ in an IHC test.

As used herein, Nectin is a novel cell adhesion molecule belonging to the immunoglobulin superfamily that can participate in intercellular interactions and form intercellular adhesive junctions without relying on $Ca^{2+}$. The Nectin protein family has four members: Nectin-1, Nectin-2, Nectin-3, and Nectin-4. Nectin 1-3 are widely expressed in normal human tissues. Wherein Nectin-1-2 are commonly found in immune organs (bone marrow, thymus, spleen, and lymph nodes), while Nectin-3 is mainly expressed in the testes and placenta. Unlike other members of the Nectin family, which are widely expressed in normal human tissues, Nectin-4 is expressed at high levels in normal embryonic and fetal tissues, but at very low levels in healthy adult tissues. However, abnormally high expression of Nectin-4 can be detected in various cancer samples. It is involved in the formation of intercellular adhesion junctions, mediates intercellular connections, and participates in the regulation of physiological processes such as cell proliferation, differentiation, and migration. Although the exact molecular mechanisms of tumorigenesis and progression are not yet clear, multiple studies have shown that Nectin-4 can induce angiogenesis, promote cancer cell proliferation and migration, induce epithelial-mesenchymal transition, and is associated with the progression of various cancers. Based on the combined expression patterns and levels of Nectin-4, it has become an attractive target for research on the treatment of various tumor diseases.

ROR1, short for Receptor-tyrosine-kinase-like orphan receptor, is a type I single-transmembrane protein and a member of the receptor tyrosine kinase (RTK) family. It is expressed at low levels during embryonic development but at high levels in various malignant tumors or tissues, such as chronic lymphocytic leukemia (CLL), polar lymphocytic leukemia (ALL), breast cancer, ovarian cancer, melanoma, and lung adenocarcinoma. Extensive data show that ROR1 plays a significant role in promoting tumor growth and metastasis, inducing drug resistance in tumor cells, and inhibiting apoptosis. The human ROR1 molecule consists of an extracellular region, a transmembrane region, and an intracellular region. The extracellular region includes an immunoglobulin-like domain (Ig-like), a cysteine-rich domain or frizzled domain (CRD or FZD), and a Kring (KNG) domain (KRD). The intracellular region comprises a tyrosine kinase domain (TKD), two serine/threonine-rich domains (Ser/ThrD), and a proline-rich domain (PRD). Overexpression of ROR1 can promote tumor growth during cancer progression. ROR1 can bind to Wnt5a, participate in Wnt signal transduction, and interact with signaling pathways such as EGFR and Met, promoting the growth, proliferation, and migration of tumor cells. ROR1 activates AKT/PI3K with CK1ε (casein kinase 1), thereby activating the pCREB pathway and leading to cell proliferation. ROR1 enhances EGFR signaling and induces epithelial-mesenchymal transition (EMT). ROR1 is highly expressed in most hematologic malignancies and solid tumors; for example, the proportion of ROR1+ patients exceeds 90% in CLL patients. In some solid tumors, such as lung cancer, pancreatic cancer, and colorectal cancer cells, ROR1 expression is also significantly increased and closely related to disease progression and treatment efficacy. Therefore, ROR1 can serve as a specific targeted marker for the development of tumor therapeutic drugs.

The term "antibody" is used in the broadest sense and specifically covers monoclonal antibodies (including full-length monoclonal antibodies), multispecific antibodies (e.g., bispecific antibodies), and antibody fragments, provided they exhibit the desired biological activity or function. As used herein, the terms "immunoglobulin" (Ig) and "antibody" are used interchangeably.

As used herein, "full-length antibody" refers to the natural biological form of an antibody, including the variable

region and the constant region of the molecule. For example, in most mammals (including humans and mice), full-length antibodies of the IgG isotype are tetramers and consist of two identical pairs of two immunoglobulin chains, each pair having one light chain (L or LC) and one heavy chain (H or HC). Each light chain comprises an immunoglobulin domain light chain variable region (VL) and a light chain constant region (CL), and each heavy chain comprises an immunoglobulin domain the heavy chain variable region (VH), heavy chain constant regions CH1, CH2, and CH3. In some mammals, such as camels and llamas, IgG antibodies may consist of only two heavy chains, each containing a variable domain attached to the Fc region. Unless otherwise stated, in the embodiments of this application, LC represents light chain and HC represents heavy chain.

As used herein, the "Fc region" refers to a polypeptide that comprises the constant region of the antibody heavy chain and excludes the immunoglobulin domain of the first constant region. For IgG, the Fc region may comprise immunoglobulin domains CH2 and CH3, as well as a hinge between CH1 and CH2.

Unless otherwise stated herein, amino acid residues in the Fc region or constant region are numbered according to the EU numbering system, also known as the EU index, as described in Kabat et al., Sequences of Proteins of Immunological Interest, 5th edition, Public Health Service, National Institutes of Health, Bethesda, MD (1991). Those skilled in the art should understand that, unless otherwise specified, the numbering of amino acid residues in the Fc region or constant region of a given antibody according to the EU numbering system should be understood to cover the numbering of amino acid residues in the corresponding Fc region or constant region as defined by any other numbering system. Although the scope of protection claimed in the claims of this application is based on the definition rules of the EU numbering system, the amino acid numbers corresponding to the definition rules of other numbering systems should also fall within the scope of protection of this application.

The term "constant region" refers to the portion of an immunoglobulin molecule that has a more conserved amino acid sequence compared to the other portions of the immunoglobulin (variable domains) (which comprise antigen-binding sites). The constant domain comprises heavy chain CH1, CH2 and CH3 domains (collectively referred to as CH) and light chain CHL (or CL) domains.

The "variable region" or "variable domain" of an antibody refers to the amino-terminal domain of the heavy or light chain of the antibody. The variable structural domain of a heavy chain can be referred to as "VH". The variable structural domain of a light chain can be referred to as "VL". These domains are typically the most variable portions of an antibody and comprise antigen-binding sites.

As used herein, the term "CDR" or "complementarity-determining region" is intended to refer to a non-neighboring antigen combination site found in the variable region of both heavy and light chain polypeptides. These specific regions have been described in Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273:927-948 (1997); MacCallum et al., J. Mol. Biol. 262:732-745 (1996); Abhinandan and Martin, Mol. Immunol.,45:3832-3839 (2008); Lefranc M. P. et al., Dev. Comp. Immunol., 27:55-77 (2003); and Honegger and Plückthun, J. Mol. Biol., 309:657-670 (2001), wherein the definition, when used in relation to comparisons, includes overlaps or subsets of amino acid residues. CDR prediction algorithms and interfaces are known in the art, including, for example, those described in Abhinandan and Martin Mol. Immunol., 45:3832-3839 (2008); Ehrenmann F. et al., Nucleic Acids Res., 38:D301-D307 (2010); and Adolf-Bryfogle J. et al., Nucleic Acids Res., 43:D432-D438 (2015). The references cited in this paragraph are incorporated herein by reference in their entirety for use in this application and for use in one or more claims that may be included herein.

The term "antigen-binding fragment" used herein refers to an antibody fragment that retains the ability to specifically bind to an antigen. It has been shown that the antigen-binding function of antibodies can be achieved through fragments of full-length antibodies. Examples of binding fragments encompassed by the term "antigen-binding fragment" of antibody include, but are not limited to, Fv, Fab, Fab', Fab'-SH, $F(ab')_2$, Fd, diabody, linear antibody, single-chain antibody molecule (e.g., scFv), and multispecific antibodies formed from antibody fragments. Fab fragment, a monovalent fragment consisting of VL, VH, CL, and CH1 domains; $F(ab')_2$ fragment, a bivalent fragment containing two Fab fragments connected by a disulfide bridge in the hinge region; Fd fragment, consisting of VH and CH1 domains; Fv fragment, consisting of VL and VH domains of the antibody single arm; dAb fragment (Ward et al., 1989 Nature 341: 544-546), consisting of the VH domain; and isolated CDR. Although the two domains VL and VH of the Fv fragment are encoded by separate genes, they can be connected by recombination via synthetic linkers, allowing them to be produced as single protein chains. The VL and VH regions pair to form monovalent molecules (known as single-chain Fv (scFv); see, for example, Bird et al., 1988 Science 242:423-426; Huston et al., 1988 Proc. Natl. Acad. Sci. 85: 5879-5883). In this application, single-chain antibodies are also encompassed in the scope of "antigen-binding fragments". "Diabodies" refer to small antibody fragments with two antigen-binding sites. These fragments comprise a heavy chain variable region (VH) and a light chain variable region (VL) connected together on a polypeptide chain (VH-VL). By using a very short linker, two domains on the same chain cannot pair up and must instead pair up with a complementary domain on another chain, thus forming two antigen-binding sites. A more detailed description of the biantibody can be found in EP 404,097; WO93/11161; and Hollinger et al., Proceedings of the National Academy of Sciences, 90:6444-6448 (1993). Antigen-binding fragments

can be obtained using conventional techniques known to those skilled in the art.

The term "solvent accessibility" is described as access surface area (ASA) or solvent accessibility surface area (SASA), which is the surface area of biomolecules that are accessible to a solvent. ASA measurements are typically described in square angstroms (the standard unit of measurement in molecular biology), and the "rolling ball algorithm" is commonly used to calculate ASA.

"Kabat numbering" and similar terms are recognized in the art and refer to a system for numbering amino acid residues in the variable regions of the heavy and light chains of antibodies or antigen-binding fragments thereof. In certain respects, CDR can be determined according to the Kabat numbering system (see, for example, Kabat EA and Wu TT (1971) Ann NY Acad Sci 190:382-391; and Kabat EA et al. (1991) Sequences of Proteins of Immunological Interest, 5th ed., U.S. Department of Health and Human Services, NIH Publication No. 91-3242). Using the Kabat numbering system, CDRs within antibody heavy chain molecules are typically located at amino acid positions 31 to 35, and the CDRs may optionally include one or two additional amino acids after position 35 (referred to as 35A and 35B in the Kabat numbering scheme) (CDR1); amino acid positions 50 to 65 (CDR2); and amino acid positions 95 to 102 (CDR3). Using the Kabat numbering system, CDRs within the antibody light chain molecule are typically located at amino acid positions 24 to 34 (CDR1), amino acid positions 50 to 56 (CDR2), and amino acid positions 89 to 97 (CDR3). In one specific embodiment, the CDR of the antibody described herein is determined according to the Kabat numbering scheme.

"IMGT numbering": The CDR of an antibody or an antigen-binding fragment thereof can be determined according to the IMGT numbering system described in Lefranc M-P, (1999) The Immunologist 7:132-136 and Lefranc M-P et al. (1999) Nucleic Acids Res 27:209-212. According to the IMGT numbering scheme, VH-CDR1 is located at positions 26 to 35, VH-CDR2 at positions 51 to 57, VH-CDR3 at positions 93 to 102, VL-CDR1 at positions 27 to 32, VL-CDR2 at positions 50 to 52, and VL-CDR3 at positions 89 to 97.

"Chothia numbering": The Chothia CDR-H1 ring appears at positions 26 to 32, 33 or 34 of heavy chain amino acids; the Chothia CDR-H2 ring appears at positions 52 to 56 of heavy chain amino acids; and the Chothia CDR-H3 ring appears at positions 95 to 102 of heavy chain amino acids. The Chothia CDR-L1 ring appears at positions 24 to 34 of light chain amino acids; the Chothia CDR-L2 ring appears at positions 50 to 56 of light chain amino acids; and the Chothia CDR-L3 ring appears at positions 89 to 97 of light chain amino acids.

In the application, "transglutaminase" can be used interchangeably with "TGase" and refers to an enzyme that can carry out transglutaminase reactions. As used herein, the term "transglutamine" refers to a reaction in which the γ-glutamine acyl group of a receptor glutamine residue from a protein/peptide is transferred to an amine group, such as a primary amine or the ε-amino group of lysine. The prior art has disclosed several types of transglutaminases from various living organisms (including microorganisms), such as TGase from guinea pig liver (GTGase), TGase from fish liver (FTGase), and TGase from microorganisms (mTGase), as well as any recombinant TGase (rTGase). Suitable TGases include, but are not limited to, bacterial transglutaminases (BTGs), such as enzymes with EC index number EC 2.3.2.13 (protein-glutamine-γ-glutamyltransferase). In some embodiments, TGase is derived from Streptomyces ladaccatus (TG_SL, SEQ ID NO:35). In some embodiments, TGase is derived from Streptomyces mobara (TG_SM, SEQ ID NO:36). In some embodiments, TGase is a recombinant TGase (TG_SL, SEQ ID NO:37) from Streptomyces ladaccatus.

In some embodiments, the transglutaminase used in the methods described herein is a recombinant protein prepared using recombinant technology.

In some embodiments, the transglutaminase is wild-type, e.g., TGase having the sequence SEQ ID NO:35. In some embodiments, the transglutaminase is a recombinant TGase containing the sequence of SEQ ID NO:36, wherein the recombinant TGase further comprises an additional proline at the N-terminus and an optional purification tag (such as a multihistidine tag). In some embodiments, the transglutaminase is a recombinant TGase having the sequence of SEQ ID NO:37.

In some embodiments, the transglutaminase is engineered. In some embodiments, the transglutaminase is an engineered transglutaminase specifically designed to catalyze transglutaminase reactions at the receptor glutamine site near the N-glycosylation site.

The term "receptor glutamine residue," when referring to amino acid residues of a polypeptide or protein, refers to such glutamine residues that, under suitable conditions, are recognized by TGase and can be cross-linked with a conjugate containing the donor amine via a reaction between TGase and a donor amine (such as lysine or a structurally related primary amine such as aminopentyl).

"Endogenous glutamine" refers to natural glutamine that has not been engineered, e.g., glutamine in variable domains, CDRs, or constant regions.

"Reactive glutamine obtained by ...... " refers to glutamine that has been engineered with antibodies (e.g., by amino acid modification) to become susceptible to, exposed to, or reactive to amine donor reagents. For example, in the presence of transglutaminase, the amide group on reactive glutamine can undergo acyl transfer reactions with other primary amines.

"Reactive glutamine" refers to glutamine that is susceptible to, exposed to, or reactive to amine donor reagents. For example, in the presence of transglutaminase, the amide group on reactive glutamine can undergo acyl transfer

reactions with other primary amines.

"Amino acid modification" refers to a change in the amino acid sequence of a predetermined amino acid sequence. Exemplary modifications include amino acid substitutions, insertions, and/or deletions. The preferred amino acid modifications herein are insertion or substitution. "Amino acid substitution" refers to replacing at least one amino acid residue in a predetermined amino acid sequence with another different "substituted" amino acid residue. "Amino acid insertion" refers to incorporating at least one amino acid into a predetermined amino acid sequence. In this application, "insertion" includes larger "peptide insertions," such as insertions of about three to about five or even up to about ten amino acid residues. "Amino acid deletion" refers to the removal of at least one amino acid from the original amino acid sequence.

"Capital English letter 1 + number + capital English letter 2" used herein indicates that the amino acid residue 1 at the numerical position is substituted by amino acid residue 2. For example, "R355Q" means that the arginine residue at position 355 is substituted by a glutamine residue. In any of the above embodiments, "number + capital English letter 3" indicates that the amino acid residue at the numerical position is substituted by another amino acid residue 3. For example, "355Q" means that the amino acid at position 355 is substituted by a glutamine residue.

"Capital English letter + number" used herein indicates the amino acid residue at the numerical position. For example, "G200" means that the amino acid number (EU numbering) at position 200 is a glycine residue that has not been substituted by other amino acid residues. "Capital English letter 1 + number + capital English letter 1", such as "G200G", means that the amino acid number (EU numbering) 200 is a glycine residue that has not been substituted by other amino acid residues.

In any of the above embodiments, "capital English letter 1 + number 1 + capital English letter 2 -capital English letter 3 + number 2 + capital English letter 4" means that the amino acids at the numerical positions 1 and 2 are mutated simultaneously, wherein the amino acid residue at numerical position 1 is mutated from the amino acid residue represented by capital English letter 1 to the amino acid residue represented by capital English letter 2, and the amino acid residue at numerical position 2 is mutated from the amino acid residue represented by capital English letter 3 to the amino acid residue represented by capital English letter 4. For example, "S354L-R355Q-E356G" means that the serine residue at amino acid position 354 of the antibody is mutated to a leucine residue, the arginine residue at amino acid position 355 is mutated to a glutamine residue, and the glutamic acid residue at amino acid position 356 is mutated to a glycine residue.

The term "354L-355Q-356G (Q438)" or "S354L-R355Q-E356G (Q438)" used herein refers to the fact that when the constant region of an antibody comprises the heavy chain 354L-355Q-356G (or S354L-R355Q-E356G) mutation, the antibody mutation exposes the glutamine spatial position at Q438, making the glutamine at Q438 becoming reactive glutamine. The terms 354L-355Q-356G and 354L-355Q-356G (Q438) have the same meaning; the terms S354L-R355Q-E356G and S354L-R355Q-E356G (Q438) have the same meaning.

The term "Q-containing tag", "a tag containing Q", "having Q tag" or "short peptide having Q tag" refers to an amino acid sequence comprising reactive glutamine residues. When glutamine residues are incorporated (e.g., attached to) into a polypeptide sequence is under suitable conditions, the glutamine residues are recognized by transglutaminase ("TGase") and cross-linked by the reaction between the amino acid side chains and the active groups within the amino acid sequence via TGase. Identification tags can be peptide sequences that are not naturally present in polypeptides. In certain embodiments, the TGase identification tag comprises at least one glutamine. In some embodiments, the TGase identification tag comprises the amino acid sequence XXQX, wherein X is any amino acid or is absent (e.g., conventional amino acids Leu, Ala, Gly, Ser, Val, Phe, Tyr, His, Arg, Asn, Glu, Asp, Cys, Gln, Ile, Met, Pro, Thr, Lys, or Trp, or unconventional amino acids).

In the present application, the 20 common amino acids refer to alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), and tyrosine (Tyr or Y).

The term "UC961-vc-MMAE" is also known as VLS-101. It is an ADC obtained by using the cleavable linker MC-Val-Cit-PAB to conjugate the microtubule polymerization inhibitor MMAE to the cysteine residue of the antibody Cirmtuzumab (UC961), with a DAR mean value of 4. The antibody sequence can be referenced from SEQ ID NO.: 3-6 of CN111587124A.

The term " $NH_2$-$PEG_3$-Val-Cit-pABC " used herein refers to the following structure:

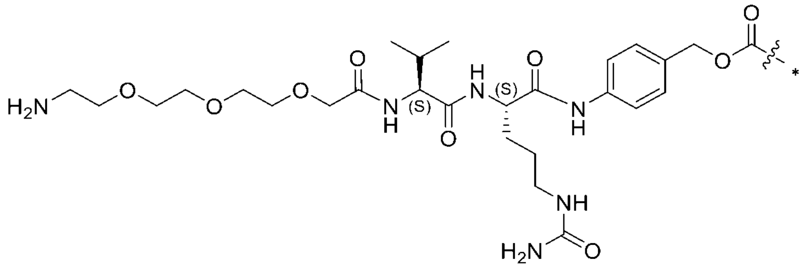

wherein, the position * is connected to a bioactive molecule X, such as MMAE. When it is connected to an antibody, it has the following structure:

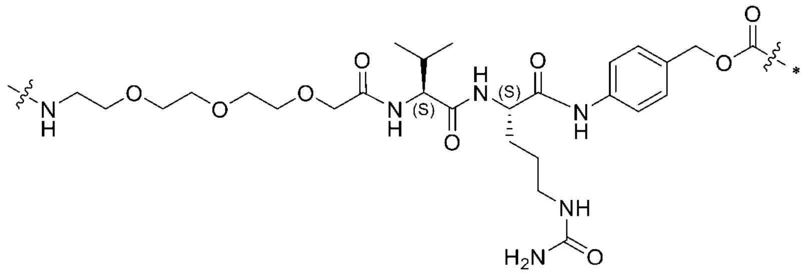

which is connected to the antibody via an amino group (-NH-).

The term "MMAE" refers to methylauratestatin E, which has the following structure:

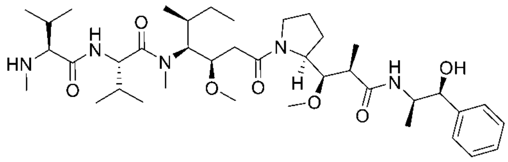

When connected to a linker, it has the following structure:

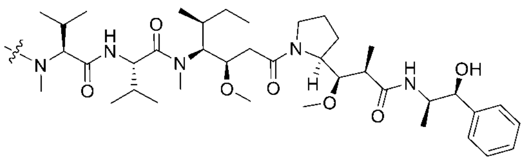

Exemplary routes of administration for ADCs include, but are not limited to, oral, intravenous, intracavitary, intratumoral, intraarterial, intramuscular, subcutaneous, parenteral, transmucosal, transdermal, ocular, topical, intraperitoneal, intracranial, intrapleural, and epidermal routes, or delivery to lymph nodes, body spaces, organs, or tissues known to comprise cancer cells. In some embodiments, the ADC is administered intravenously. In some embodiments, the ADC is administered by infusion. In some embodiments, the ADC is administered by injection.

The conjugated moiety of the ADCs described herein comprises toxins, such as cytotoxic agents that can be used in cancer therapy. In some embodiments, the toxin is a chemotherapeutic agent. In some embodiments, the toxin is a small molecule drug. Examples of cytotoxic agents include, but are not limited to, anthracycline, Auristatin, Dolastatin, CC-1065, Duocarmycin, enediyne, Geldmycin, maytansine, puromycin, taxane, vinca alkaloids, SN-38, tubulysin, hemiasterlin, and stereoisomers, isosteres, analogues or derivatives thereof. In some embodiments, the conjugated moiety comprises monodansyl cadaverine (MDC). In some embodiments, the conjugated moiety comprises TAM1. In some embodiments, the conjugated moiety comprises Monomethyl Auristatin E (MMAE) or derivatives thereof.

Anthracyclines are derived from the bacteria Streptomyces and have been used to treat a wide range of cancers, such as leukemia, lymphoma, breast cancer, uterine cancer, ovarian cancer, and lung cancer. Exemplary anthracyclines include, but are not limited to, daunorubicin, doxorubicin (i.e., adriamycin), epirubicin, idarubicin, penoxorubicin, and mitoxantrone.

Dolastatin and peptide analogs and derivatives thereof, such as Auristatins, are highly effective antimitotic

agents that have been shown to have anticancer and antifungal activities. See, for example, U.S. Patent No. 5,663,149 and Pettet et al., Antimicrob. Agents Chemother. 42:2961-2965 (1998). Exemplary Dolastatins and Auristatins include, but are not limited to, aurestatin E, Auristatin EB (AEB), Auristatin EFP (AEFP), MMAD, MMAF, MMAE, and AE 5-benzoylvalerate (AEVB).

Duocarmycin and CC-1065 are DNA alkylating agents with cytotoxic efficacy. See, Boger and Johnson, PNAS 92:3642-3649 (1995). Exemplary Dolastatins and Auristatins include, but are not limited to, (+)-Duocarmycin A and (+)-Duocarmycin SA and (+)-CC-1065.

Enediynes are a class of antitumor bacterial products, characterized by nine- and ten-membered rings or cyclic systems containing conjugated triple-double-triple bonds. Exemplary enediynes include, but are not limited to, calicheamicin, esperamicin, and dynemicin.

Geldmycin are Benzenic ansamycins antibiotics that bind to Hsp90 (heat shock protein 90) and have been used as antitumor drugs. Exemplary Geldmycins include, but are not limited to, 17-AAG (17-N-allylamino-17-demethoxy Geldmycin) and 17-DMAG (17-dimethylaminoethylamino-17-demethoxy Geldmycin).

Maytansine or derivatives thereof, Maytansine alkaloids, inhibit cell proliferation by suppressing microtubule formation during mitosis through the inhibition of tubulin polymerization. See, Remillard et al., Science 189:1002-1005 (1975). Examples of Maytansine and Maytansine alkaloids include, but are not limited to, mertansine (DM1) and derivatives thereof, as well as anthraquinone.

Taxanes are diterpenoids used as anti-microtubule agents or mitotic inhibitors. Exemplary taxanes include, but are not limited to, paclitaxel (e.g., TAXOL®) and Docetaxel (TAXOTERE®).

Vinca alkaloids are also anti-microtubule protein agents. Exemplary vinca alkaloids include, but are not limited to, vincristine, vinblastine, and vinorelbine.

### Example 1: Point Mutation Design

Computer-aided drug design was used to model the constant region of a HER2-targeting monoclonal IgG1 antibody (DP001). Glutamine mutations were introduced at sites with high solvent accessibility and exposed spatial conformation, or the amino acids surrounding native glutamine residues in the antibody were substituted with amino acids with smaller spatial structures, or short peptides containing glutamine (Q) residues were inserted. These modifications enhance the exposure of glutamine, increase the number of reactive glutamine, and enable these reactive glutamines to serve as conjugation sites catalyzed by mTGase (SEQ ID NO: 35, which were used in all examples), thereby increasing the DAR of the ADC. The point mutations designed in this application are shown in Table 1 below.

Table 1. Point-mutated sequences containing glutamine

| Antibody Mutation Design Name | Original Amino Acid Position (Eu numbering) | Mutation Site (Eu numbering) | Theoretical DAR |
|---|---|---|---|
| AB5 | - | LC: Addition of GGLQSGA at the C-terminus | 4 |
| MFC1 | HC:K288 | HC:K288Q | 4 |
| MFC2 | HC:H310,Q311,D312 | HC:H310L-Q311-D312G | 4 |
| MFC3 | HC:N315 | HC:N315Q | 4 |
| MFC4 | HC:P329 | HC:P329Q | 4 |
| MFC5 | HC:K360,N361,Q362 | HC:K360Q-N361G-Q362A | 4 |
| MFC6 | HC:D399,S400,D401 | HC:D399L-S400Q-D401G | 4 |
| MFC7 | HC:Q418, Q419 | HC:Q418L-Q419 | 4 |
| MFC8 | HC:S354,R355,E356 | HC:S354L-R355Q-E356G | 4 |
| MFC9 | HC:H268,E269,D270 | HC:H268L-E269Q-D270G | 4 |
| MFC10 | HC:M252,1253 | HC:M252Q-1253G | 4 |
| MFC11 | HC:E294,Q295,Y296 | HC:E294L-Q295-Y296G | 4 |
| MFC12 | HC:S444,P445,G446 | HC:S444Q-P445G-G446A | 4 |

(continued)

| Antibody Mutation Design Name | Original Amino Acid Position (Eu numbering) | Mutation Site (Eu numbering) | Theoretical DAR |
|---|---|---|---|
| MFC13 | HC:M252,1253<br>HC:S354,R355,E356 | HC:M252Q-1253G<br>HC:S354L-R355Q-E356G<br>HC: Removal of K and addition of LQSGA at the C-terminus | 8 |
| MFC14 | HC:S354,R355,E356 | HC:S354L-R355Q-E356G<br>LC: Addition of GGLQSGA at the C-terminus | 6 |
| MFC15 | HC:M252,1253 | HC:M252Q-1253G<br>LC: Addition of GGLQSGA at the C-terminus | 6 |
| DP1 | HC:S354,R355,E356<br>HC:H268,E269,D270,P271 | HC:S354L-R355Q-E356G<br>HC:H268L-E269Q-D270G-P271G | 6 |
| DP2 | HC:S354,R355,E356<br>HC:G281 | HC:S354L-R355Q-E356G<br>HC:G281Q | 6 |
| DP3 | HC:S354,R355,E356<br>HC:V282 | HC:S354L-R355Q-E356G<br>HC:V282Q | 6 |
| DP4 | HC:S354,R355,E356<br>HC:E283,V284,H285,N286 | HC:S354L-R355Q-E356G<br>HC:E283L-V284-H285Q-N286G | 6 |
| DP5 | HC:S354,R355,E356<br>HC:H285,N286 | HC:S354L-R355Q-E356G<br>HC:H285L-N286Q | 6 |
| DP6 | HC:S354,R355,E356<br>HC:L309 | HC:S354L-R355Q-E356G<br>HC:L309Q | 6 |
| DP7 | HC:S354,R355,E356<br>HC:N325,K326 | HC:S354L-R355Q-E356G<br>HC:N325L-K326Q | 6 |
| DP8 | HC:S354,R355,E356<br>HC:P329,A330 | HC:S354L-R355Q-E356G<br>HC:P329Q-A330G | 6 |
| DP9 | HC:S354,R355,E356<br>HC:K340,G341,Q342,P343 | HC:S354L-R355Q-E356G<br>HC:K340L-G341L-Q342-P343G | 6 |
| DP10 | HC:S354,R355,E356<br>HC:K360,N361,Q362 | HC:S354L-R355Q-E356G<br>HC:K360L-N361Q-Q362G | 6 |
| DP11 | HC:S354,R355,E356<br>HC:N384,G385,Q386 | HC:S354L-R355Q-E356G<br>HC:N384Q-G385-Q386G | 6 |
| DP12 | HC:S354,R355,E356<br>HC:N3 84,G3 85,Q3 86,P3 87 | HC:S354L-R355Q-E356G<br>HC:N384L-G385Q-Q386G-P387G | 6 |
| DP13 | HC:S354,R355,E356<br>HC:W417,Q418, Q419 | HC:S354L-R355Q-E356G<br>HC:W417L-Q418L-Q419 | 6 |
| DP14 | HC:S354,R355,E356<br>P445,G446,K447 | HC:S354L-R355Q-E356G<br>HC:P445Q-G446 (Removal of K at the C-terminus) | 6 |
| DP21 | HC:P445,G446,K447 | HC:P445Q-G446 (Removal of K at the C-terminus) | 4 |
| DP22 | HC:P445,G446,K447 | HC:P445Q-G446 (Removal of K at the C-terminus)<br>LC: Addition of GGLQSGA at the C-terminus | 6 |

(continued)

| Antibody Mutation Design Name | Original Amino Acid Position (Eu numbering) | Mutation Site (Eu numbering) | Theoretical DAR |
|---|---|---|---|
| DP23 | HC:Q295<br>HC:P445,G446,K447<br>HC:S354,R355,E356 | HC:Q295A<br>HC:P445Q-G446 (Removal of K at the C-terminus)<br>HC:S354L-R355Q-E356G<br>LC: Addition of GGLQSGA at the C-terminus | 6 |
| DP24 | HC:P445,G446,K447<br>HC:S354,R355,E356 | HC:P445Q-G446 (Removal of K at the C-terminus)<br>HC:S354L-R355Q-E356G<br>LC: Addition of GGLQSGA at the C-terminus | 8 |
| DP25 | HC:H285,N286<br>HC:P445,G446,K447 | HC:H285L-N286Q<br>HC:P445Q-G446 (Removal of K at the C-terminus)<br>LC: Addition of GGLQSGA at the C-terminus | 8 |
| DP26 | HC:H268,E269,D270,P271<br>HC:P445,G446,K447 | HC:H268L-E269Q-D270G-P271G<br>HC:P445Q-G446 (Removal of K at the C-terminus)<br>LC: Addition of GGLQSGA at the C-terminus | 8 |
| DP27 | HC:H268,E269,D270,P271<br>HC:S354,R355,E356<br>HC:P445,G446,K447 | HC:H268L-E269Q-D270G-P271G<br>HC:S354L-R355Q-E356G<br>HC:P445Q-G446 (Removal of K at the C-terminus)<br>LC: Addition of GGLQSGA at the C-terminus | 10 |
| DP28 | HC:S354,R355,E356<br>HC:Q438<br>HC:P445,G446,K447 | HC:S354L-R355Q-E356G<br>HC:Q438I<br>HC:P445Q-G446 (Removal of K at the C-terminus)<br>LC: Addition of GGLQSGA at the C-terminus | 6 |
| DP29 | HC:S117,A118,S119,T120<br>HC:S354,R355,E356 | HC:S117L-A118Q-S119G-T120G<br>HC:S354L-R355Q-E356G<br>LC: Addition of GGLQSGA at the C-terminus | 8 |
| DP30 | HC:S354,R355,E356<br>LC: V205,T206,K207,S208 | HC:S354L-R355Q-E356G<br>LC: Addition of GGLQSGA at the C-terminus<br>LC: V205L-T206Q-K207G-S208G | 8 |
| DP31 | HC:S354,R355,E356<br>LC:T109,V110,A111,A112 | HC:S354L-R355Q-E356G<br>LC:T109L-V110Q-A111G-A112G<br>LC: Addition of GGLQSGA at the C-terminus | 8 |
| DP32 | HC:S354,R355,E356<br>LC: K126,S127,G128,T129 | HC:S354L-R355Q-E356G<br>LC:K126L-S127Q-G128-T129G<br>LC: Addition of GGLQSGA at the C-terminus | 8 |
| DP35 | HC:S354,R355,E356 | HC:S354L-R355Q-E356G<br>LC: Addition of GGLQSGA at the C-terminus<br>HC: Insertion of LQSG after T135 | 8 |

(continued)

| Antibody Mutation Design Name | Original Amino Acid Position (Eu numbering) | Mutation Site (Eu numbering) | Theoretical DAR |
|---|---|---|---|
| DP36 | HC:S354,R355,E356 | HC:S354L-R355Q-E356G<br>LC: Addition of GGLQSGA at the C-terminus<br>LC G200-L201-S202 is substituted by LQSG | 8 |

**Example 2: Design and Detection of Short Peptides with Q-containing tags**

Short peptides with Q-containing tags were added to the C-terminus of the heavy chain (HC) of the anti-Nectin 4 antibody Enfortumab (its amino acid sequence is shown in Table 24-1). The modification efficiency of Q on each short peptide with Q-containing tags was compared: EvHcQ1 (K removed from the C-terminus of HC, LQSGA added), EvHcQ2 (K removed from the C-terminus of HC, EEQYGA added), EvHcQ3 (K removed from the C-terminus of HC, EEQYQSG added), and EvHcQ04 (K removed from the C-terminus of HC, LLQGA added). After plasmid construction, antibody expression, and purification (using the same methods as in Examples 3-5 below), LND1002 was conjugated to prepare ADC. After 12 hours of conjugation, the conjugated modification solution was collected and reduced, and the amount of unconjugated antibody was detected by RP-HPLC to reflect the modification efficiency of each Q-containing tag.

RP-HPLC detection method: Dithiothreitol with a final concentration of 20 mM was added to the modification solution, and the resulting solution was incubated in a water bath at 30°C for 30 minutes. The sample with the disulfide bonds between the ADC chains thus cleaved was used for HPLC analysis. The HPLC system selected was e2695 (Waters), the column selected was HiChrome, the column temperature was 60°C, the mobile phase A was 0.1% trifluoroacetic acid (TFA) aqueous solution, and the mobile phase B was 0.1% trifluoroacetic acid (TFA) solution in acetonitrile. The sample loading volume was 50 μL. The gradient program for mobile phase A was as follows: 0-6 min 70%-32%, 6-17 min 68%-65%, 17-20 min 65%-63%, 20-21 min 63%-61%, 21-25 min 61%-60%, 25-28 min 60%-58%, 28-31 min 58%-50%, 31-37 min 50%-10%, 37-38 min 10%-70%, and 38-41 min 70%. In contrast to the unconnected light chains (L0) and heavy chains (H0), the light chains (the light chain connecting a drug, L1) and heavy chains (the heavy chain connecting a drug, H1) connecting drugs were eluted in the order of L0, L1, H0, and H1. The modification efficiency was calculated based on the peak area at 280 nm.

**Table 2. ADC modification efficiency of short peptides with Q-containing tags**

| Name of Anti-Nectin4 Antibody | ADC Name | Short peptide with Q-containing tag | H0+L0 |
|---|---|---|---|
| EvHcQ1 | EvHcQ 1-4LND 1002 | Removal of K and addition of LQSGA at the C-terminus of HC | 1.09% |
| EvHcQ2 | EvHcQ2-4LND 1002 | Removal of K and addition of EEQYGA at the C-terminus of HC | 2.27% |
| EvHcQ3 | EvHcQ3-4LND1002 | Removal of K and addition of EEQYQSG at the C-terminus of HC | 4.55% |
| EvHcQ04 | EvHcQ04-4LND1002 | Removal of K and addition of LLQGA at the C-terminus of HC | 2.86% |

The results show that the proportion of unconjugated H0 and L0 was the lowest at 1.09% for EvHcQ1 antibody after 12 hours of conjugation, 2.27% for EvHcQ2 antibody after 12 hours of conjugation with LND1002, 4.55% for EvHcQ3 antibody after 12 hours of conjugation, and 2.86% for EvHcQ04 antibody after 12 hours of conjugation. The EvHcQ1 antibody conjugated with LND1002 shows the highest modification efficiency, indicating that it has the highest DAR and more uniform ADC.

**Example 3: Expression vector construction and plasmid preparation**

1) PCR cloning a target sequence, followed by gel electrophoresis and gel extraction to obtain a target fragment.
2) Ligating and transforming the target fragment obtained with plasmid vector PCDNA3.4 (comprising an ampicillin

resistance gene) to obtain a recombinant plasmid, transforming the recombinant plasmid into Escherichia coli, and plating on ampicillin-containing plates to screen positive colonies.
3) Randomly selecting 4 positive colonies for sequence verification, expanding the culture of the positive bacterial solution with correct sequencing results, and extracting the plasmid for monoclonal antibody expression.

**Example 4: Monoclonal Antibody Expression**

1) Taking $145\times10^6$ CHO cells and centrifuging to remove the supernatant.
2) Adding 0.5 mL of electroporation buffer to the cells, mixing well, and then adding an appropriate amount of plasmid (concentration: 500 ng/μL).
3) Fully mixing the aforementioned cell-plasmid suspension, taking 1 mL of the mixture to add into an electroporation cuvette, and placing the cuvette into an electroporator for electroporation.
4) After completion of electroporation, aliquoting the cells in the electroporation cuvette into pre-prepared shake flasks containing 20 mL of culture medium, and incubating statically for 40 minutes.
5) Placing the shake flasks in an incubator at 37°C, 270 rpm, and 8% $CO_2$ for culture after incubation; adding feed supplement/sodium butyrate/double antibodies after 24 hours, and continuing the culture for 3-7 days. Sampling for random inspection on the 5th day and sending the samples for ELISA detection.

**Example 5: Monoclonal Antibody Purification**

1) Using an AKTA purification system, and equilibrating the chromatography column: 1xPBS pH 7.0, flow rate 1ml/min, 20ml;
2) Sample loading: Flow rate 1 ml/min;
3) Washing: 1×PBS pH 7.0, flow rate 1 ml/min, 20 ml;
4) Eluting: Sodium acetate buffer (pH 3.4), 1 ml/min, collected in separate tubes, approximately 500 μl per tube. A total of 10 tubes were collected, and the absorbance value at 280 nm was read using a NanoDrop instrument;
5) Dialyzing: Aspirating the high-concentration protein into a dialysis bag and placing it into a beaker containing 1×PBS (pH 7.0) for dialysis.

**Example 6: ADC Conjugation and Purification**

Linker or linker-drug (e.g., LND1002, JSSW038, or Linker1) at 20 molar equivalents relative to the antibody, the antibody (e.g., DP001, Hu17-H2L1, Hu17-H3L1, or Hu17-H4L1, whose amino acid sequences are shown in Tables 24-1 and 24-2), mTGase, and $H_2O$ were separately added into an EP tube. After being sealed and mixed uniformly, the mixture was incubated at 30°C for no more than 4 days, during which mTGase catalyzed the conjugation between the antibody and the linker-toxin to form antibody-drug conjugates. The conjugation reaction was considered completed when the heavy chain conjugation efficiency exceeded 95%, and purification was performed immediately. The purification method of the ADC was the same as that described in Example 5.
Among them, the mTGase is wild-type mTGase, and its amino acid sequence is set forth in SEQ ID NO: 35.

**Example 7: Physicochemical property analysis and identification of ADC**

**1. Determination of DAR value of LND1002 by enzymatic conjugation**

The antibodies used in ADC in Table 3-1 were modified according to the corresponding numbers in Example 1 using the Her2-targeting monoclonal antibody (DP001) as a template, and the drug conjugated moiety was LND1002. ADC was obtained by conjugation and purification according to the method in Example 6. Antibodies with the same name in the following examples were all prepared according to this method. The antibodies used in ADC in Table 3-2 were modified according to the corresponding number in Example 1 using the ROR1-targeting monoclonal antibody (sequence was shown in Table 24-2) as a template, and the drug conjugated moiety was LND1002. ADC was obtained by conjugation and purification according to the method in Example 6.
A chromatographic column (Agilent PLRP-S, 5 μm, 2.1 mm × 50 mm) was selected. Mobile phase A was 0.1% trifluoroacetic acid (TFA) aqueous solution, and mobile phase B was 0.1% TFA solution in acetonitrile. The ratio of mobile phases A and B was adjusted for gradient elution, with a flow rate of 0.25 mL/min, a column temperature of 80°C, and a

detection wavelength of 280 nm. DAR was calculated by the area normalization method, or the intact molecular weight of the ADC was determined by mass spectrometry (MS). The test results are shown in Tables 3-1 and 3-2 below.

Table 3-1 Summary of DAR values for enzymatic conjugation

| ADC name | Original amino acid position (Eu numbering) | Mutation site (Eu numbering) | Theoretical DAR | Actual DAR |
|---|---|---|---|---|
| DP303c | HC:Q295 | No mutations | 2 | 2.0 |
| AB5-ADC | HC:Q295 | LC: Addition of GGLQSGA at the C-terminus | 4 | 3.9 |
| MFC1-ADC | HC:K288 | HC:K288Q | 4 | 2.1 |
| MFC2-ADC | HC:H310,Q311,D312 | HC:H310L-Q311-D312G | 4 | 3.88 |
| MFC3-ADC | HC:N315 | HC:N315Q | 4 | 1.87 |
| MFC4-ADC | HC:P329 | HC:P329Q | 4 | 2.02 |
| MFC5-ADC | HC:K360,N361,G362 | HC:K360Q-N361G-G362A | 4 | 2.01 |
| MFC6-ADC | HC:D399,S400,D401 | HC:D399L-S400Q-D401G | 4 | 4.00 |
| MFC7-ADC | HC:Q418 | HC:Q418L | 4 | 1.95 |
| MFC8-ADC | HC:S354,R355,E356 | HC:S354L-R355Q-E356G | 4 | 3.80 |
| MFC9-ADC | HC:H268,E269,D270 | HC:H268L-E269Q-D270G | 4 | 3.80 |
| MFC10-ADC | HC:M252,1253 | HC:M252Q-1253G | 4 | 3.90 |
| MFC11-ADC | HC:E294,Q295,Y296 | HC:E294L-Q295-Y296G | 4 | 2.00 |
| MFC12-ADC | HC:S444,P445,G446 | HC:S444Q-P445G-G446A | 4 | 3.84 |
| MFC13-ADC | HC:M252,1253<br>HC:S354,R355,E356 | HC:M252Q-1253G<br>HC:S354L-R355Q-E356G<br>HC: Removal of K and addition of LQSGA at the C-terminus | 8 | 7.87 |
| MFC14-ADC | HC:S354,R355,E356 | HC:S354L-R355Q-E356G<br>LC: Addition of GGLQSGA at the C-terminus | 6 | 5.66 |
| MFC15-ADC | HC:M252,1253 | HC:M252Q-1253G<br>LC: Addition of GGLQSGA at the C-terminus | 6 | 5.81 |
| DP1-ADC | HC:S354,R355,E356<br>HC:H268,E269,D270,P271 | HC:S354L-R355Q-E356G<br>HC:H268L-E269Q-D270G-P271G | 6 | 5.03 |
| DP2-ADC | HC:S354,R355,E356<br>HC:G281 | HC:S354L-R355Q-E356G<br>HC:G281Q | 6 | 4.51 |
| DP3-ADC | HC:S354,R355,E356<br>HC:V282 | HC:S354L-R355Q-E356G<br>HC:V282Q | 6 | 5.53 |

(continued)

| ADC name | Original amino acid position (Eu numbering) | Mutation site (Eu numbering) | Theoretical DAR | Actual DAR |
|---|---|---|---|---|
| DP4-ADC | HC:S354,R355,E356<br>HC:E283,V284,H285,N286 | HC:S354L-R355Q-E356G<br>HC:E283L-V284-H285Q-N286G | 6 | 5.16 |
| DP5-ADC | HC:S354,R355,E356<br>HC:H285,N286 | HC:S354L-R355Q-E356G<br>HC:H285L-N286Q | 6 | 5.85 |
| DP6-ADC | HC:S354,R355,E356<br>HC:L309 | HC:S354L-R355Q-E356G<br>HC:L309Q | 6 | 4.43 |
| DP7-ADC | HC:S354,R355,E356<br>HC:N325,K326 | HC:S354L-R355Q-E356G<br>HC:N325L-K326Q | 6 | 4.83 |
| DP8-ADC | HC:S354,R355,E356<br>HC:P329,A330 | HC:S354L-R355Q-E356G<br>HC:P329Q-A330G | 6 | 4.72 |
| DP9-ADC | HC:S354,R355,E356<br>HC:K340,G341,Q342,P343 | HC:S354L-R355Q-E356G<br>HC:K340L-G341L-Q342-P343G | 6 | 5.69 |
| DP10-ADC | HC:S354,R355,E356<br>HC:K360,N361,Q362 | HC:S354L-R355Q-E356G<br>HC:K360L-N361Q-Q362G | 6 | 4.59 |
| DP11-ADC | HC:S354,R355,E356<br>HC:N384,G385,Q386 | HC:S354L-R355Q-E356G<br>HC:N384Q-G385-Q386G | 6 | 4.66 |
| DP12-ADC | HC:S354,R355,E356<br>HC:N3 84,G3 85,Q3 86,P3 87 | HC:S354L-R355Q-E356G<br>HC:N384L-G385Q-Q386G-P387G | 6 | 5.68 |
| DP13-ADC | HC:S354,R355,E356<br>HC:W417,Q418 | HC:S354L-R355Q-E356G<br>HC:W417L-Q418L | 6 | 4.67 |
| DP14-ADC | HC:S354,R355,E356, P445,G446,K447 | HC:S354L-R355Q-E356G, HC:P445Q-G446 (Removal of K at the C-terminus) | 6 | 5.44 |
| DP21-ADC | HC:P445,G446,K447 | HC:P445Q-G446 (Removal of K at the C-terminus) | 4 | 3.85 |
| DP22-ADC | HC:P445,G446,K447 | HC:P445Q-G446 (Removal of K at the C-terminus) LC: Addition of GGLQSGA at the C-terminus | 6 | 5.49 |
| DP23-ADC | HC:Q295<br>HC:P445,G446,K447<br>HC:S354,R355,E356 | HC:Q295A<br>HC:P445Q-G446 (Removal of K at the C-terminus)<br>HC:S354L-R355Q-E356G<br>LC: Addition of GGLQSGA at the C-terminus | 6 | 5.00 |
| DP24-ADC | HC:P445,G446,K447<br>HC:S354,R355,E356 | HC:P445Q-G446 (Removal of K at the C-terminus)<br>HC:S354L-R355Q-E356G<br>LC: Addition of GGLQSGA at the C-terminus | 8 | 7.21 |
| DP25-ADC | HC:H285,N286<br>HC:P445,G446,K447 | HC:H285L-N286Q<br>HC:P445Q-G446 (Removal of K at the C-terminus)<br>LC: Addition of GGLQSGA at the C-terminus | 8 | 7.35 |

(continued)

| ADC name | Original amino acid position (Eu numbering) | Mutation site (Eu numbering) | Theoretical DAR | Actual DAR |
|---|---|---|---|---|
| DP26-ADC | HC:H268,E269,D270,P271<br>HC:P445,G446,K447 | HC:H268L-E269Q-D270G-P271G<br>HC:P445Q-G446 (Removal of K at the C-terminus)<br>LC: Addition of GGLQSGA at the C-terminus | 8 | 7.34 |
| DP27-ADC | HC:H268,E269,D270,P271<br>HC:S354,R355,E356<br>HC:P445,G446,K447 | HC:H268L-E269Q-D270G-P271G<br>HC:S354L-R355Q-E356G<br>HC:P445Q-G446 (Removal of K at the C-terminus)<br>LC: Addition of GGLQSGA at the C-terminus | 10 | 8.81 |
| DP28-ADC | HC:S354,R355,E356<br>HC:Q438<br>HC:P445,G446,K447 | HC:S354L-R355Q-E356G<br>HC:Q438I<br>HC:P445Q-G446 (Removal of K at the C-terminus)<br>LC: Addition of GGLQSGA at the C-terminus | 6 | 5.80 |
| DP29-ADC | HC:S117,A118,S119,T120<br>HC:S354,R355,E356 | HC:S117L-A118Q-S119G-T120G<br>HC:S354L-R355Q-E356G<br>LC: Addition of GGLQSGA at the C-terminus | 8 | 6.84 |
| DP30-ADC | HC:S354,R355,E356<br>LC: V205,T206,K207,S208 | HC:S354L-R355Q-E356G<br>LC: Addition of GGLQSGA at the C-terminus<br>LC: V205L-T206Q-K207G-S208G | 8 | 6.67 |
| DP31-ADC | HC:S354,R355,E356<br>LC:T109,V110,A111,A112 | HC:S354L-R355Q-E356G<br>LC:T109L-V110Q-A111G-A112G<br>LC: Addition of GGLQSGA at the C-terminus | 8 | 7.11 |
| DP32-ADC | HC:S354,R355,E356<br>LC: K126,S127,G128,T129 | HC:S354L-R355Q-E356G<br>LC:K126L-S127Q-G128-T129G<br>LC: Addition of GGLQSGA at the C-terminus | 8 | 7.15 |
| DP33-ADC | HC:S354,R355,E356<br>HC:S134,T135,S136G | HC:S354L-R355Q-E356G<br>HC: S134L-T135Q-S136G<br>LC: Addition of GGLQSGA at the C-terminus | 8 | 6.0 |
| DP34-ADC | HC:S354,R355,E356<br>LC: Q199-G200-L201-S202 | HC:S354L-R355Q-E356G<br>LC: Q199L-G200Q-L201S-S202G<br>LC: Addition of GGLQSGA at the C-terminus | 8 | 6.0 |
| DP35-ADC | HC:S354,R355,E356 | HC:S354L-R355Q-E356G<br>LC: Addition of GGLQSGA at the C-terminus<br>HC: Insertion of LQSG after T135 | 8 | 7.89 |

(continued)

| ADC name | Original amino acid position (Eu numbering) | Mutation site (Eu numbering) | Theoretical DAR | Actual DAR |
|---|---|---|---|---|
| DP36-ADC | HC:S354,R355,E356 | HC:S354L-R355Q-E356G<br>LC: Addition of GGLQSGA at the C-terminus<br>LC: G200-L201-S202 is substituted by LQSG | 8 | 7.93 |

Table 3-2 Summary of DAR values for enzymatic conjugation

| Name of Anti-ROR1 Antibody | ADC Name | Original Amino Acid Position (Eu numbering) | Mutation Site (Eu numbering) | Theoretical DAR | Actual DAR |
|---|---|---|---|---|---|
| Hu17-H2L1 | Hu17-H2L1-4LND10 02 | HC:Q295 | LC: Addition of GGLQSGA at the C-terminus | 4 | 4.0 |
| Hu17-H3L1 | Hu17-H3L1-4LND10 02 | HC:Q295 | LC: Addition of GGLQSGA at the C-terminus | 4 | 4.0 |
| Hu17-H4L1 | Hu17-H4L1-4LND10 02 | HC:Q295 | LC: Addition of GGLQSGA at the C-terminus | 4 | 4.0 |

**2. Determination of DAR value of JSSW038 by enzymatic conjugation**

The antibodies used in ADC in Table 4 were modified according to the corresponding numbers in Example 1 using the Her2-targeting monoclonal antibody (DP001) as a template. The drug conjugated moiety was JSSW038. The ADC was obtained by conjugation and purification according to the method in Example 6. The DAR value was obtained by detection according to the method in Example 7.

Table 4 Summary of DAR values of JSSW038 by enzymatic conjugation

| ADC name | Original amino acid position (Eu numbering) | Mutation site (Eu numbering) | Theoretical DAR | Actual DAR |
|---|---|---|---|---|
| DP001-JSSW038 | HC:Q295 | No mutations | 2 | 1.89 |
| MFC8-JSSW038 | HC:S354,R355,E356 | HC:S354L-R355Q-E356G | 4 | 3.67 |
| DP28-JSSW038 | HC:S354,R355,E356<br>HC:Q438<br>HC:P445,G446,K447 | HC:S354L-R355Q-E356G<br>HC:Q438I<br>HC:P445Q-G446 (Removal of K at the C-terminus)<br>LC: Addition of GGLQSGA at the C-terminus | 6 | 5.78 |
| DP35-JSSW038 | HC:S354,R355,E356 | HC:S354L-R355Q-E356G<br>LC: Addition of GGLQSGA at the C-terminus<br>HC: Insertion of LQSG after T135 | 8 | 8 |

(continued)

| ADC name | Original amino acid position (Eu numbering) | Mutation site (Eu numbering) | Theoretical DAR | Actual DAR |
|---|---|---|---|---|
| DP36-JSSW038 | HC:S354,R355,E356 | HC:S354L-R355Q-E356G<br>LC: Addition of GGLQSGA at the C-terminus<br>LC: G200-L201-S202 is substituted by LQSG | 8 | 8 |

The results are shown in Tables 3-1, 3-2, and 4, indicating ADCs obtained by enzymatic conjugation of some monoclonal antibodies designed with glutamine point mutations can meet the DAR value requirements. In particular, the DAR values of AB5-ADC, MFC2-ADC, MFC6-ADC, MFC8-ADC, MFC8-JSSW038, MFC9-ADC, MFC10-ADC, MFC12-ADC, MFC13-ADC, MFC14-ADC, MFC15-ADC, DP5-ADC, DP9-ADC, DP12-ADC, DP14-ADC, DP22-ADC, DP24-ADC, DP25-ADC, DP26-ADC, DP28-ADC, DP28-JSSW038, DP35-ADC, DP36-ADC, DP35-JSSW038, DP36-JSSW038, Hu17-H2L1-4LND1002, Hu17-H3L1-4LND1002, and Hu17-H4L1-4LND1002 are close to the theoretical DAR value.

**3. Verification of Conjugation Sites**

The ADC sample (the antibody was modified according to the corresponding number in Example 1 using the Her2-targeting monoclonal antibody (DP001) as a template, and the drug conjugated moiety was LND1002, and the ADC was prepared according to Example 6) was enzymatically digested using protease. The digested peptide samples were detected using ultra-high performance liquid chromatography and high-resolution mass spectrometry, and the raw files from the liquid chromatography-mass spectrometry analysis was analyzed using UNIFI software in UPLC-MS/MS to identify the modification sites and related modification intensities of the test sample.

100 μg of the sample was taken, and a guanidine hydrochloride solution was added to a final concentration of 6 M, followed by the addition of 1 M dithiothreitol (DTT) to a final concentration of 20 mM. After mixing uniformly, the mixture was incubated at 37°C for 90 minutes. Upon cooling to room temperature, 1 M iodoacetamide was added to a final concentration of 50 mM, and the mixture was mixed uniformly and reacted at room temperature in the dark for 45 minutes. 2 M urea solution (Tris-HCl, pH 7.5) was added to the upper layer of a 10 kDa ultrafiltration membrane, followed by centrifugation at 13,000 rpm for 10 minutes. The waste liquid was discarded, and the buffer exchange was repeated twice. Trypsin was added at an enzyme-to-protein ratio of 1:25 (w:w), and after mixing uniformly, the mixture was incubated at 37°C for 4 hours. After the reaction was completed, 0.5 μL of FA was added to the enzymatic hydrolysate to terminate the enzymatic digestion reaction. The supernatant was collected by centrifugation for subsequent analysis.

The ultra-high performance liquid chromatograph used was ACQUITY UPLC H-Class PLUS (Waters). The parameters were set as follows: column temperature 50°C, flow rate 0.3 ml/min, detection wavelength 214 nm, mobile phase A: 0.1% formic acid aqueous solution, and mobile phase B: 0.1% formic acid solution in acetonitrile. The high-resolution mass spectrometer used was Vion Q-TOF (Waters) with MSE acquisition mode, capillary voltage (kV) 3V, McSE collision energy 20-45eV, and ion source temperature 120°C. The raw files from liquid chromatography-mass spectrometry (LC-MS) analysis were analyzed using UNIFI software.

The percentage of modified sites is calculated as follows: Percent Q (X)=Area Q (X)/ (Area (X)+Area Q (X))×100%. Note: Percent Q (X) is the percentage of amino acid sites X that are modified.

For the HER2 target, the inventors measured the modification efficiencies of each site of DP303c, AB5-ADC, MFC8-ADC, DP21-ADC, MFC14-ADC, DP22-ADC, DP14-ADC, DP23-ADC, DP28-ADC, DP24-ADC, DP25-ADC, DP26-ADC, and DP27-ADC.

For the Trop2 target, the inventors measured the modification efficiencies of each site of AB5-sacituzumab-ADC, MFC8-sacituzumab-ADC, MFC14-sacituzumab-ADC, DP28-sacituzumab-ADC, DP35-sacituzumab-ADC, and DP36-sacituzumab-ADC.

For the EGFR target, the inventors measured the modification efficiencies of each site of AB5-cexitumab-ADC, MFC8-cexitumab-ADC, MFC14-cexitumab-ADC, DP28-cexitumab-ADC, DP35-cexitumab-ADC and DP36-cexitumab-ADC.

For the Tissue factor (TF) target, the inventors measured the modification efficiencies of each site of AB5-tisotumab-ADC, MFC8-tisotumab-ADC, MFC14-tisotumab-ADC, DP28-tisotumab-ADC, DP35-tisotumab-ADC and DP36-tisotumab-ADC.

For the ROR1 target, the inventors measured the modification efficiencies of each site of AB5-cirmtuzumab-ADC, MFC8-cirmtuzumab-ADC, MFC14-cirmtuzumab-ADC, DP28-cirmtuzumab-ADC, DP35-cirmtuzumab-ADC and

DP36-cirmtuzumab-ADC.

AB5-antibody-ADC indicates the insertion of the GGLQSGA peptide at the C-terminus of LC in the constant region of the antibody; MFC8-antibody-ADC indicates the presence of the following amino acid modification in HC of the constant region of the antibody: S354L-R355Q-E356G; MFC 14-antibody-ADC indicates the insertion of the GGLQSGA peptide at the C-terminus of LC in the constant region of the antibody and the presence of the following amino acid modification in the HC: S354L-R355Q-E356G; DP28-antibody-ADC indicates the insertion of the GGLQSGA peptide at the C-terminus of LC in the constant region of the antibody and the presence of the following amino acid modification in HC: S354L-R355Q-E356G, Q438I, and P445Q-G446; DP35-antibody-ADC indicates the insertion of the GGLQSGA peptide at the C-terminus of LC in the constant region of the antibody and the presence of the following amino acid modification in HC: S354L-R355Q-E356G and LQSG is inserted after T135; DP36-antibody-ADC indicates the insertion of the GGLQSGA peptide at the C-terminus of LC in the constant region of the antibody and the presence of the following amino acid modification in HC: S354L-R355Q-E356G and the presence of the following amino acid modification in LC: G200-L201-S202 was substituted by LQSG peptide.

After being conjugated to an ADC, the modification efficiency of each site was detected, indicating that the modification rate of each coupling site is stable and uniform.

**Table 5 Modification efficiency of each coupling site**

| Name | Modification efficiency of each site (Eu numbering) | | | | | | |
|---|---|---|---|---|---|---|---|
| | LC: Addition of GGLQSG A at the C-terminus | HC:Q295 | HC:S354L-R3 55Q-E356G (Modification at position Q438) | HC:P445Q-G446 (Removal of K at the C-terminus) | HC:H268 L-E269Q-D270G-P 271G | HC:H28 5L-N28 6Q | others |
| DP303c | - | 100% | - | - | - | - | - |
| AB5-ADC | 100% | 99% | - | - | - | - | - |
| MFC8-ADC | - | 98% | 89% | - | - | - | 5.1% |
| DP21-ADC | - | 99% | - | 81% | - | - | 2.28% |
| MFC14-ADC | 100% | 93% | 89% | - | - | - | - |
| DP22-ADC | 100% | 92% | - | 86% | - | - | 1.6% |
| DP14-ADC | - | 99% | 61% | 100% | - | - | 3.5% |
| DP23-ADC | 100% | - | 56% | 99.90% | - | - | - |
| DP28-ADC | 100% | 97% | - | 100% | - | - | - |
| DP24-ADC | 100% | 97% | 40% | 99% | - | - | - |
| DP25-ADC | 100% | 99% | - | 87% | - | 72% | 5.7% |
| DP26-ADC | 100% | 99% | | 89% | 95% | - | 3.4% |
| DP27-ADC | 100% | 98% | 39% | 99% | 88% | - | 0.9% |
| Note: "-" indicates that the mutation to glutamine has not occurred. | | | | | | | |

To determine the conjugation modification efficiency of each mutation site in the ADC after conjugation and

purification, peptide profiling was performed on the enzymatically digested peptide samples. The results are shown in Table 5, which indicate that each mutation site can achieve a high conjugation modification efficiency.

For DAR4 ADCs, i.e., ADC molecules added with one reactive glutamine, such as AB5-ADC (LC: insertion of GGLQSGA at the C-terminus), the modification efficiency of the glutamine in the Q-containing tag in LC (hereinafter referred to as the terminal Q-tag) is 100%, and the modification efficiency of the endogenous glutamine in HC Q295-V296-N297 (hereinafter referred to as Q295) is 99%. For MFC8-ADC (HC: S354L-R355Q-E356G), the modification efficiency at Q295 is 98%, wherein the S354L-R355Q-E356G mutation exposes the spatial position of the glutamine at Q438, enabling the modification of Q438 with a modification efficiency of 89%; for DP21-ADC (HC: P445Q-G446), the modification efficiency at Q295 is 99%, and the modification efficiency at Q445 is 81%.

For DAR6 ADCs, i.e., ADC molecules added with two reactive glutamine, such as MFC14-ADC (HC: S354L-R355Q-E356G and LC: insertion of GGLQSGA at the C-terminus), the modification efficiency of the terminal Q-tag is 100%, the modification efficiency at Q295 is 93%, and the modification efficiency at Q438 is 89%; for DP22-ADC (HC: P445Q-G446 and LC: insertion of GGLQSGA at the C-terminus), the modification efficiency of the terminal Q-tag is 100%; the modification efficiency at Q295 is 92%, and the modification efficiency at 445Q is 86%; for DP14-ADC (HC: S354L-R355Q-E356G and HC: P445Q-G446), the modification efficiency at Q295 is 99%, but the modification efficiency at Q438 decreases to 61%, while the modification efficiency at 445Q is 100%; for DP23-ADC (HC: Q295A, P445Q-G446, S354L-R355Q-E356G and LC: insertion of GGLQSGA at the C-terminus), the modification efficiency of the terminal Q-tag is 100%, the modification efficiency at Q438 is 56%, and the modification efficiency at 445Q is 99.9%. For DP28-ADC (HC: Q295, S354L-R355Q-E356G, Q438I, P445Q-G446 and LC: insertion of GGLQSGA at the C-terminus), the modification efficiency of the terminal Q-tag in LC is 100%, the modification efficiency at Q295 is 97%, and the modification efficiency at 445Q is 100%.

For DAR 8 ADCs, i.e., ADC molecules added with three reactive glutamine, such as DP24-ADC (HC: P445Q-G446, S354L-R355Q-E356G and LC: insertion of GGLQSGA at the C-terminus), the modification efficiency of the terminal Q-tag is 100%, the modification efficiency at Q295 is 97%, the modification efficiency at Q438 is 40%, and the modification efficiency at 445Q is 99%; for DP25-ADC (HC: H285L-N286Q, P445Q-G446 and LC: insertion of GGLQSGA at the C-terminus), the modification efficiency of the terminal Q-tag is 100%, the modification efficiency at Q295 is 99%, the modification efficiency at 445Q is 87%, and the modification efficiency at 286Q is 72%; for DP26-ADC (HC: H268L-E269Q-D270G-P271G, P445Q-G446 and LC: insertion of GGLQSGA at the C-terminus), the modification efficiency of the terminal Q-tag is 100%; the modification efficiency at Q295 is 99%, the modification efficiency at 445Q is 89%, and the modification efficiency at 269Q is 95%.

For DAR 10 ADCs, i.e., ADC molecules added with four reactive glutamine, such as DP27-ADC (HC: H268L-E269Q-D270G-P271G, S354L-R355Q-E356G, P445Q-G446, and LC: insertion of GGLQSGA at the C-terminus), the modification efficiency of the terminal Q-tag is 100%; the modification efficiency at Q295 is 98%, the modification efficiency at Q438 is 39%, the modification efficiency at 445Q is 99%, and the modification efficiency at 269Q is 88%.

In summary, it can be observed that the Q438 sites exposed by the HC P445Q-G446 and HC S354L-R355Q-E356G mutations have a mutual influence on the modification efficiency of conjugated small molecules due to their close spatial proximity. For example, MFC8-ADC and MFC14-ADC both comprise the HC S354L-R355Q-E356G mutation but not the HC P445Q-G446 mutation, and the modification efficiency at Q438 is 89% in both cases. When the HC P445Q-G446 mutation is added based on the HC S354L-R355Q-E356G mutation, such as DP14-ADC, DP24-ADC, and DP27-ADC, due to the presence of HC P445Q-G446, the modification efficiency at Q438 is reduced to 61%, 40%, and 39%, respectively. In addition, for DP21-ADC, DP22-ADC, DP25-ADC, and DP26-ADC, when the HC S354L-R355Q-E356G mutation is absent, the modification efficiency at 445Q is 81%, 86%, 87%, and 89%, respectively. When the HC S354L-R355Q-E356G mutation is added, such as DP14-ADC, DP24-ADC, and DP27-ADC, the modification efficiency at 445Q is increased to 100%, 99%, and 99%, respectively. It is evident that adding the S354L-R355Q-E356G mutation can increase the exposure of positions Q438 and 445Q. However, since Q438 and 445Q are spatially close, their co-existence leads to a decrease in the modification efficiency at Q438. Therefore, DP28 can increase the modification efficiency at the position 445Q by using the Q438I method.

As can be seen, the DAR 4 ADC molecules designed in this application, such as AB5-ADC, MFC8-ADC and DP21-ADC, DAR 6 ADC molecules, such as MFC14-ADC, DP14-ADC, DP22-ADC, DP28-ADC and DP23-ADC, DAR 8 ADC molecules, such as DP24-ADC, DP25-ADC and DP26-ADC, and DAR 10 ADC molecules, such as DP27-ADC, have all achieved multi-site site-specific conjugation, and the glutamine modification efficiency at each mutation site is high, providing a new method for the development of site-specific conjugation ADC drugs with high DAR values.

### 4. Thermal Stability Detection of ADC (DSC)

The thermal stability of ADC samples can be detected by differential scanning calorimetry (DSC). Before detection, the concentration of ADC samples was determined using Nano Drop. The concentration of ADC samples was diluted to approximately 1 mg/mL with 1×PBS pH 7.0 buffer, filtered through a 0.22 μm filter membrane, and 350 μL of

sample was added to each well. The detection was performed using a Malven MicroCal PEAQ-DSC differential scanning calorimetry. The parameters were set as follows: scanning starting temperature: 20°C, scanning end temperature: 100°C, scanning rate: 90°C/h. The results are shown in Table 6.

**Table 6. Results of enzymatically conjugated samples detected by microcalorimetry differential scanning calorimetry (DSC)**

| Name | Mutation site (EU numbering) | DSC | | |
|---|---|---|---|---|
| | | Tonset (°C) | Tm1 (°C) | Tm2 (°C) |
| DP303c | No mutations | 60.61 | 65.16 | 82.30 |
| AB5-ADC | LC: Addition of GGLQSGA at the C-terminus | 59.21 | 64.01 | 82.18 |
| MFC8-ADC | HC: S354L-R355Q-E356G | 53.94 | 62.74 | 81.75 |
| DP21-ADC | HC: P445Q-G446 (Removal of K at the C-terminus) | 60.36 | 66.07 | 81.68 |
| MFC14-ADC | HC: S354L-R355Q-E356G<br>LC: Addition of GGLQSGA at the C-terminus | 54.33 | 60.34 | 81.77 |
| DP22-ADC | HC: P445Q-G446 (Removal of K at the C-terminus)<br>LC: Addition of GGLQSGA at the C-terminus | 58.89 | 63.63 | 81.59 |
| DP14-ADC | HC: S354L-R355Q-E356G<br>HC: P445Q-G446 (Removal of K at the C-terminus) | 54.24 | 61.28 | 81.18 |
| DP23-ADC | HC: Q295A<br>HC: P445Q-G446 (Removal of K at the C-terminus)<br>HC: S354L-R355Q-E356G<br>LC: Addition of GGLQSGA at the C-terminus | 54.91 | 61.30 | 81.21 |
| DP28-ADC | HC: S354L-R355Q-E356G<br>HC: Q438I<br>HC: P445Q-G446 (Removal of K at the C-terminus)<br>LC: Addition of GGLQSGA at the C-terminus | 54.41 | 60.66 | 81.64 |
| DP24-ADC | HC: P445Q-G446 (Removal of K at the C-terminus)<br>HC: S354L-R355Q-E356G<br>LC: Addition of GGLQSGA at the C-terminus | 52.84 | 55.11 | 81.59 |
| DP25-ADC | HC: H285L-N286Q<br>HC: P445Q-G446 (Removal of K at the C-terminus)<br>LC: Addition of GGLQSGA at the C-terminus | 52.82 | 54.47 | 81.5 |
| DP26-ADC | HC: H268L-E269Q-D270G-P271G<br>HC: P445Q-G446 (Removal of K at the C-terminus)<br>LC: Addition of GGLQSGA at the C-terminus | 54.39 | 56.59 | 81.51 |
| DP27-ADC | HC: H268L-E269Q-D270G-P271G<br>HC: S354L-R355Q-E356G<br>HC: P445Q-G446 (Removal of K at the C-terminus)<br>LC: Addition of GGLQSGA at the C-terminus | 50.06 | 55.90 | 81.27 |

The results are shown in Table 6. The Tonset (melting temperature) of each ADC is about 50-60°C, Tm1 (melting temperature 1) is about 55-65°C, and Tm2 (melting temperature 2) is 80-85°C. After the antibody is mutated with glutamine, it is conjugated via mTGase to obtain ADCs with high DAR values. This process does not significantly affect the denaturation temperature of the ADC sample, indicating that the ADC obtained through this method exhibits favorable druggability.

### 5. ADC Thermal Stability Detection (DLS)

The particle size distribution of the ADC sample was detected using a WYATT Dyna Pro Plate Reader III high-throughput automated dynamic and static laser scattering analyzer. Before detection, the sample was slowly mixed evenly, and 80 μL of sample was placed in a 384-well plate. The plate was then placed in the instrument. The parameters were set as follows: Experiment Type: Isothermal, DLS Acquisitions per measurement: 10; DLS Aquisitions Time: 5s; Start Tempperature: 25°C. The results are shown in Table 7.

**Table 7 Results of enzymatically conjugated samples detected by dynamic light scattering (DLS)**

| Name | Mutation site (EU number) | Theoretical DAR | Actual DAR | Radius (nm) |
|---|---|---|---|---|
| DP303c | - | 2 | 2.0 | 5.5 |
| AB5-ADC | LC: Addition of GGLQSGA at the C-terminus | 4 | 3.88 | 5.8 |
| MFC8-ADC | HC: S354L-R355Q-E356G | 4 | 3.80 | 6.2 |
| DP21-ADC | HC: P445Q-G446 (Removal of K at the C-terminus) | 4 | 3.85 | 6.0 |
| MFC14-ADC | HC: S354L-R355Q-E356G<br>LC: Addition of GGLQSGA at the C-terminus | 6 | 5.66 | 4.4 |
| DP22-ADC | HC: P445Q-G446 (Removal of K at the C-terminus)<br>LC: Addition of GGLQSGA at the C-terminus | 6 | 5.49 | 5.9 |
| DP14-ADC | HC: S354L-R355Q-E356G<br>HC: P445Q-G446 (Removal of K at the C-terminus) | 6 | 5.44 | 10.7 |
| DP23-ADC | HC: Q295A<br>HC: P445Q-G446 (Removal of K at the C-terminus)<br>HC: S354L-R355Q-E356G<br>LC: Addition of GGLQSGA at the C-terminus | 6 | 5.00 | 8.7 |
| DP28-ADC | HC: S354L-R355Q-E356G<br>HC: Q438I<br>HC: P445Q-G446 (Removal of K at the C-terminus)<br>LC: Addition of GGLQSGA at the C-terminus | 6 | 5.80 | 6.8 |
| DP24-ADC | HC: P445Q-G446 (Removal of K at the C-terminus)<br>HC: S354L-R355Q-E356G<br>LC: Addition of GGLQSGA at the C-terminus | 8 | 7.21 | 8.6 |
| DP25-ADC | HC: H285L-N286Q<br>HC: P445Q-G446 (Removal of K at the C-terminus)<br>LC: Addition of GGLQSGA at the C-terminus | 8 | 7.35 | 8.5 |
| DP26-ADC | HC: H268L-E269Q-D270G-P271G<br>HC: P445Q-G446 (Removal of K at the C-terminus)<br>LC: Addition of GGLQSGA at the C-terminus | 8 | 7.34 | 7.7 |
| DP27-ADC | HC: H268L-E269Q-D270G-P271G<br>HC: S354L-R355Q-E356G<br>HC: P445Q-G446 (Removal of K at the C-terminus)<br>LC: Addition of GGLQSGA at the C-terminus | 10 | 8.81 | 12.6 |

The results are shown in Table 7. After the antibody was mutated with glutamine, ADCs with different DARs were obtained by mTGase conjugation and DLS detection was performed. The particle size of the ADCs increases slightly, but the increase is not significant and no large particle aggregates are generated, indicating that it has no significant impact on the thermal stability of the samples. This suggests that the ADC samples obtained by this method have good druggability.

**6. Comparison of the thermal aggregation temperatures of antibodies and ADCs**

In this experiment, the ADC Hu17-H2L1-4LND1002 was prepared by modifying the monoclonal IgG1 antibody (Hu17-H2L1) targeting ROR1 according to the corresponding number in Example 1, the drug conjugated moiety was

LND1002, which was prepared according to Example 6.

**The drug conjugated moiety (including the linker) of the positive control UC961-vc MMAE is shown in Formula VIII:**

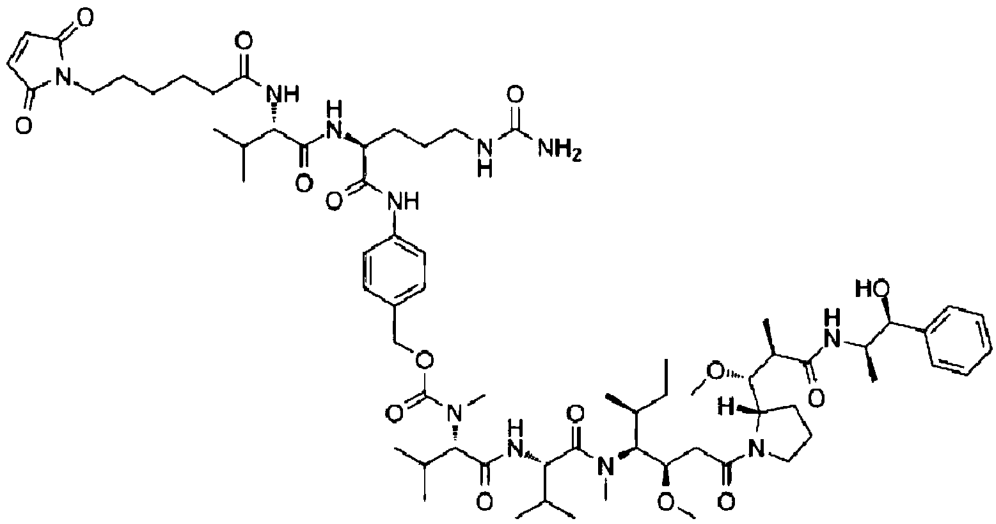

Formula VIII

**The preparation process of UC961-vc MMAE is as follows:**

The UC961 antibody was replaced in PBS 7.0/5mM EDTA buffer to a concentration of 10 mg/mL. 3 molar equivalents of TCEP reducing agent were added according to the antibody molar concentration, and the mixture was heated in a water bath at 37°C for 1 h. Then, 2.5 molar equivalents of ve-MMAE (compound of formula VIII, CAS No.: 646502-53-6) were added, and the reaction was carried out at room temperature for 30 min. Finally, 2.5 molar equivalents of N-acetyl-L-cysteine were added, and the reaction was stopped after 30 min. After the reaction was stopped, the free small molecules and other impurities were removed by ultrafiltration concentration and liquid exchange to obtain UC961-vc MMAE.

The distribution of drug antibody conjugation ratio (DAR) was determined by hydrophobic chromatography-HPLC, and the DAR value of UC961-vc MMAE was calculated to be 4.0. All UC961-vc MMAE used in the following examples were prepared using the method of this example.

The particle size distribution of the samples was detected using a WYATT Dyna Pro Plate Reader III high-throughput automated dynamic and static laser scattering analyzer. The effects of enzymatic conjugation and chemical conjugation on thermal stability were evaluated by the change in Tagg (thermal aggregation temperature). Prior to detection, the sample was gently mixed until homogeneous. 30 μL of the sample was pipetted into a 384-well plate, with precautions taken to avoid introducing foreign particles during the process. After ensuring the absence of air bubbles, the 384-well plate was placed into the instrument for detection. The experimental parameters were set as follows: ① Experiment creation; ② Isothermal incubation at 25°C; ③ DLS acquisition time: 5 seconds; ④ Number of DLS data acquisition cycles: 10. The results are shown in Table 8.

**Table 8 Comparison of the thermal aggregation temperature of antibodies and ADCs**

| Sample Name | Hu17-H2L1 | Hu17-H2L1-4LND1002 | UC961 | UC961-vc MMAE |
|---|---|---|---|---|
| Tagg (°C) | 78.21 | 76.84 | 74.59 | 54.2 |

The results are shown in Table 8. After obtaining an ADC with four LND1002 molecules via enzymatic conjugation for the anti-ROR1 antibody Hu17-H2L1, the Tagg (thermal aggregation temperature) decreases from 78.21°C to 76.84°C, with a reduction of 1.37°C. After obtaining an ADC with four VC MMAE molecules via chemical conjugation for the anti-ROR1 antibody UC961, the thermal aggregation temperature decreases from 74.59°C to 54.2°C, with a reduction of 20.39°C. Enzymatic conjugation of ADCs does not affect the interchain disulfide bonds of the antibody, while chemical conjugation of ADCs disrupts the interchain disulfide bonds of the antibody, resulting in a greater impact on the thermal

stability of the antibody. The ADC sample obtained by enzymatic conjugation has good druggability.

**Example 8: Fab binding activity analysis and identification (Fortebio)**

The affinity of Fab of ADC was detected using bio-layer interferometry (BLI).

Using the Octet RED96e instrument, the AHC capture sensor was used to immobilized different ADC samples at 5 μg/ml; the HER2 antigen was serially diluted, starting from 100 nM, with 2-fold serial dilutions, for a total of 7 gradients. The buffer system was PBS pH 7.4 + 0.02% Tween 20. The steps were set as follows: ① Baseline 60s, ② Loading 800s (antibody immobilization, threshold set to 0.50nm), ③ Baseline 2 120s, ④ Association 120s, ⑤ Dissociation 300s, ⑥ Regeneration 300s (Regenerable Sensor). A blank treatment was performed using a blank buffer, the baseline was aligned with the y-axis, and the data was fitted using Savitzky-Golay in Octet data analysis software. The results are shown in Table 9.

**Table 9. Affinity detection results of enzymatically conjugated samples using Bio-Layer Interferometry (BLI)**

| ADC name | Mutation site (Eu numbering) | kon (1/Ms) | kdis (1/s) | KD (M) |
|---|---|---|---|---|
| DP303c | No mutations | 1.00E+05 | 1.40E-04 | 1.40E-09 |
| AB5-ADC | LC: Addition of GGLQSGA at the C-terminus | 1.14E+05 | 1.61E-04 | 1.41E-09 |
| MFC8-ADC | HC: S354L-R355Q-E356G | 1.08E+05 | 2.49E-04 | 2.30E-09 |
| DP21-ADC | HC: P445Q-G446 (Removal of K at the C-terminus) | 2.99E+05 | 6.04E-05 | 2.02E-10 |
| MFC14-ADC | HC: S354L-R355Q-E356G<br>LC: Addition of GGLQSGA at the C-terminus | 1.08E+05 | 2.49E-04 | 2.30E-09 |
| DP22-ADC | HC: P445Q-G446 (Removal of K at the C-terminus)<br>LC: Addition of GGLQSGA at the C-terminus | 3.06E+05 | 1.08E-04 | 3.55E-10 |
| DP14-ADC | HC: S354L-R355Q-E356G<br>HC: P445Q-G446 (Removal of K at the C-terminus) | 3.26E+05 | 9.25E-05 | 2.84E-10 |
| DP23-ADC | HC: Q295A<br>HC: P445Q-G446 (Removal of K at the C-terminus)<br>HC: S354L-R355Q-E356G<br>LC: Addition of GGLQSGA at the C-terminus | 3.09E+05 | 6.28E-05 | 2.03E-10 |
| DP28-ADC | HC: S354L-R355Q-E356G<br>HC: Q438I<br>HC: P445Q-G446 (Removal of K at the C-terminus)<br>LC: Addition of GGLQSGA at the C-terminus | 3.06E+05 | 8.04E-05 | 2.630E-10 |
| DP24-ADC | HC: P445Q-G446 (Removal of K at the C-terminus)<br>HC: S354L-R355Q-E356G<br>LC: Addition of GGLQSGA at the C-terminus | 3.11E+05 | 1.03E-04 | 3.30E-10 |
| DP25-ADC | HC: H285L-N286Q<br>HC: P445Q-G446 (Removal of K at the C-terminus)<br>LC: Addition of GGLQSGA at the C-terminus | 3.37E+05 | 1.04E-04 | 3.08E-10 |
| DP26-ADC | HC: H268L-E269Q-D270G-P271G<br>HC: P445Q-G446 (Removal of K at the C-terminus)<br>LC: Addition of GGLQSGA at the C-terminus | 3.56E+05 | 7.03E-05 | 1.98E-10 |
| DP27-ADC | HC: H268L-E269Q-D270G-P271G<br>HC: S354L-R355Q-E356G<br>HC: P445Q-G446 (Removal of K at the C-terminus)<br>LC: Addition of GGLQSGA at the C-terminus | 3.78E+05 | 8.50E-05 | 2.25E-10 |

The results are shown in Table 9. After the antibody was mutated to increase the conjugable sites in the constant region, it was catalyzed by mTGase to obtain ADC samples with high DAR values. The affinity of the ADC samples for

HER2 antigen was tested. Compared with the ADC sample DP303c without glutamine mutation, the affinity of the ADC samples with high DAR value for HER2 antigen is not significantly different. This indicates that the glutamine mutation in the constant region followed by enzymatic conjugation to obtain ADC with high DAR value does not affect the affinity between the antibody and the antigen.

**Example 9: In vitro killing activity analysis and identification**

1) Experimental Objective

The purpose of this experiment was to detect the inhibitory activity of the antibody-drug conjugate SK-BR-3 on the in vitro proliferation of tumor cells. Cells were treated in vitro with ADC samples of different concentrations (the ADCs in Table 11 were modified according to the corresponding numbers in Example 1 using Her2-targeting monoclonal IgG1 antibody (DP001) as a template, and the drug conjugated moiety was JSSW038, prepared according to Example 6). After culturing, Resazurin was added, and the fluorescence values at ex550nm/ex610nm were read by a Microplate Reader. The IC50 value was obtained by four-parameter fitting data, thereby calculating the biological activity of the compound.

1) Experimental materials and equipment

Material

| Product Name | Manufacturer/Catalog Number |
|---|---|
| SK-BR-3 Cell Line | ATCC/HTB-30 |
| MTT Assay Kit | Abcam |
| DMEM, High Glucose | Hyclone/SH30022.01 |
| 0.25% Trypsin-EDTA | Gibco/25200-072 |
| Fetal Bovine Serum (FBS) | Gibco/16000-044 |
| 100×Dual Antibodies | Gibco/15240-062 |
| Tissue Culture Plate with Black Walls and Clear Bottoms | Corning/3603 |
| Sodium Resazurin | Sigma Aldrich/199303-25G |

Equipment

| Name | Model | Manufacturer |
|---|---|---|
| Biological Safety Cabinet | 1300 Series, Model A2 | Thermo Fisher Scientific |
| $CO_2$ Incubator | Model 3111 | Thermo Fisher Scientific |
| Inverted Microscope | CKX31 | Olympus Corporation |
| Microplate Reader | Infinite M200 | Tecan Group Ltd. |
| Micro Oscillator | Model MM-I | Shanghai Yarong Biochemical Instrument Factory |

2) Experimental Operation Method

3.1 Culture medium: DMEM, 10% FBS, 1× antibiotics
3.2 Cell Culture: A vial of frozen SK-BR-3 cells was retrieved from liquid nitrogen and thawed for culture in a 75 $cm^2$ culture flask.
3.3 Cell Collection: SK-BR-3 cells were harvested when the culture flask was nearly confluent. The culture medium was discarded, and the cells were washed with PBS to remove dead cells and residual medium. Subsequently, 2-3 mL of 0.25%

Trypsin-EDTA was added, the culture flask was gently shaken, and the cells were digested by incubation at 37°C for 2-3 minutes.

3.4 Cell Density Determination: Cell counting was performed using a hemocytometer under a microscope.

3.5 Assay Plate Seeding:

The cells were diluted with culture medium to a concentration of $1\times10^5$ cells/mL, and 100 μL of the cell suspension was inoculated into each well of the assay plate (excluding rows A and H). For rows A and H, 120 μL of culture medium was added to each well as blank controls. The cells were incubated at 37°C with 5% $CO_2$ for 4-6 hours to allow adherence.

3.6 ADC Sample Dilution:

The initial concentration of the ADC samples was set to 18 μg/mL, followed by 3-fold serial dilution to obtain a total of 11 gradient solutions. Column 12 was used as a blank control.

3.7 Drug Treatment:

20 μL of the ADC sample dilutions from columns 1-11 of the dilution plate was added to the corresponding columns 1-11 of the assay plate, respectively. For column 12 and rows A and H, 20 μL of fresh culture medium was added to each well. The plate was gently shaken on a plate shaker for 10-15 seconds. The ADC conjugated with LND1002 was incubated in the culture plate at 37°C for 3 days, while the ADC conjugated with JSSW038 was incubated at 37°C for 7 days.

3.8 Analysis:

After incubation, 20 μL of 0.03% resazurin (diluted in 1×PBS) was added to each well, and the plate was gently shaken for 10-15 seconds. Following incubation at 37°C for 3-4 hours, absorbance values were measured using a microplate reader. Excel software was used for data plotting and fitting to calculate the $IC_{50}$ values of the reference standard and samples. The unit was ng/mL, and the results were expressed as the percentage of cytotoxicity relative to DP303c. The results are shown in Tables 10 and 11.

Table 10. in vitro killing results of ADC

| ADC name | Mutation site (Eu numbering) | Theoretical DAR | Actual DAR | killing |
|---|---|---|---|---|
| DP303c | No mutations | 2 | 2.0 | 100% |
| AB5-ADC | LC: Addition of GGLQSGA at the C-terminus | 4 | 3.88 | 166% |
| MFC8-ADC | HC: S354L-R355Q-E356G | 4 | 3.80 | 153% |
| DP21-ADC | HC: P445Q-G446 (Removal of K at the C-terminus) | 4 | 3.85 | 154% |
| MFC14-ADC | HC: S354L-R355Q-E356G<br>LC: Addition of GGLQSGA at the C-terminus | 6 | 5.66 | 347% |
| DP22-ADC | HC: P445Q-G446 (Removal of K at the C-terminus)<br>LC: Addition of GGLQSGA at the C-terminus | 6 | 5.49 | 277% |
| DP14-ADC | HC: S354L-R355Q-E356G<br>HC: P445Q-G446 (Removal of K at the C-terminus) | 6 | 5.44 | 211% |
| DP23-ADC | HC: Q295A<br>HC: P445Q-G446 (Removal of K at the C-terminus)<br>HC: S354L-R355Q-E356G<br>LC: Addition of GGLQSGA at the C-terminus | 6 | 5.00 | 265% |
| DP28-ADC | HC: S354L-R355Q-E356G<br>HC: Q438I<br>HC: P445Q-G446 (Removal of K at the C-terminus)<br>LC: Addition of GGLQSGA at the C-terminus | 6 | 5.80 | 287% |
| DP24-ADC | HC: P445Q-G446 (Removal of K at the C-terminus)<br>HC: S354L-R355Q-E356G<br>LC: Addition of GGLQSGA at the C-terminus | 8 | 7.21 | 343% |
| DP25-ADC | HC: H285L-N286Q<br>HC: P445Q-G446 (Removal of K at the C-terminus)<br>LC: Addition of GGLQSGA at the C-terminus | 8 | 7.35 | 251% |
| DP26-ADC | HC: H268L-E269Q-D270G-P271G<br>HC: P445Q-G446 (Removal of K at the C-terminus)<br>LC: Addition of GGLQSGA at the C-terminus | 8 | 7.34 | 356% |

(continued)

| ADC name | Mutation site (Eu numbering) | Theoretical DAR | Actual DAR | killing |
|---|---|---|---|---|
| DP35-ADC | HC:S354L-R355Q-E356G<br>LC: Addition of GGLQSGA at the C-terminus<br>HC: Insertion of LQSG after T135 | 8 | 7.89 | 379% |
| DP36-ADC | HC:S354L-R355Q-E356G<br>LC: Addition of GGLQSGA at the C-terminus<br>LC: G200-L201-S202 is substituted by LQSG | 8 | 7.93 | 374% |
| DP27-ADC | HC: H268L-E269Q-D270G-P271G<br>HC: S354L-R355Q-E356G<br>HC: P445Q-G446 (Removal of K at the C-terminus)<br>LC: Addition of GGLQSGA at the C-terminus | 10 | 8.81 | 465% |

Table 11. ADC in vitro killing results

| ADC name | Mutation site (Eu numbering) | Theoretical DAR | Actual DAR | IC50 (ng/ml) | killing activity |
|---|---|---|---|---|---|
| DP001-JSSW038 | No mutations | 2 | 2.0 | 363.69 | 100% |
| AB5-JSSW038 | LC: Addition of GGLQSGA at the C-terminus | 4 | 3.9 | 205.04 | 177% |
| MFC8-JSSW038 | HC: S354L-R355Q-E356G | 4 | 4.0 | 143.07 | 254% |
| MFC14-JSSW038 | HC: S354L-R355Q-E356G<br>LC: Addition of GGLQSGA at the C-terminus | 6 | 5.7 | 110.03 | 331% |
| DP28-JSSW038 | HC: S354L-R355Q-E356G<br>HC: Q438I<br>HC: P445Q-G446 (Removal of K at the C-terminus)<br>LC: Addition of GGLQSGA at the C-terminus | 6 | 5.8 | 100.54 | 362% |
| DP35-JSSW038 | HC:S354L-R355Q-E356G<br>LC: Addition of GGLQSGA at the C-terminus<br>HC: Insertion of LQSG after T135 | 8 | 7.8 | 56.39 | 645% |
| DP36-JSSW038 | HC:S354L-R355Q-E356G<br>LC: Addition of GGLQSGA at the C-terminus<br>LC: G200-L201-S202 is substituted by LQSG | 8 | 7.9 | 48.73 | 746% |

The results are shown in Table 10. After glutamine mutation, the antibody was conjugated with LND1002 via mTGase to obtain an ADC sample with a high DAR value. As the DAR value increases, the in vitro killing activity of the ADC increases relative to DP303c, indicating that the method can obtain an ADC sample with higher killing activity. The results are shown in Table 11. After glutamine mutation, the antibody was conjugated with JSSW038 via mTGase to obtain an ADC sample with a high DAR value. Compared with DP001-JSSW038 without mutation, the in vitro killing activity of the ADC increases with the increase of DAR value, indicating that the method can obtain an ADC sample with higher killing activity.

**Example 10: Fc Affinity Evaluation (Fortebio)**

The affinity of Fc for FcγR of the ADC was detected using bio-layer interferometry (BLI).

The HISIK capture sensor was used to immobilize 5 μg/ml of FcRn, RcγRIa (CD64), RcγRIIa (CD32A), RcyRIIb (CD32B), RcγRIIIa (F176), or RcγRIIIa (V176). The test sample was serially diluted, starting from 100 nM and then 2-fold, for a total of 7 gradients. The buffer system was PBS pH 7.4 + 0.02% Tween 20. The setup steps were the same as in Example 8.

Detection method of affinity of C1q for antibody: The test sample was coated and serially diluted in a 3-fold gradient starting from an initial concentration of 66.7 μg/mL, a total of 9 gradients. The buffer system was PBS pH 7.4 + 0.1% Tween 20 + 1% BSA. The procedure was set as follows: ① Coating was performed overnight at 4°C; ② 100 μL of 3% non-fat milk powder was added to each well for blocking, and the mixture was incubated with shaking at room temperature for 2 hours; ③ The wells were washed 3 times with PBST, then 100 μL of 5 μg/mL C1q was added to each well, followed by incubation with shaking at room temperature for 2 hours; ④ The wells were washed 4 times with PBST, and 100 μL of 5 μg/mL Shp polyclonal antibody (pAb) to C1q (HRP-conjugated) was added to each well, with shaking at room temperature for 1 hour; ⑤ The wells were washed 4 times with PBST, then 100 μL of TMB substrate solution was added to each well for color development for 10 minutes; ⑥ 100 μL of 2 M $H_2SO_4$ stop solution was added to each well to terminate the reaction; ⑦ The absorbance (OD) values were measured at 450/650 nm using a microplate reader.

**Table 12 Affinity results of enzymatically conjugated samples for FcγRs**

| Name | FcRn | RcγRIa (CD64) | RcγRIIa (CD32A) | RcγRIIb (CD32B) | RcγRIIIa (F176) | RcγRIIIa (V176) | C1q |
|---|---|---|---|---|---|---|---|
| DP303c | 5.77E-08 | 4.28E-09 | 1.42E-06 | 8.59E-06 | 1.47E-06 | 6.81E-06 | 1.78E-07 |
| AB5-ADC | 4.85E-08 | 3.93E-09 | 6.75E-07 | 1.36E-06 | 1.41E-06 | 7.36E-07 | 5.90E-08 |
| MFC8-ADC | 3.48E-08 | 3.48E-09 | 5.86E-07 | 1.49E-06 | 1.34E-06 | 7.24E-07 | 1.12E-07 |
| MFC14-ADC | 3.01E-08 | 4.06E-09 | 9.85E-07 | 2.46E-06 | 2.64E-06 | 1.31E-06 | 8.03E-08 |
| DP28-ADC | 4.20E-08 | 2.83E-09 | 6.38E-07 | 2.18E-06 | 1.01E-06 | 4.78E-07 | 1.24E-08 |
| DP25-ADC | 5.49E-07 | 3.71E-09 | 1.35E-06 | 3.00E-06 | 1.35E-06 | 1.49E-06 | 8.79E-08 |

The results are shown in Table 12. After glutamine mutation, the antibody was catalyzed by mTGase to obtain ADC samples with higher DAR values. There is no significant difference in the affinity of the ADC's Fc region for FcγR receptors (FcRn, CD64, CD32A, CD32B, CD16PHE, CD16VAL, C1q), indicating that the conjugation of small molecules to the antibody does not affect the function of the antibody's Fc region.

**Example 11: In vitro plasma stability**

Rat plasma, human plasma, and PBST (negative control) were used as experimental systems. ADC samples were added to these systems and incubated in vitro at 37°C. The stability of the ADC samples was evaluated by detecting the shedding rate of small molecules. The prepared plasma samples and negative control samples were incubated at 37°C. The reaction was terminated by adding 300 μL of ice-cold precipitant to the incubation system at 0 h, 24 h, 72 h, 96 h, 120 h, 144 h, and 168 h, respectively. The concentration of small molecules in plasma was determined by LC-MS/MS. The detection method was as follows: under light-shielding conditions, 50 μL of the sample to be treated was added to 200 μL of internal standard working solution and vortexed at 2500 rpm for 3 min at room temperature; centrifuged at 12000 rpm for 5 min at 2-8°C; 200 μL of the supernatant was collected at room temperature for analysis. The results are shown in Table 13.

Liquid chromatography conditions: Mobile phase A: 0.1% formic acid aqueous solution; Mobile phase B: 0.1% formic acid solution in acetonitrile; Column: Waters Xselect® HSS T3 2.5 μm, 2.1*50 mm Column; Flow rate: 0.5mL/min; Injection volume: 5μL; Autosampler temperature: 4±5°C; Column temperature: 40±5°C; Elution gradient settings: 0-0.5min: A 85%; 0.5-0.7min: A 85%~45%; 0.7-1.5min: A 45%~5%; 1.5-1.51min: A 5%~85%; 1.51-2min: A 85%;

Mass spectrometry conditions: Ion source: Electrospray Ionization (ESI); Ion mode: Positive ion mode; Detection mode: Multiple reaction monitoring (MRM); Spray voltage (Ion spray voltage): 5500V; Vortex ion spray temperature (Turbo Ion Spray Temp): 550°C; Curtain gas: 35psi; Collision gas: 9psi; Nebulizing gas (Gas1): 60psi; Auxiliary gas (Gas2): 60psi; Data acquisition time: 2.0min.

**Table 13 Results of in vitro plasma stability of enzymatically conjugated samples**

| ADC | DP303c | | | AB5-ADC | | | MFC8-ADC | | | MFC14-ADC | | | DP28-ADC | | |
|---|---|---|---|---|---|---|---|---|---|---|---|---|---|---|---|
| Shedding rate (%) | | | | | | | | | | | | | | | |
| Hour | PBST | Rat | Human | PBST | Rat | Human | PBST | Rat | Human | PBST | Rat | Human | PBST | Rat | Human |
| 0 | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 |
| 24 | 0.04 | 0.02 | 0.04 | 0.03 | 0.02 | 0.02 | 0.03 | 0.03 | 0.02 | 0.04 | 0.04 | 0.03 | 0.02 | 0.03 | 0.02 |
| 72 | 0.10 | 0.05 | 0.07 | 0.06 | 0.08 | 0.05 | 0.04 | 0.12 | 0.05 | 0.06 | 0.11 | 0.07 | 0.04 | 0.08 | 0.04 |
| 96 | 0.07 | 0.05 | 0.11 | 0.09 | 0.08 | 0.08 | 0.07 | 0.12 | 0.08 | 0.10 | 0.11 | 0.12 | 0.07 | 0.08 | 0.07 |
| 120 | 0.12 | 0.12 | 0.13 | 0.10 | 0.13 | 0.10 | 0.07 | 0.19 | 0.10 | 0.15 | 0.24 | 0.14 | 0.08 | 0.18 | 0.08 |
| 144 | 0.17 | 0.17 | 0.15 | 0.14 | 0.18 | 0.11 | 0.11 | 0.26 | 0.13 | 0.22 | 0.32 | 0.20 | 0.11 | 0.25 | 0.10 |
| 168 | 0.20 | 0.20 | 0.21 | 0.15 | 0.21 | 0.15 | 0.12 | 0.36 | 0.11 | 0.31 | 0.43 | 0.24 | 0.13 | 0.32 | 0.12 |

The results are shown in Table 13 and Figures A-E in Figure 3. The x-axis represents the incubation time (h) and the y-axis represents the shedding rate of the toxin molecule. The ADC sample was incubated at 37°C for 168 h, and the shedding rate in human plasma and rat plasma was less than 0.5%, indicating that the ADC sample is stable in plasma. This ADC has better safety and can avoid systemic toxicity and adverse reactions caused by premature release of toxin molecules.

**Example 12: Endocytosis of ADC**

MDA-MB-231 cells stably expressing ROR1 were collected and resuspended in DMEM medium. The resuspended target cells were gently pipetted several times to obtain a single-cell suspension. Cell viability and cell count were identified using trypan blue staining. The cell density was adjusted to $1\times10^5$ cells/mL. 100 μL/well of the solution was inoculated into a 96-well plate, with $1\times10^4$ cells seeded per well. ADCs (Hu17-H2L1-4LND1002, Hu17-H3L1-4LND1002, and Hu17-H4L1-4LND1002) were added to the 96-well plates to a final concentration of 2 μg/mL. The plates were then incubated at 37°C in a 5% $CO_2$ incubator for 27 hours. Cells were harvested at 3, 6, 9, and 27 hours, resuspended, and then treated with fluorescently tagged goat anti-human Alexa Fluro 488 (Thermo). The remaining ADC on the cell surface was detected by flow cytometry, with UC961-vc MMAE as a positive control.

The experimental results are shown in Figure 4. All ADCs (Hu17-H2L1-4LND1002, Hu17-H3L1-4LND1002, Hu17-H4L1-4LND1002) underwent endocytosis in MDA-MB-231-ROR1 cells, and the endocytosis rate of Hu17-H2L1-4LND1002 is superior to that of UC961-vc MMAE.

**Example 13: In vivo efficacy of ADC**

**1. In vivo efficacy of H1975 human lung adenocarcinoma xenografts**

In this experiment, age-appropriate female NUNU mice were inoculated with H1975 cells to construct a human lung adenocarcinoma H1975 xenograft model in nude mice. When the tumor volume reached approximately 100 $mm^3$, 28 animals with good tumor growth were selected and divided into 4 groups (D0) according to tumor volume. A: Solvent control group (n=7), administered 0.9% sodium chloride injection (0.9% INJNS, solvent control group); B: UC961-vc MMAE (n=7); C: Hu17-H2L1-4LND1002 experimental group (n=7); D: Hu17-H3L1-4LND1002 experimental group (n=7). All groups were administered once a week, with administration on D0 and D7, for a total of 2 administrations. The first administration was 3 mg/kg, and the second administration was 4 mg/kg. The mice were weighed after administration, and the data were recorded. The tumor growth was dynamically observed by measuring the tumor diameter at different time points after administration. The experiment ended on day 20. After the mice were asphyxiated with carbon dioxide, the tumors were removed and weighed. The experimental results are shown in Table 14 and Figure 5.

Tumor inhibition rate (TWI) = (Tumor weight in control group - Tumor weight in experimental group) / Tumor weight in control group * 100%

**Table 14 In vivo efficacy of ADC in H1975 human lung adenocarcinoma xenografts**

| Group | Dose | Administration Route | Mean Tumor Volume ($mm^3$) | | Tumor Weight (g) | TWI (%) |
|---|---|---|---|---|---|---|
| | | | D0 | D20 | | |
| 0.9% INJ NS | -- | i.v qw | 107.7 ±22.2 | 1,335.1±285.6 | 1.4188±0. 4344 | -- |
| UC961-vc MMAE | 3mpk (D0) 4mpk (D7) | | 111.7 ±26.9 | 569.5±157.9*** | 0.6956±0. 3240** | 51.0 |
| Hu17-H2L1-4LND10 02 | | | 108.2 ±20.6 | 307.8±152.7*** | 0.4858±0. 2744*** | 65.8 |
| Hu17-H3L1-4LND10 02 | | | 108.4 ±20.2 | 509.1±189.9*** | 0.6570±0. 2272** | 53.7 |
| ***: P<0.001; **: P<0.01; *: P<0.05 | | | | | | |

Under the experimental conditions, the tumor inhibition rates of UC961-vc MMAE, Hu17-H2L1-4LND1002, and Hu17-H3L1-4LND1002 are 51.0%, 65.8%, and 53.7%, respectively. Compared with the solvent control group, all groups significantly inhibit tumor growth. Compared with the positive control UC961-vc MMAE group, the Hu17-H2L1-4LND1002 group shows a more significant inhibitory effect on tumors, while the Hu17-H3L1-4LND1002 group has a comparable inhibitory effect on tumors compared to the positive control UC961-vc MMAE group.

**2. In vivo efficacy in HCC1187 human breast cancer transplant tumors**

In this experiment, age-appropriate female NUNU mice were inoculated with HCC1187 cells to construct a human breast cancer HCC1187 xenograft model in nude mice. When the tumor volume reached approximately 100 $mm^3$, 28 animals with good tumor growth were selected and divided into 4 groups (D0) according to tumor volume. The solvent control group consisted of 7 animals, and the remaining experimental groups consisted of 7 animals. The animals were intravenously administered 0.9% sodium chloride injection (0.9% INJ NS, solvent control group) and positive controls UC961-vc MMAE, Hu17-H2L1-4LND1002, and Hu17-H3L1-4LND1002 (administered on D0, D8, and D12, for a total of 3 administrations). After administration, the mice were weighed and the data was recorded. The growth of the tumor was dynamically observed by measuring the tumor diameter at different times after administration. After the experiment was completed on D14, the mice were asphyxiated with carbon dioxide, and the tumors were removed and weighed. The results are shown in Table 15 and Figure 6.

Table 15. In vivo efficacy of ADC in HCC1187 human breast cancer xenografts

| Group | Dose | Administration Route | Mean Tumor Volume ($mm^3$) | | Tumor Weight (g) | TWI (%) |
|---|---|---|---|---|---|---|
| | | | D0 | D14 | | |
| 0.9% INJ NS | -- | i.v | 99.4±15.2 | 1,015.5±122.6 | 1.4753±0.2926 | -- |
| UC961-vc MMAE | 5mpk (D0, D8, D12) | qw | 99.4±15.4 | 662.0±210.3** | 0.9129±0.2505 ** | 38.1 |
| Hu17-H2L1-4LND1002 | | | 99.3±14.4 | 541.7±270.2** | 0.6538±0.2502 *** | 55.7 |
| Hu17-H3L1-4LND1002 | | | 99.7±14.1 | 626.4±214.0** | 0.7711±0.1918 *** | 47.7 |
| ***: P<0.001; **: P<0.01; *: P<0.05 | | | | | | |

Under the conditions of this experiment, the tumor inhibition rates of UC961-vc MMAE, Hu17-H2L1-4LND1002, and Hu17-H3L1-4LND1002 are 38.1%, 55.7%, and 47.7%, respectively. Compared with the solvent control group, all the experimental groups can inhibit tumor growth. Compared with the positive control UC961-vc MMAE group, the experimental groups Hu17-H2L1-4LND1002 and Hu17-H3L1-4LND1002 show more significant inhibitory effects.

**Example 14: PK Study for Rat**

Male SD rats were divided into 5 groups of 3 rats each and administered 10 mg/kg ADC drug. The levels of total antibodies and MMAE were measured at 0, 0.5, 8, 24, 48, 96 (4d), 168 (7d), and 336 (14d) hours after administration. The concentrations of total antibodies and small molecules in plasma were determined by LC-MS/MS.

The method for detecting small molecule concentration was as described in Example 11, and the method for detecting total antibody was as follows:

Coating: 100 μL/well of the coating working solution was added to a 96-well plate, which was then sealed with a plate sealing film and incubated at 2-8 °C for 16-18 hours.
Washing: 300 μL/well of washing buffer was added to the 96-well plate, the process was repeated 5 times, and the plate was patted dry on absorbent paper.
Blocking: 200 μL of blocking buffer was added to each reaction well of the 96-well plate, followed by static incubation at 37±2 °C for 2 hours ±10 minutes.
Washing: 300 μL/well of washing buffer was added to the 96-well plate, the process was repeated 4 times, and the plate was patted dry on absorbent paper.
Sample Incubation: Processed samples (including blanks and standard curves) were added to the 96-well plate at 100 μL/well, followed by shaking incubation at 450 rpm and 37±2 °C for 2 hours ±10 minutes.
Washing: 300 μL/well of washing buffer was added to the 96-well plate, the process was repeated 4 times, and the plate was patted dry on absorbent paper.
Detection Antibody Incubation: 100 μL/well of the detection antibody working solution was added to the 96-well plate, followed by shaking incubation at 450 rpm and 37±2 °C for 1 hour ±5 minutes.
Washing: 300 μL/well of washing buffer was added to the 96-well plate, the process was repeated 4 times, and the plate was patted dry on absorbent paper.

Streptavidin-HRP Incubation: 100 μL/well of the Streptavidin-HRP working solution was added to the 96-well plate, followed by shaking incubation at 450 rpm and 37±2 °C for 1 hour ±5 minutes.

Washing: 300 μL/well of washing buffer was added to the 96-well plate, the process was repeated 4 times, and the plate was patted dry on absorbent paper.

Color Development and Termination: After TMB was equilibrated to room temperature, it was added to the 96-well plate at 100 μL/well, followed by a light-protected reaction at 25±2 °C for 5-25 minutes. After color development is completed, 50 μL/well of ELISA stop solution was added to terminate the reaction.

Plate Reading: A microplate reader was used, with the detection wavelength set at 450 nm and the reference wavelength at 650 nm. Reading was completed within 10 minutes.

**Table 16 Pharmacokinetic parameters of total antibodies**

| Molecule | | DP303c | | AB5-ADC | | MFC8-ADC | | DP28-ADC | | MFC14-ADC | |
|---|---|---|---|---|---|---|---|---|---|---|---|
| | | MEAN | SD | MEAN | SD | MEAN | SD | MEAN | SD | MEAN | SD |
| t1/2 | (h) | 446.7 | 316.2 | 184 | 123.7 | 143.8 | 31.1 | 49.5 | 1.5 | 69.7 | 1 |
| Cmax | (μg/mL) | 218.4 | 51.1 | 277.9 | 12.5 | 234.8 | 30.1 | 203.1 | 16.9 | 328.5 | 25.1 |
| AUC0-t | (h*μg/mL) | 19772.1 | 2377.3 | 15841 | 1555.4 | 18313.2 | 2815.9 | 5514.9 | 198.4 | 13534.2 | 908.3 |
| AUC0-∞ | (h*μg/mL) | 46021 | 26281.1 | 20349.3 | 4607.5 | 22357.2 | 2048.8 | 5639.4 | 188.5 | 13904.9 | 951.6 |
| Vz | (mL/kg) | 134.2 | 24 | 123.7 | 57.3 | 94.5 | 29.2 | 126.7 | 7.5 | 72.6 | 5.9 |
| CL | (mL/h/kg) | 0.3 | 0.1 | 0.5 | 0.1 | 0.4 | 0 | 1.8 | 0.1 | 0.7 | 0 |
| MRT0-t | (h) | 130.6 | 9.8 | 95.8 | 1.4 | 107.9 | 3.9 | 43.5 | 2.4 | 67.1 | 0.5 |

**Table 17 Pharmacokinetic parameters of MMAE**

| Molecule | | DP303c | | AB5-ADC | | MFC8-ADC | | DP28-ADC | | MFC14-ADC | |
|---|---|---|---|---|---|---|---|---|---|---|---|
| | | MEAN | SD | MEAN | SD | MEAN | SD | MEAN | SD | MEAN | SD |
| t1/2 | (h) | 191.8 | 92.1 | 85.0 | 7.1 | 170.5 | 124.5 | 56.4 | 44.9 | 72.6 | 19.6 |
| Tmax | (h) | 72.0 | 83.1 | 13.3 | 9.2 | 48.2 | 47.8 | 48.0 | 0.0 | 32.0 | 13.9 |
| Cmax | (ng/mL) | 0.136 | 0.089 | 0.183 | 0.033 | 0.345 | 0.098 | 0.426 | 0.044 | 0.535 | 0.147 |
| AUC0-t | (h*ng/mL) | 20.8 | 6.8 | 28.7 | 9.8 | 46.8 | 13.6 | 38.6 | 3.2 | 58.1 | 14.0 |
| AUC0-∞ | (h*ng/mL) | 26.8 | 5.7 | 32.5 | 6.6 | 60.9 | 15.5 | 48.0 | 15.0 | 72.9 | 17.5 |
| Vz | (mL/kg) | 114114715 | 75954723 | 38889668 | 9300991 | 39919221 | 23020043 | 15382043 | 7604704 | 14936943 | 5797318 |
| CL | (mL/h/kg) | 391559 | 86388 | 316488 | 63442 | 173064 | 51443 | 221459 | 63643 | 142352 | 32373 |
| MRT0-t | (h) | 138 | 17 | 95 | 25 | 115 | 13 | 56 | 8 | 75 | 14 |

**Table 18 Percentage of free small molecules in the total small molecules in the ADC**

| | DP303c | AB5-ADC | MFC8-ADC | DP28-ADC | MFC14-ADC |
|---|---|---|---|---|---|
| AUC (MMAE)/AUC (total antibodies) | 0.010996 | 0.009453 | 0.01335 | 0.02434 | 0.01496 |

The results are shown in Tables 16-18 and Figures 9-10. Although the more small molecules are conjugated, the higher the risk of MMAE release, the rat PK results show that even with a DAR of 6, the Cmax of free MMAE is significantly lower than 1 ng/mL, and the $AUC_{(MMAE)}/AUC_{(total\ antibodies)}$ of MMAE in ADCs with different DAR values is lower than 0.025. By mutation or the addition of Q-containing tag, ADC molecules with different DARs are generated via enzymatic conjugation. The level of free small molecules is extremely low, leading to a reduced risk of off-target toxicity, which indicates that this type of molecules has good safety.

In rat PK experiment, the pharmacokinetic parameters of total antibodies show that ADCs with different DAR values exhibit different pharmacokinetic behaviors. ADCs with the same DAR value but different conjugation sites also show differences in pharmacokinetic behavior. Therefore, appropriate conjugation sites and DAR values can be selected based on target characteristics and small molecules to achieve optimal efficacy and safety.

**Example 15: TK Study for Cynomolgus monkeys**

Cynomolgus monkeys were given a single dose of 6 mg/kg each of the test sample Hu17-H2L1-4LND1002 (represented by H2L1-LND in the figure) and the control sample UC961-vc MMAE (represented by VLS in the figure). Blood samples were collected immediately after administration (within 1 min), at 1 h, 4 h, 8 h, 24 h, 48 h, 72 h, 96 h, 120 h, 168 h, 336 h, and 504 h to monitor the levels of ADC and free MMAE. The results are shown in Tables 19 and 20, and Figures 7 and 8.

The concentration of small molecules in plasma was determined by LC-MS/MS. The detection method was as follows: under light-shielding conditions, 50 μL of the sample to be processed was added with 200 μL of internal standard working solution and vortexed at 2500 rpm for 3 min at room temperature, and centrifuged at 12000 rpm for 5 min at 2-8°C. 200 μL of the supernatant was collected at room temperature and injected for analysis. Liquid chromatography conditions: Mobile phase A: 0.1% formic acid aqueous solution; Mobile phase B: 0.1% formic acid solution in acetonitrile; Column: Waters Xselect® HSS T3 2.5 μm , 2.1*50 mm Column; Flow rate: 0.5mL/min; Injection volume: 5μL; Autosampler temperature: 4±5°C; Column temperature: 40±5°C; Elution gradient settings: 0-0.5min: A 85%; 0.5-0.7min: A 85% ~ 45%; 0.7-1.5min: A 45% ~ 5%; 1.5-1.51min: A 5% ~ 85%; 1.51-2min: A 85%; Mass spectrometry conditions: Ion source: Electrospray Ionization (ESI); Ion mode: Positive ion mode; Detection mode: Multiple reaction monitoring (MRM); Spray voltage (Ion Spray Voltage): 5500V; Vortex ion spray temperature (Turbo Ion Spray Temp): 550°C; Curtain Gas: 35psi; Collision Gas: 9psi; Atomizing Gas (Gas1): 60psi; Auxiliary Gas (Gas2): 60psi; Data Acquisition Time: 2.0min.

**Table 19 Pharmacokinetic Parameters of ADC**

| Pharmacokinetic parameters | **Unit** | **UC961-vc MMAE** | | | **H2L1-4LND1002** | | |
|---|---|---|---|---|---|---|---|
| | | D1-6mg/k g | D22-6mg/kg | D43-6mg/kg | D1-6mg/k g | D22-9mg/kg | D43-9mg/kg |
| Cmax | (μg/mL) | 232 | 274 | 194 | 199 | 332 | 289 |
| $AUC_{0-t}$ | (h*μg/mL) | 10939 | 14387 | 12816 | 11144 | 20442 | 22984 |
| $AUC_{0-\infty}$ | (h*μg/mL) | 11155 | 14526 | 13214 | 11192 | 20595 | 23134 |
| CL | (mL/h/kg) | 0.534 | 0.419 | 0.482 | 0.542 | 0.438 | 0.391 |
| MRT0-t | (h) | 103 | 92 | 105 | 93.8 | 104.7 | 102.2 |
| Vss | (mL/kg) | 61.3 | 40 | 54.3 | 51.7 | 47.7 | 41 |
| Tmax | (h) | 0.333 | 0.333 | 0.333 | 0.333 | 0.6665 | 0.333 |
| t1/2 | (h) | 92.8 | 77.7 | 90.8 | 67.2 | 70.6 | 64.9 |
| Note: Cmax: maximum concentration; $AUC_{0-t}$, $AUC_{0-\infty}$: area under the plasma drug concentration-time curve; CL: clearance rate; $MRT_{0-t}$: mean residence time; Vss: volume of distribution; Tmax: time to maximum concentration; $t_{1/2}$: half-life | | | | | | | |

**Table 20 Pharmacokinetic parameters of MMAE**

| Pharmacokinetic parameters | Unit | UC961-vc MMAE | | | H2L1-4LND1002 | | |
|---|---|---|---|---|---|---|---|
| | | D1-6mg/kg | D22-6mg/kg | D43-6mg/kg | D1-6mg/kg | D22-9mg/kg | D43-9mg/kg |
| Cmax | (pg/mL) | 579 | 361 | 361 | 312 | 520 | 496 |
| $AUC_{0-t}$ | (h*pg/mL) | 84752 | 60676 | 62435 | 56156 | 97368 | 94945 |
| $AUC_{0-\infty}$ | (h*pg/mL) | 87233 | 62810 | 63637 | 59377 | 99605 | 97172 |
| CL | (mL/h/kg) | 69476 | 95671 | 95481 | 101165 | 90561 | 92686 |
| $MRT_{0-t}$ | (h) | 127 | 141 | 132 | 136 | 150 | 142 |
| Vss | (mL/kg) | 9774752 | 15279184 | 12542392 | 16692230 | 14639293 | 14323754 |
| Tmax | (h) | 60 | 36 | 36 | 72 | 40 | 26 |
| t1/2 | (h) | 94.8 | 110 | 83.1 | 123 | 96 | 100 |
| Note: Cmax: maximum concentration; $AUC_{0-t}$, $AUC_{0-\infty}$: area under the plasma drug concentration-time curve; CL: clearance rate; $MRT_{0-t}$: mean residence time; Vss: volume of distribution; Tmax: time to maximum concentration; $t_{1/2}$: half-life | | | | | | | |

Tables 19 and 20, and Figures 7 and 8 show that, at the same dose, the exposure levels of H2L1-4LND1002 ADC and UC961-vc MMAE ADC, are comparable, with an exposure ratio of approximately 1.0. At the same dose, the exposure level of the free small molecule H2L1-4LND1002 is lower than that of UC961-vc MMAE, with an exposure ratio of only 0.7 that of UC961-vc MMAE. It can be seen that the exposure level of H2L1-4LND1002 ADC is equivalent to that of UC961-vc MMAEADC, and the exposure level of the free small molecules is lower than that of UC961-vc MMAE, indicating that H2L1-4LND1002 has better safety, and there is no significant accumulation of free small molecules after administration of H2L1-4LND1002.

**Example 16: Safety Evaluation Study for Cynomolgus monkey**

Four age-appropriate cynomolgus monkeys were selected for this experiment. The control innovator drug UC961-vc MMAE and the test sample Hu17-H2L1-4LND1002 were administered via intravenous injection, and the toxic reactions were observed. Both the test sample and the control had a DAR of approximately 4. The dosage design is shown in the table below. The test sample was injected intravenously once every three weeks for three consecutive injections. The dosage of the control UC961-vc MMAE was 6 mg/kg, 6 mg/kg, and 6 mg/kg for the three administrations, respectively. The dosage of the experimental group Hu17-H2L1-4LND1002 was 6 mg/kg, 9 mg/kg, and 9 mg/kg for the three administrations, respectively. Continuous observations were conducted after administration, with general observation twice a day and detailed observation once a day. Weight changes were observed 1 day before administration and weekly after administration, and hematological and blood biochemical indices were tested. The specific arrangements and results of the pre-toxicology tests are shown in Table 21 below.

**Table 21 Pre-toxicology Design and Results**

| | **Group** | First administration (D1) | Second administration (D22) | Third administration (D43) |
|---|---|---|---|---|
| **Dosage** | Group 1: UC961-vc MMAE (in-house prepared) | 6mg/kg | 6mg/kg | 6mg/kg |
| | Group 2: H2L1-4LND1002 | 6mg/kg | 9mg/kg | 9mg/kg |
| **Number of animals** | Two animals/group; Administration frequency Q3W×3 | | | |

(continued)

| | Control group (UC961-ve MMAE) | Experimental group ( Hu17-H2L1-4LND1002) |
|---|---|---|
| **General observation** | Sparse dorsal hair was observed on Day 8 to Day 50 (D8-D50); | Sparse dorsal hair was observed on Day 35 to Day 50 (D35-D50), which gradually recovered thereafter. |
| **Body Weight/Food Intake** | A decrease in food intake and body weight was observed on Day 6 to Day 13 (D6-13) after administration, which gradually recovered thereafter. | No abnormal changes were observed. |
| **Clinical Pathology** | **Hematocyte** For the first administration, in the control group (6 mg/kg), the reduction rates of white blood cells (WBC) and neutrophils (NEUT) were 69% and 98%, respectively; while in the experimental group (6 mg/kg), the reduction rates of WBC and NEUT were 29% and 56%, respectively. The degree of hematocyte reduction in the experimental group was less than that in the control group. Both the control group (administered 6 mg/kg for the first and second administration) and the experimental group (administered 9 mg/kg for the second and third administration ) showed reductions in WBC, NEUT, and red blood cell-related indices (red blood cells [RBC], hemoglobin [HGB], hematocrit [HCT]), with similar reduction degrees. | |
| | **Blood Biochemical Indices** In the control group (after administration of 6 mg/kg), increases in urea (Urea), creatinine (Crea), lactate dehydrogenase (LDH), and triglycerides (TG), as well as a decrease in albumin (Alb), were observed. Prior to the second administration, one animal in the control group still exhibited decreases in red blood cells (RBC), hemoglobin (HGB), and hematocrit (HCT), while all other altered indices had partially recovered. In the experimental group (after administration of 9 mg/kg), a transient increase in LDH and a decrease in Alb were observed, which subsequently returned to normal. | |
| Note: WBC: White blood cell count; NEUT: neutrophil count; RBC: Red blood cell count; HGB: Hemoglobin count; HCT: hematocrit; Urea: urea; Crea: creatinine content; LDH: lactate dehydrogenase; Alb: Albumin; TG: Thyroid globulin; Urea: urea, Crea: creatinine | | |

The safety evaluation experiment results show that the toxicity of ADC Hu17-H2L1-4LND1002 in this application is significantly lower than that of the positive control UC961-vc MMAE.

**Example 17: Conjugation and purification of multivalent ADCs with high drug load**

Linker 1, antibody (DP001, AB5, MFC8, MFC14, DP28, DP35 or DP36), mTGase, and $H_2O$, each equivalent to 20 times the molar equivalent of the antibody, were added to an EP tube. The mTGase catalyzed the conjugation reaction between linker 1 and the glutamine of the antibody. After sealing and mixing, the reaction solution was placed at room temperature for no more than 4 days. When the conjugation efficiency of the heavy chain exceeded 95%, the conjugation reaction was considered complete and the reaction was stopped. Subsequently, free small molecules and other impurities were removed via ultrafiltration and buffer exchange. The ultrafiltration-concentrated ADC intermediate was exchanged into PBS 7.0/5mM EDTA buffer to a concentration of 10 mg/mL. 20 molar equivalents of JSSW-SY (structural formula shown below) were added according to the molar concentration of the antibody. The mixture was sealed, mixed, and placed at room temperature. The reaction was stopped after shaking for 24 h and then purified immediately. The ADC purification method were the same as that in Example 6.

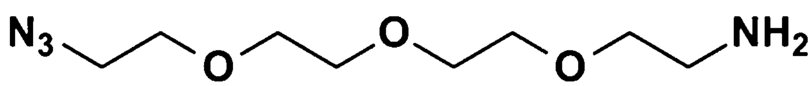

Linker 1

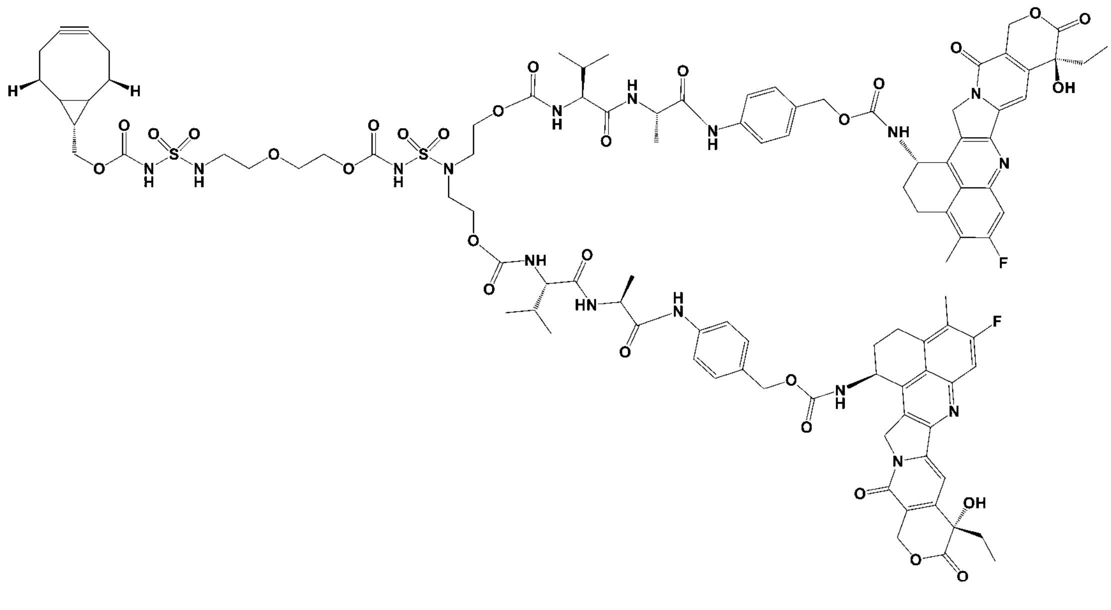

JSSW-SY

**Example 18: Determination of multivalent ADC with high drug load**

The antibodies in the ADC samples in Table 22 were modified according to the corresponding numbers in Example 1 using Her2-targeting monoclonal IgG1 antibody (DP001) as a template, and the drug conjugated moiety was JSSW-SY, which was prepared according to Example 17. The DAR value was detected according to the method in Example 7.

**Table 22 Summary of DAR values for multivalent, high drug-loaded ADCs**

| ADC Name | Mutation site (Eu numbering) | Theoretical DAR | Actual DAR |
|---|---|---|---|
| DP001-JSSW-SY | No mutations | 4 | 4 |
| AB5-JSSW-SY | LC: Addition of GGLQSGA at the C-terminus | 8 | 7.6 |
| MFC8-JSSW-SY | HC: S354L-R355Q-E356G | 8 | 7.2 |
| MFC14-JSSW-SY | HC: S354L-R355Q-E356G<br>LC: Addition of GGLQSGA at the C-terminus | 12 | 10.7 |
| DP28-JSSW-SY | HC: S354L-R355Q-E356G<br>HC: Q438I<br>HC: P445Q-G446 (Removal of K at the C-terminus)<br>LC: Addition of GGLQSGA at the C-terminus | 12 | 11.0 |
| DP35-JSSW-SY | HC:S354L-R355Q-E356G<br>LC: Add GGLQSGA at the C-terminal<br>HC: Insertion of LQSG after T135 | 16 | 14.6 |
| DP36-JSSW-SY | HC:S354L-R355Q-E356G<br>LC: Add GGLQSGA at the C-terminal<br>LC: G200-L201-S202 is substituted by LQSG | 16 | 13.9 |

Compared to Example 6, after enzymatically coupling a fragment with one or more active linkers such as azide, and then chemically coupling an active molecule with one or more conjugates to the aforementioned fragment, the DAR value of the target conjugate in the ADC molecule can be further improved.

**Example 19: Analysis and Identification of Killing Activity of ADC with High Drug Load**

The inhibitory activity of the antibody-drug conjugate SK-BR-3 with high drug load in Example 18on the in vitro proliferation of tumor cells was detected, and the experimental method was the same as in Example 9.

**Table 23 In vitro killing results of ADCs with high drug load**

| ADC Name | Mutation site (Eu numbering) | Theoretical DAR | Actual DAR | IC50 (ng/ml) | Killing activity |
|---|---|---|---|---|---|
| DP001-JSSW-SY | No mutations | 4 | 4 | 99.620 | 100% |
| AB5-JSSW-SY | LC: Addition of GGLQSGA at the C-terminus | 8 | 7.6 | 40.562 | 246% |
| MFC8-JSSW-SY | HC: S354L-R355Q-E356G | 8 | 7.2 | 38.641 | 258% |
| MFC14-JSSW-SY | HC: S354L-R355Q-E356G<br>LC: Addition of GGLQSGA at the C-terminus | 12 | 10.7 | 32.157 | 310% |
| DP28-JSSW-SY | HC: S354L-R355Q-E356G<br>HC: Q438I<br>HC: P445Q-G446 (Removal of K at the C-terminus)<br>LC: Add GGLQSGA at the C-terminal | 12 | 11.0 | 30.535 | 326% |
| DP35-JSSW-SY | HC:S354L-R355Q-E356G<br>LC: Addition of GGLQSGA at the C-terminus<br>HC: Insertion of LQSG after T135 | 16 | 14.6 | 20.712 | 481% |
| DP36-JSSW-SY | HC:S354L-R355Q-E356G<br>LC: Add GGLQSGA at C-terminal<br>LC: G200-L201-S202 is substituted by LQSG substituted by LQSG | 16 | 13.9 | 25.136 | 396% |

The results are shown in Table 23. After glutamine mutation, the antibody was subjected to mTGase catalysis and chemical reaction to obtain ADCs with high drug load. Its killing efficiency is higher than that of the unmutated DP001-JSSW-SY, which improves the in vitro killing effect of the ADC.

**Table 24-1 Antibody-related sequences involved in this application**

| Sequence name | DP001 (anti-HER2 antibody) | Enfortumab (anti-Nectin 4 antibody) |
|---|---|---|
| HCDR1 | DTYIH (SEQ ID NO:1) | SYNMN (SEQ ID NO:11) |
| HCDR2 | RIYPTNGYTRYADSVKG (SEQ ID NO:2) | YISSSSSTIYYADSVKG (SEQ ID NO:12) |
| HCDR3 | WGGDGFYAMDY (SEQ ID NO:3) | AYYYGMDV (SEQ ID NO:13) |
| LCDR1 | RASQDVNTAVA (SEQ ID NO:4) | RASQGISGWLA (SEQ ID NO:14) |
| LCDR2 | SASFLYS (SEQ ID NO:5) | AASTLQS (SEQ ID NO:15) |
| LCDR3 | QQHYTTPPT (SEQ ID NO:6) | QQANSFPPT (SEQ ID NO:16) |

(continued)

| Sequence name | DP001 (anti-HER2 antibody) | Enfortumab (anti-Nectin 4 antibody) |
|---|---|---|
| HV | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSS (SEQ ID NO:7) | EVQLVESGGGLVQPGGSLRLSCAASGFTFSSYNMNWVRQAPGKGLEWVSYISSSSSTIYYADSVKGRFTISRDNAKNSLSLQMNSLRDEDTAVYYCARAYYYGMDVWGQGTTVTVSS (SEQ ID NO:17) |
| LV | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIK (SEQ ID NO:8) | DIQMTQSPSSVSASVGDRVTITCRASQGISGWLAWYQQKPGKAPKFLIYAASTLQSGVPSRFSGSGSGTDFTLTISSLQPEDFATYYCQQANSFPPTFGGGTKVEIK (SEQ ID NO:18) |
| Wild type HC constant region | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:9) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:19) |

(continued)

| Sequence name | DP001 (anti-HER2 antibody) | Enfortumab (anti-Nectin 4 antibody) |
|---|---|---|
| Wild type LC constant region | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:10) | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:20) |

**Table 24-2** Antibody-related sequences involved in this application (anti-ROR1 antibody)

| **Sequence name** | **Hu17-H3L1-4LND1002** | **Hu17-H2L1-4LND1002** | **Hu17-H4L1-4LND1002** |
|---|---|---|---|
| HCDR1 | TSGMGVA (SEQ ID NO:21) | TSGMGVA (SEQ ID NO:21) | TSGMGVA (SEQ ID NO:21) |
| HCDR2 | HIWWDDDKRYNPSLKS (SEQ ID NO:22) | HIWWDDDKRYNPALKS (SEQ ID NO:29) | HIWWDDDKRYGPSLKS (SEQ ID NO:31) |
| HCDR3 | PQFITTVVAFYWYFDV (SEQ ID NO:23) | PQFITTVVAFYWYFDV (SEQ ID NO:23) | PQFITTVVAFYWYFDV (SEQ ID NO:23) |
| LCDR1 | RASENIYSNLA (SEQ ID NO:24) | RASENIYSNLA (SEQ ID NO:24) | RASENIYSNLA (SEQ ID NO:24) |
| LCDR2 | AATNLAD (SEQ ID NO:25) | AATNLAD (SEQ ID NO:25) | AATNLAD (SEQ ID NO:25) |
| LCDR3 | QHFWGTPWT (SEQ ID NO:26) | QHFWGTPWT (SEQ ID NO:26) | QHFWGTPWT (SEQ ID NO:26) |
| HV | QVTLKESGPTLVKPTQTL TLTCSFSGFSLSTSGMGV AWIRQPPGKALEWLAHI WWDDDKRYNPSLKSRL TITKDTSKNQVVLTMTN MDPVDTATYYCARPQFI TTVVAFYWYFDVWGQG TTVTVSS (SEQ ID NO:27) | EVTLKESGPTLVKPTQTL TLTCSFSGFSLSTSGMGV AWIRQPPGKALEWLAHI WWDDDKRYNPALKSRL TITKDTSKNQVVLTITNV DPVDTATYYCARPQFITT VVAFYWYFDVWGQGTT VTVSS (SEQ ID NO:30) | QVTLKESGPTLVKPTQTL TLTCTFSGFSLSTSGMGV AWIRQPPGKALEWLAHI WWDDDKRYGPSLKSRL TITKDTSKNQVVLTMTN MDPVDTATYYCARPQFI TTVVAFYWYFDVWGQG TTVTVSS (SEQ ID NO:32) |
| LV | DILLTQSPSSLSVSVGDRVTITCRASENIYSNLAWYQQKPGKAPKLLVYAATNLADG VPSRFSGSGSGTQYTLTISSLQPEDFATYYCQHFWGTPWTFGGGTKLEIK (SEQ ID NO:28) | | |

(continued)

| Sequence name | Hu17-H3L1-4LND1002 | Hu17-H2L1-4LND1002 | Hu17-H4L1-4LND1002 |
|---|---|---|---|
| HC constant region | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PEAAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALAASIEKTISKAKG QPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK**GGLQSG** **A** (SEQ ID NO:33) | | |
| LC constant region | RTVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC (SEQ ID NO:34) | | |
| Note: Tables 21-1 and 21-2 use the Kabat numbering rules. | | | |

**Table 24-3 Antibody-related sequences involved in this application**

| Sequence name | Sacituzumab | Cexitumab |
|---|---|---|
| HC | QVQLQQSGSELKKPGASVKVSCKASGYTFTNYGMNWVKQAPGQGLKWMGWINTYTGEPTYTDDFKGRFAFSLDTSVSTAYLQISSLKADDTAVYFCARGGFGSSYWYFDVWGQGSLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:40) | QVQLKQSGPGLVQPSQSLSITCTVSGFSLTNYGVHWVRQSPGKGLEWLGVIWSGGNTDYNTPFTSRLSINKDNSKSQVFFKMNSLQSNDTAIYYCARALTYYDYEFAYWGQGTLVTVSAASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:42) |
| LC | DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:41) | DILLTQSPVILSVSPGERVSFSCRASQSIGTNIHWYQQRTNGSPRLLIKYASESISGIPSRFSGSGSGTDFTLSINSVESEDIADYYCQQNNNWPTTFGAGTKLELKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:43) |

**Table 24-4 Antibody-related sequences involved in this application**

| Sequence name | Tisotumab | Cirmtuzumab |
|---|---|---|
| HC | EVQLLESGGGLVQPGGSLRLSCAASGF TFSNYAMSWVRQAPGKGLEWVSSISG SGDYTYYTDSVKGRFTISRDNSKNTLY LQMNSLRAEDTAVYYCARSPWGYYLD SWGQGTLVTVSSASTKGPSVFPLAPSS KSTSGGTAALGCLVKDYFPEPVTVSWN SGALTSGVHTFPAVLQSSGLYSLSSVVT VPSSSLGTQTYICNVNHKPSNTKVDKR VEPKSCDKTHTCPPCPAPELLGGPSVFL FPPKPKDTLMISRTPEVTCVVVDVSHE DPEVKFNWYVDGVEVHNAKTKPREE QYNSTYRVVSVLTVLHQDWLNGKEYK CKVSNKALPAPIEKTISKAKGQPREPQV YTLPPSREEMTKNQVSLTCLVKGFYPS DIAVEWESNGQPENNYKTTPPVLDSDG SFFLYSKLTVDKSRWQQGNVFSCSVMH EALHNHYTQKSLSLSPGK (SEQ ID NO:44) | QVQLQESGPGLVKPSQTLSLTCTVSGY AFTAYNIHWVRQAPGQGLEWMGSFDP YDGGSSYNQKFKDRLTISKDTSKNQVV LTMTNMDPVDTATYYCARGWYYFDY WGHGTLVTVSSASTKGPSVFPLAPSSK STSGGTAALGCLVKDYFPEPVTVSWNS GALTSGVHTFPAVLQSSGLYSLSSVVTV PSSSLGTQTYICNVNHKPSNTKVDKKV EPKSCDKTHTCPPCPAPELLGGPSVFLF PPKPKDTLMISRTPEVTCVVVDVSHED PEVKFNWYVDGVEVHNAKTKPREEQ YNSTYRVVSVLTVLHQDWLNGKEYKC KVSNKALPAPIEKTISKAKGQPREPQVY TLPPSRDELTKNQVSLTCLVKGFYPSDI AVEWESNGQPENNYKTTPPVLDSDGSF FLYSKLTVDKSRWQQGNVFSCSVMHE ALHNHYTQKSLSLSPGK (SEQ ID NO:46) |
| LC | DIQMTQSPPSLSASAGDRVTITCRASQG ISSRLAWYQQKPEKAPKSLIYAASSLQS GVPSRFSGSGSGTDFTLTISSLQPEDFAT YYCQQYNSYPYTFGQGTKLEIKRTVAA PSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC (SEQ ID NO:45) | DIVMTQTPLSLPVTPGEPASISCRASKSI SKYLAWYQQKPGQAPRLLIYSGSTLQS GIPPRFSGSGYGTDFTLTINNIESEDAAY YFCQQHDESPYTFGEGTKVEIKRTVAA PSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC (SEQ ID NO:47) |
| | UC961 (Monoclonal antibodies targeting ROR1) | |

(continued)

| Sequence name | Tisotumab | Cirmtuzumab |
|---|---|---|
| HC | QVQLQESGPGLVKPSQTLSLTCTVSGYAFTAYNIHWVRQAPGQGLEWMGSFDPYDGGSSYNQKFKDRLTISKDTSKNQVVLTMTNMDPVDTATYYCARGWYYFDYWGHGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:38) | |
| LC | DIVMTQTPLSLPVTPGEPASISCRASKSISKYLAWYQQKPGQAPRLLIYSGSTLQSGIPPRFSGSGYGTDFTLTINNIESEDAAYYFCQQHDESPYTFGEGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:39) | |

**Table 24-5 mTGase-related sequences involved in this application**

| Sequence name | Amino acid sequence |
|---|---|
| wild type TG_SL | DSDERVTPPAEPLDRMPDPYRPSYGRAETIVNNYIRKWQQVYSHRDGRKQQMTEEQREWLSYGCVGVTWVNSGQYPTNRLAFAFFDEDKYKNELKNGRPRSGETRAEFEGRVAKDSFDEAKGFQRARDVASVMNKALENAHDEGAYLDNLKKELANGNDALRNEDARSPFYSALRNTPSFKDRNGGNHDPSKMKAVIYSKHFWSGQDRSGSSDKRKYGDPEAFRPDRGTGLVDMSRDRNIPRSPTSPGESFVNFDYGWFGAQTEADADKTVWTHGNHYHAPNGSLGAMHVYESKFRNWSDGYSDFDRGAYVVTFVPKSWNTAPDKVTQGWP (SEQ ID NO:35) |
| wild type TG_SM | DSDDRVTPPAEPLDRMPDPYRPSYGRAETVVNNYIRKWQQVYSHRDGRKQQMTEEQREWLSYGCVGVTWVNSGQYPTNRLAFASFDEDRFKNELKNGRPRSGETRAEFEGRVAKESFDEEKGFQRAREVASVMNRALENAHDESAYLDNLKKELANGNDALRNEDARSPFYSALRNTPSFKERNGGNHDPSRMKAVIYSKHFWSGQDRSSSADKRKYGDPDAFRPAPGTGLVDMSRDRNIPRSPTSPGEGFVNFDYGWFGAQTEADADKTVWTHGNHYHAPNGSLGAMHVYESKFRNWSEGYSDFDRGAYVITFIPKSWNTAPDKVKQGWP (SEQ ID NO:36) |
| Recombinant type TG_SL | PDSDERVTPPAEPLDRMPDPYRPSYGRAETIVNNYIRKWQQVYSHRDGRKQQMTEEQREWLSYGCVGVTWVNSGQYPTNRLAFAFFDEDKYKNELKNGRPRSGETRAEFEGRVAKDSFDEAKGFQRARDVASVMNKALENAHDEGAYLDNLKKELANGNDALRNEDARSPFYSALRNTPSFKDRNGGNHDPSKMKAVIYSKHFWSGQDRSGSSDKRKYGDPEAFRPDRGTGLVDMSRDRNIPRSPTSPGESFVNFDYGWFGAQTEADADKTVWTHGNHYHAPNGSLGAMHVYESKFRNWSDGYSDFDRGAYVVTFVPKSWNTAPDKVTQGWPLEHHHHHHHH (SEQ ID NO:37) |

**Table 24-6 Sequences of short peptides with Q-containing tag**

| Peptide sequence | Sequence number |
|---|---|
| GGLQSGA | SEQ ID NO:48 |
| LQSGA | SEQ ID NO:49 |
| LQSG | SEQ ID NO:50 |
| EEQYGA | SEQ ID NO:51 |
| EEQYQSG | SEQ ID NO:52 |
| LLQGA | SEQ ID NO:53 |

## Claims

1. An antibody-drug conjugate with high drug load, comprising an antibody or an antigen-binding fragment thereof and a drug conjugated moiety, wherein a constant region of the antibody or an antigen-binding fragment thereof comprises one or more reactive glutamine, and the reactive glutamine, based on EU numbering rules, is selected from the group consisting of:

   (i) a glutamine in a Q-containing tag selected from the group consisting of: GGLQSGA, LQSGA, EEQYGA and EEQYQSG, and the Q-containing tag is located at carboxyl terminus of a light chain, heavy chain, or both the light chain and the heavy chain;
   (ii) a reactive glutamine obtained by amino acid modification, which is located in an amino acid sequence selected from the group consisting of: heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q;
   (iii) a reactive glutamine obtained by amino acid modification, which is located in an amino acid sequence selected from the group consisting of: light chain 126L-127Q-G128-129G, light chain 205L-206Q-207G-208G, light chain 109L-110Q-111G-112G and light chain 199L-200Q-201S-202G;
   (iv) an endogenous glutamine in heavy chain Q295; and
   (v) a glutamine in the Q-containing tag LQSG that replaces light chain G200-L201-S202, and glutamine in the Q-containing tag LQSG that inserts after heavy chain T135.

2. An engineered antibody, wherein a constant region of the antibody comprises one or more reactive glutamine, and the reactive glutamine, based on EU numbering rules, is selected from the group consisting of:

   (i) a glutamine in a Q-containing tag selected from the group consisting of: GGLQSGA, LQSGA, EEQYGA and EEQYQSG, and the Q-containing tag is located at carboxyl terminus of light chain, heavy chain, or both the light chain and the heavy chain;
   (ii) a reactive glutamine obtained by amino acid modification, which is located in an amino acid sequence selected from the group consisting of: heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q;
   (iii) a reactive glutamine obtained by amino acid modification, which is located in an amino acid sequence selected from the group consisting of: light chain 126L-127Q-G128-129G, light chain 205L-206Q-207G-208G, light chain 109L-110Q-111G-112G and light chain 199L-200Q-201S-202G;
   (iv) an endogenous glutamine in heavy chain Q295; and
   (v) a glutamine in the Q-containing tag LQSG that replaces the light chain G200-L201-S202, and glutamine in the Q-containing tag LQSG that inserts after the heavy chain T135.

3. The antibody-drug conjugate of claim 1 or the antibody of claim 2, wherein the reactive glutamine comprises heavy chain Q295 endogenous glutamine and one or more reactive glutamine selected from the group consisting of:

   (1) a glutamine in a Q-containing tag selected from the group consisting of: GGLQSGA, LQSGA, EEQYGA and EEQYQSG, and the Q-containing tag is located at the carboxyl terminus of the light chain;
   (2) a glutamine in the Q-containing tag LQSG that replaces the light chain G200-L201-S202;
   (3) a glutamine in the Q-containing tag LQSG that inserts after the heavy chain T135; and
   (4) a reactive glutamine obtained by amino acid modification, which is located in an amino acid sequence selected from the group consisting of: heavy chain 354L-355Q-356G (Q438), heavy chain 445Q-G446, and heavy chain 268L-269Q-270G-271G; preferably, the heavy chain Q295 endogenous glutamine is the endogenous glutamine in heavy chain Q295-V296-N297.

4. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the light chain; and the endogenous glutamine in the heavy chain Q295-V296-N297.

5. The antibody-drug conjugate or antibody of any one of claims 1-3, wherein the reactive glutamine comprises: the reactive glutamine obtained by amino acid modification in the heavy chain 354L-355Q-356G (Q438); and the endogenous glutamine in the heavy chain Q295-V296-N297.

6. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the light chain; the endogenous glutamine in the heavy chain Q295-V296-N297; and the reactive glutamine obtained by amino acid modification in the heavy chain 354L-355Q-356G (Q438).

7. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the light chain; the endogenous glutamine in the heavy chain Q295-V296-N297; and the reactive glutamines obtained by amino acid modification in the heavy chain 354L-355Q-356G (Q438) and heavy chain 445Q-G446; preferably, it further comprises amino acid modification of heavy chain Q438I.

8. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the light chain; the endogenous glutamine the in heavy chain Q295-V296-N297; the reactive glutamine obtained by amino acid modification in the heavy chain 354L-355Q-356G (Q438); and the glutamine in the Q-containing tag LQSG that inserts after the heavy chain T135.

9. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the light chain; the endogenous glutamine in the heavy chain Q295-V296-N297; the reactive glutamine obtained by amino acid modification in the heavy chain 354L-355Q-356G (Q438); and the glutamine in the Q-containing tag LQSG that replaces the light chain G200-L201-S202.

10. The antibody-drug conjugate or antibody of any one of claims 1-9, wherein the reactive glutamine comprises: the reactive glutamines obtained by amino acid modification in the heavy chain 354L-355Q-356G (Q438) and heavy chain 445Q-G446; preferably, the constant region of the antibody or an antigen-binding fragment thereof further comprises 4381 amino acid modification.

11. The antibody-drug conjugate or antibody of any one of claims 1-10, wherein the reactive glutamine comprises: the reactive glutamine obtained by amino acid modification in the heavy chain 445Q-G446; and K is removed from the C-terminus of the heavy chain.

12. The antibody-drug conjugate or antibody of any one of claims 1-3, wherein the reactive glutamine comprises: a glutamine in a Q-containing tag selected from the group consisting of: GGLQSGA, LQSGA, EEQYGA and EEQYQSG, and the Q-containing tag is located at the carboxyl terminus of the heavy chain and/or light chain; preferably, the heavy chain is first subjected to K removal, followed by conjugation with the Q-containing tag.

13. The antibody-drug conjugate or antibody of any one of claims 1-12, wherein the antibody is an IgG1 antibody.

14. The antibody-drug conjugate of any one of claims 1 and 3-13, wherein the antibody moiety and the drug conjugated moiety are connected by a linker containing an amino group, and the linker is covalently connected to reactive glutamine in the antibody via the amino group.

15. The antibody-drug conjugate of any one of claims 1 and 3-14, having the structure: A-(NH-L-X)d, wherein A is the antibody or an antigen-binding fragment thereof of any one of claims 1-14, -NH-L- is a linker, X is a drug molecule, and d is 1-16, preferably 1-12, more preferably 1-8, such as 1, 2, 3, 4, 5, 6, 7, or 8, including integers and decimals.

**16.** The antibody-drug conjugate of any one of claims 1 and 3-15, wherein the antibody moiety and the drug conjugated moiety are connected by a linker, and the linker is a cleavable linker or a non-cleavable linker; when the linker is a cleavable linker, the cleavable linker includes, but is not limited to, $NH_2$-$(CH_2CH_2O)_n$-Val-Cit-PABC-X and $NH_2$-$(CH_2CH_2O)_n$-(Val-Cit-PABC-X)$_2$, wherein X is a drug molecule, and n is an integer of at least 1 (such as any one of 2, 3, 4, 6, 8, 10, 12, 16, 20 or 24), PABC refers to p-aminobenzyloxycarbonyl, Val refers to valine, and Cit refers to citrulline; when the linker is a non-cleavable linker, the non-cleavable linker includes, but is not limited to, $NH_2$-R-X, $NH_2$NH-R-X and $NH_2$-O-R-X, wherein R is an alkyl or polyethylene glycol (PEG) group, X is a drug molecule, and the polyethylene glycol group can have the general formula $-(CH_2CH_2O)_n-$, wherein n is an integer of at least 1 (such as any one of 2, 4, 6, 8, 10, 12, 16, 20 or 24).

**17.** The antibody-drug conjugate or antibody of any one of claims 1-16, wherein the antibody is a monoclonal antibody, polyclonal antibody, human antibody, humanized antibody, chimeric antibody, microantibody, or bispecific antibody, or an antigen-binding fragment thereof.

**18.** The antibody-drug conjugate or antibody of any one of claims 1-17, wherein both the heavy chains and/or both light chains of the antibody are inserted with the Q-containing tag or contain the amino acid modification; preferably, the Q-containing tag or the amino acid modification is the same in both the heavy chains and/or both the light chains.

**19.** The antibody-drug conjugate of any one of claims 1 and 3-18, wherein the drug molecule of the drug conjugated moiety is an anticancer drug, including but not limited to cytotoxic drugs, immune enhancers or radioisotopes;

preferably, the cytotoxic drug is selected from the group consisting of: microtubule inhibitors, DNA topoisomerase inhibitors, DNA damaging agents, immune enhancers, antimetabolites, and antitumor antibiotics;
more preferably, the microtubule inhibitor is selected from the group consisting of: Auristatin derivatives (e.g., MMAE (Monomethyl Auristatin E), MMAF (Monomethyl Auristatin F)) and maytansine alkaloid derivatives (e.g., DM1, DM4, Ansamitocin, Mertansine, Dolastatin, or derivatives thereof);
the DNA topoisomerase inhibitors are selected from the group consisting of: camptothecin analogs, and other DNA topoisomerase I inhibitors, or derivatives thereof, including but not limited to DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyeclipticine, topotecan, letopotecan, Belotecan, Exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenyl methyl)-(2E)-2-acrylamide, 2-cyano-3-(3,4-dihydroxyphenyl)-N-(3-hydroxyphenylpropyl)-(E)-2-acrylamide, 12-β-D-glucopyranosyl-12,13-dihydro-2,10-dihydroxy-6-[[2-hydroxy-1-(hydroxymethyl)ethyl]amin o]-5H-indolo[2,3-a]pyrrolo[3,4-c]carbazole-5,7(6H)-dione, N-[2-(dimethylamino)ethyl]-4-acridinecarboxamide dihydrochloride, N-[2-(dimethylamino)ethyl]-4-acridinecarboxamide and derivatives thereof;
the DNA damaging agent is selected from the group consisting of: calicheamicin, duocarmycin, and anthramycin derivative PBD (pyrrolobenzodiazepine);
the immune enhancer is selected from the group consisting of: levamisole, pidotimod, imiquimod, isoprinosine, polyinosinic-polycytidylic acid, and polyinosinic-uridine acid;
the antimetabolite is selected from the group consisting of: methotrexate, 6-mercaptopurine and 5-fluorouracil;
the radioisotope is selected from the group consisting of: $^{211}At$, $^{131}I$, $^{125}I$, $^{90}Y$, $^{186}Re$, $^{188}Re$, $^{153}Sm$, $^{212}Bi$, $^{32}P$, $^{60}Co$ and $^{177}Lu$;
the antitumor antibiotic is selected from the group consisting of: polypeptide antibiotics (e.g., actinomycin D or bleomycin) and anthraquinone drugs (e.g., adriamycin or mitoxantrone hydrochloride).

**20.** The antibody-drug conjugate of any one of claims 1 and 3-19, wherein the antibody-drug conjugate has the following structure:

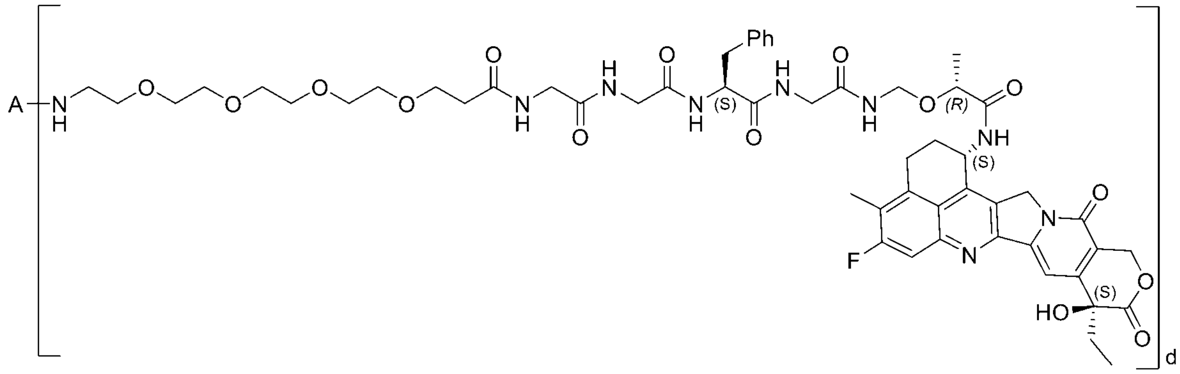

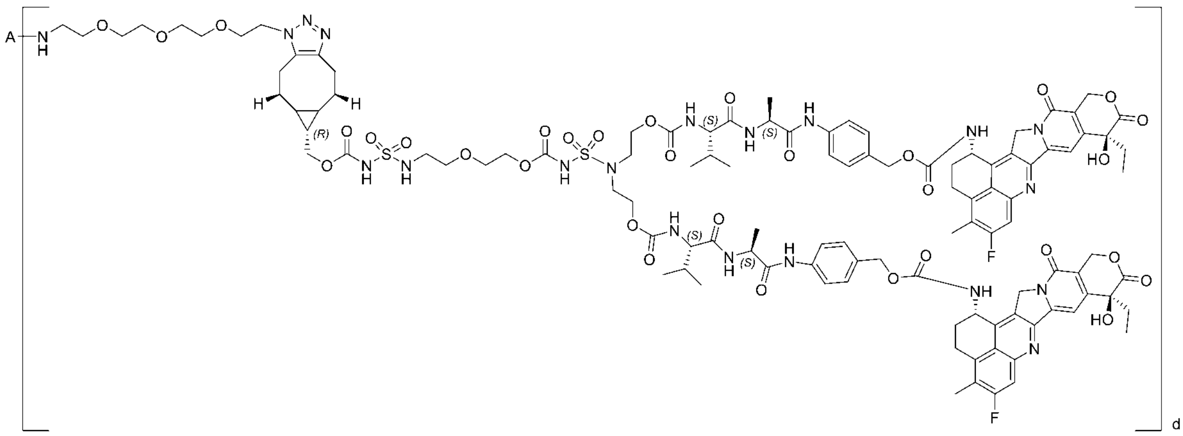

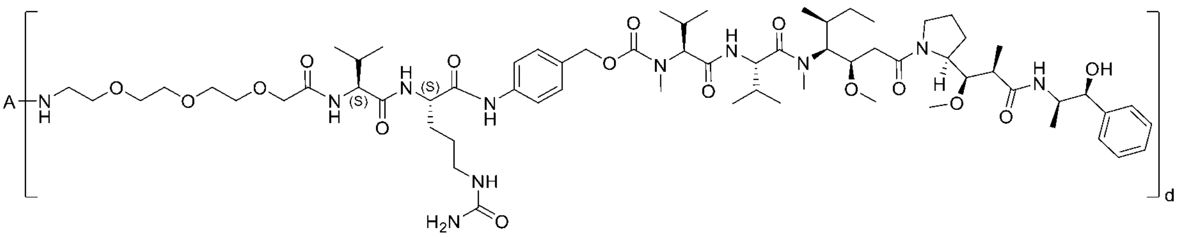

.

**21.** The antibody-drug conjugate or antibody of any one of claims 1-20, wherein the antibody is an anti-HER2 antibody, an anti-Nectin 4 antibody, or an anti-ROR1 antibody.

**22.** The antibody-drug conjugate or antibody of claim 21, wherein the anti-HER2 antibody comprises a heavy chain variable region and a light chain variable region, and wherein the heavy chain variable region comprises: a heavy chain CDR1 as shown in SEQ ID NO:1, a heavy chain CDR2 as shown in SEQ ID NO:2, and a heavy chain CDR3 as shown in SEQ ID NO:3; and the light chain variable region comprises: a light chain CDR1 as shown in SEQ ID NO:4, a light chain CDR2 as shown in SEQ ID NO:5, and a light chain CDR3 as shown in SEQ ID NO:6;

preferably, the anti-HER2 antibody comprises a heavy chain variable region as shown in SEQ ID NO:7, and/or comprises a light chain variable region as shown in SEQ ID NO:8;
more preferably, the anti-HER2 antibody comprises a heavy chain constant region and a light chain constant region, wherein the heavy chain constant region is subjected to reactive glutamine mutation based on the heavy chain constant region sequence shown in SEQ ID NO:9, and/or the light chain constant region is subjected to reactive glutamine mutation based on the light chain constant region sequence shown in SEQ ID NO:10;
more preferably, the anti-HER2 antibody is subjected to reactive glutamine mutation based on the light and heavy chain amino acid sequence of DP001 or trastuzumab.

**23.** The antibody-drug conjugate or antibody of claim 21, wherein the anti-Nectin 4 antibody comprises a heavy chain variable region and a light chain variable region, and wherein the heavy chain variable region comprises: a heavy

chain CDR1 as shown in SEQ ID NO:11, a heavy chain CDR2 as shown in SEQ ID NO:12, and a heavy chain CDR3 as shown in SEQ ID NO:13; and the light chain variable region comprises: a light chain CDR1 as shown in SEQ ID NO:14, a light chain CDR2 as shown in SEQ ID NO:15, and a light chain CDR3 as shown in SEQ ID NO:16;

preferably, the anti-Nectin 4 antibody comprises a heavy chain variable region as shown in SEQ ID NO: 17, and/or comprises a light chain variable region as shown in SEQ ID NO: 18;
more preferably, the anti-Nectin 4 antibody comprises a heavy chain constant region and a light chain constant region, wherein the heavy chain constant region is subjected to reactive glutamine mutation based on the heavy chain constant region sequence shown in SEQ ID NO:19, and/or the light chain constant region is subjected to reactive glutamine mutation based on the light chain constant region sequence shown in SEQ ID NO:20;
more preferably, the anti-Nectin4 antibody is subjected to reactive glutamine mutation based on the light and heavy chain amino acid sequences of Enfortumab.

**24.** The antibody-drug conjugate or antibody of claim 21, wherein the anti-ROR1 antibody comprises a heavy chain variable region and a light chain variable region, and wherein the heavy chain variable region comprises: a heavy chain CDR1 as shown in SEQ ID NO:21, a heavy chain CDR2 as shown in SEQ ID NO:22, SEQ ID NO:29 or SEQ ID NO:31, and a heavy chain CDR3 as shown in SEQ ID NO:23; and the light chain variable region comprises: a light chain CDR1 as shown in SEQ ID NO:24, a light chain CDR2 as shown in SEQ ID NO:25, and a light chain CDR3 as shown in SEQ ID NO:26;

preferably, the anti-ROR1 antibody comprises: a heavy chain variable region as shown in SEQ ID NO:27, SEQ ID NO:30 or SEQ ID NO:32, and/or a light chain variable region as shown in SEQ ID NO:28;
more preferably, the anti-ROR1 antibody comprises a heavy chain and a light chain, wherein the heavy chain comprises a heavy chain constant region sequence as shown in SEQ ID NO:33, and the light chain comprises a light chain constant region sequence as shown in SEQ ID NO:34.

**25.** The antibody-drug conjugate of any one of claims 1 and 3-24, wherein the drug molecule is DXD or a derivative thereof, or MMAE or a derivative thereof.

**26.** The antibody-drug conjugate of claim 25, wherein the linker is $NH_2-(CH_2CH_2O)_n$-Val-Cit-PABC-X, and X is a drug molecule; preferably, n is 3.

**27.** The antibody-drug conjugate of claim 25 or 26, wherein the drug conjugated moiety is JSSW-038 or LND1002.

**28.** The antibody-drug conjugate of claim 27, wherein the antibody-drug conjugate is AB5-ADC, MFC8-ADC, MFC10-ADC, DP28-ADC, DP35-ADC, DP36-ADC, DP35-JSSW038, DP36-JSSW038, MFC8-JSSW038, DP28-JSSW038, Hu17-H2L1-4LND1002, Hu17-H3L1-4LND1002, or Hu17-H4L1-4LND1002.

**29.** A method for preparing the antibody-drug conjugate or antibody of any one of claims 1-28, comprising a step of performing one or more insertion or substitution mutations in a constant region of the antibody to obtain a reactive glutamine.

**30.** The method for preparing the antibody-drug conjugate of any one of claims 1 and 3-29, further comprising a step of preparing the antibody and conjugating it to the drug conjugated moiety via mTGase enzymatic method to obtain the ADC.

**31.** A method for preparing an antibody-drug conjugate with high drug load, comprising firstly preparing the antibody-drug conjugate by the method of claim 29 or 30, then further conjugating it to the drug conjugated moiety by a chemical method to obtain an ADC with a higher DAR value; preferably, the chemical method is selected from one or more of click chemistry, amide condensation and Michael addition; more preferably, the chemical method is click chemistry achieved by an azide-cycloalkyne addition reaction.

**32.** The method of claim 31, wherein the drug conjugated moiety is JSSW-SY.

**33.** Use of the antibody-drug conjugate or antibody of any one of claims 1-32 in the preparation of a medicament for treating cancer.

**34.** The use of claim 33, wherein the cancer is a solid tumor or a hematoma.

**35.** The use of claim 33 or 34, wherein the cancer is selected from the group consisting of: HER2-positive cancers, such as breast cancer, colorectal cancer, ovarian cancer, gastric cancer, urethral cancer, and lung cancer (including small cell lung cancer and non-small cell lung cancer); Nectin 4-positive cancers, such as breast cancer, pancreatic cancer, lung cancer, ovarian cancer, and gastric cancer; or ROR1-positive cancers, such as chronic lymphocytic leukemia, mantle cell lymphoma, ovarian cancer, breast cancer, prostate cancer, lung cancer, melanoma, and colorectal cancer.

**36.** An acyl-donor substrate Q-containing tag for transglutaminase, wherein the Q-containing tag is selected from the group consisting of:

(i) GGLQSGA, LQSGA, EEQYGA and EEQYQSG, and the Q-containing tag is located at the carboxyl terminus of a light chain, heavy chain, or both the light chain and the heavy chain;
(ii) heavy chain 354L-355Q-356G (Q438), heavy chain 445Q-G446, heavy chain 117L-118Q-119G-120G, heavy chain 310L-Q311-312G, heavy chain 399L-400Q-401G, heavy chain 268L-269Q-270G, heavy chain 252Q-253G, heavy chain 444Q-445G-446A, heavy chain 283L-V284-285Q-286G, heavy chain 285L-286Q, heavy chain 340L-341L-Q342-343G, heavy chain 384L-385Q-386G-387G, heavy chain 268L-269Q-270G-271G, heavy chain 325L-326Q, heavy chain 329Q-330G, heavy chain 360L-361Q-362G, heavy chain 384Q-G385-386G, heavy chain 134L-135Q-136G, and heavy chain 282Q;
(iii) light chain 126L-127Q-G128-129G, light chain 205L-206Q-207G-208G, light chain 109L-110Q-111G-112G, and light chain 199L-200Q-201S-202G; and
(iv) a Q-containing tag LQSG that replaces light chain G200-L201-S202, and a Q-containing tag LQSG that inserts after heavy chain T135.

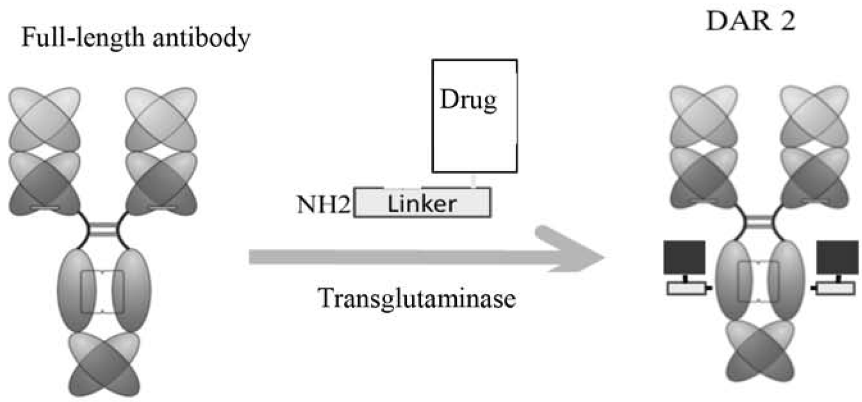


Figure 1

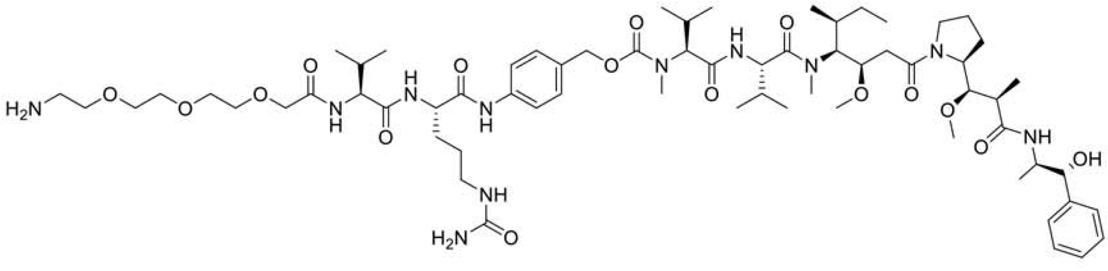

Figure 2A

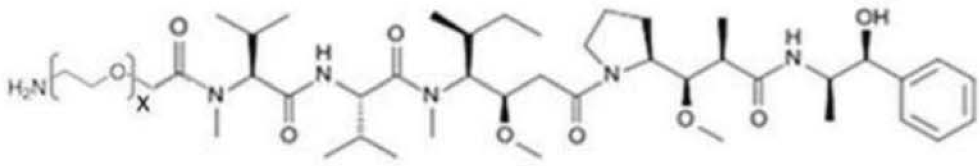

Figure 2B

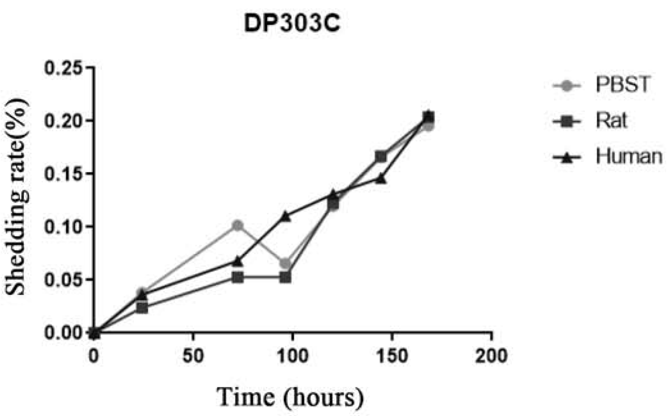


Figure 3A

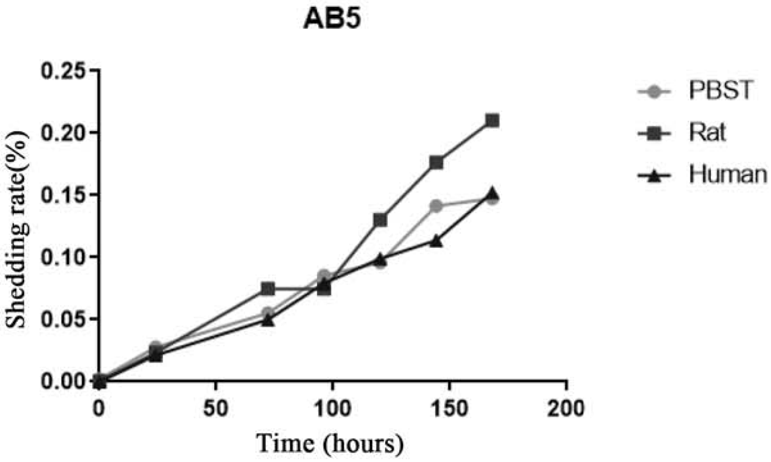


Figure 3B

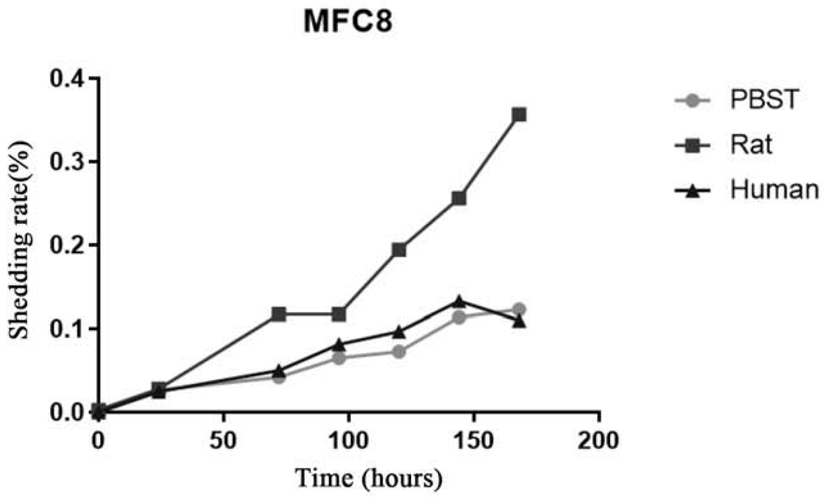


Figure 3C

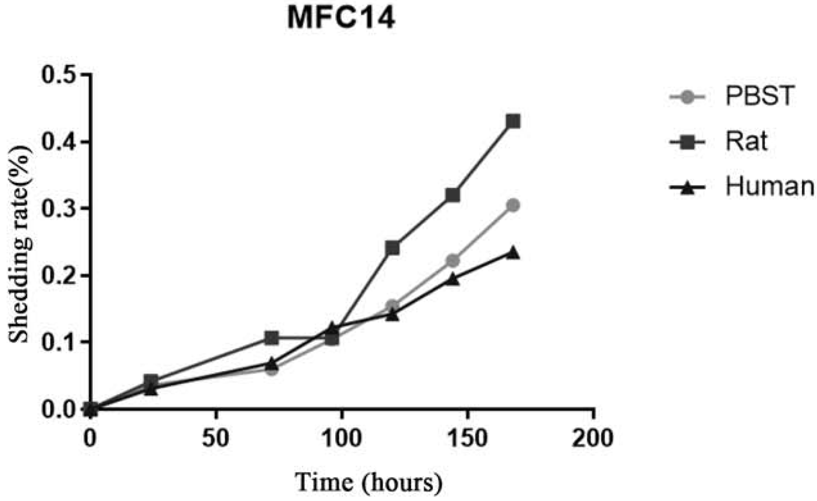


Figure 3D

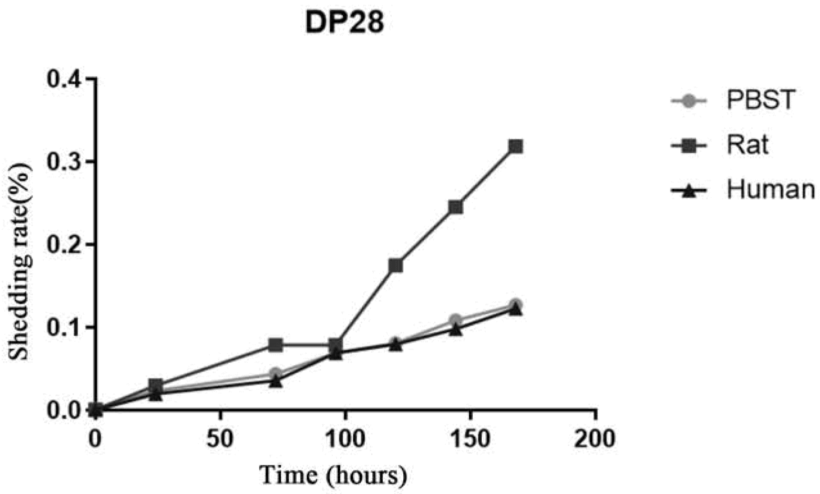


Figure 3E

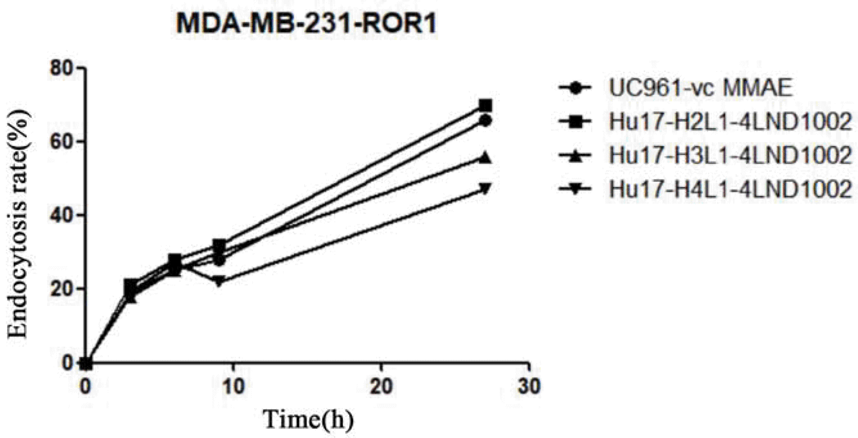


Figure 4

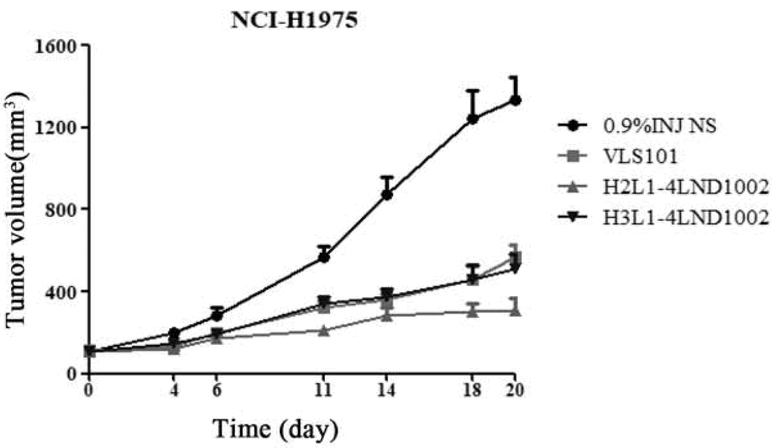


Figure 5

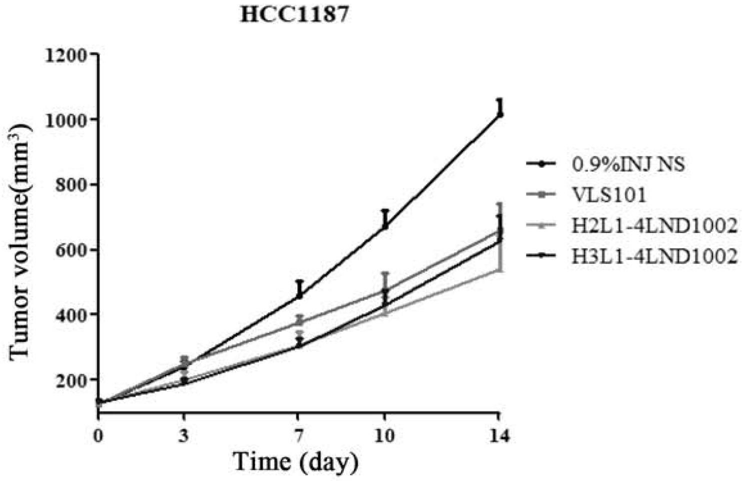


Figure 6

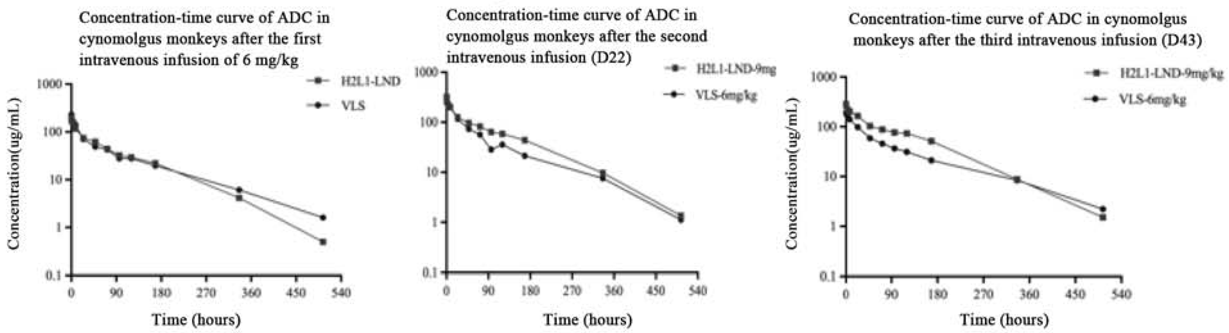


Figure 7

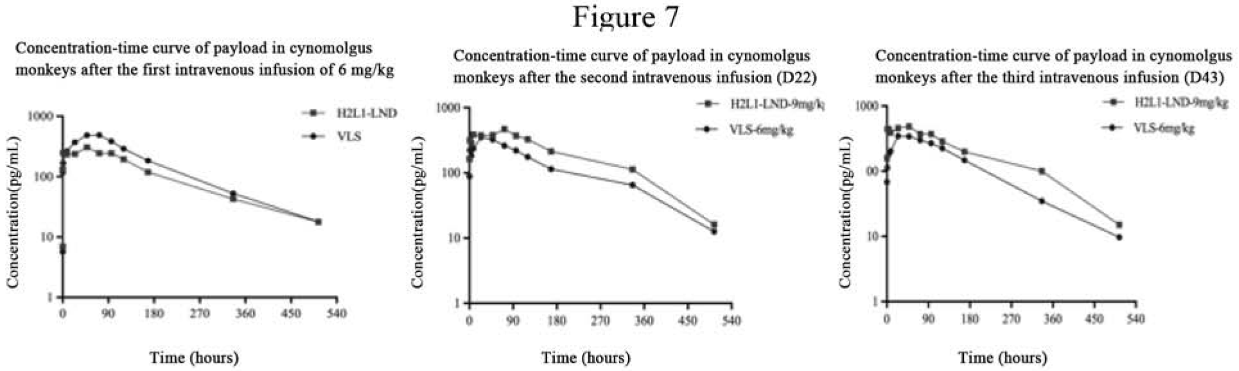


Figure 8

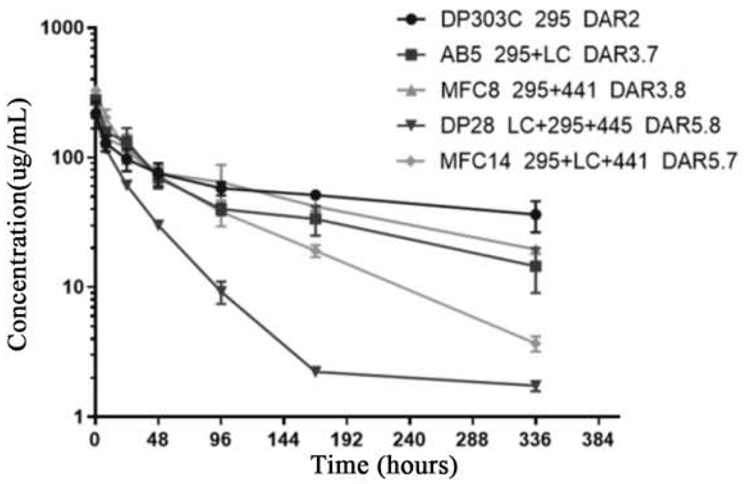


Figure 9

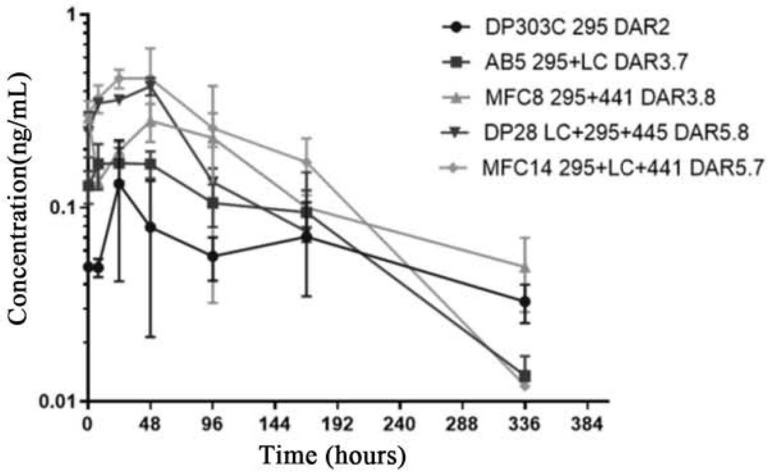


Figure 10

## INTERNATIONAL SEARCH REPORT

International application No.
**PCT/CN2024/108728**

**A. CLASSIFICATION OF SUBJECT MATTER**

A61K47/68(2017.01)i; A61K47/65(2017.01)i; C07K16/00(2006.01)i; C07K16/28(2006.01)i; C07K16/30(2006.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07K A61K A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANFANG, 读秀, DUXIU, pubmed, ISI_Web of Science, Science Direct, STNext: 谷氨酰胺, Glu, Q, 恒定区, LQSG, EEQY, LQG, Fc

**C. DOCUMENTS CONSIDERED TO BE RELEVANT**

| Category* | Citation of document, with indication, where appropriate, of the relevant passages | Relevant to claim No. |
|---|---|---|
| X | WO 2016030791 A1 (PFIZER INC. et al.) 03 March 2016 (2016-03-03)<br>description, page 34, paragraph 2-page 44, paragraph 6 | 1-36 |
| X | CN 113332449 A (CSPC DOPHEN CORP.) 03 September 2021 (2021-09-03)<br>claims 1-10 | 1-2, 13-36 |
| X | CN 114901308 A (CSPC JUSHI BIOLOGICAL PHARMACEUTICAL CO., LTD.) 12 August 2022 (2022-08-12)<br>claims 1-21 | 1-2, 13-35 |
| X | WO 2022179570 A1 (SHANGHAI ALLYGEN BIOLOGICS CO., LTD.) 01 September 2022 (2022-09-01)<br>description, paragraphs 267-278 | 1-36 |
| X | CN 106456798 A (RINAT NEUROSCIENCE CORP. et al.) 22 February 2017 (2017-02-22)<br>description, paragraphs 10, 19, 21-28, 49, 95, 119-159, and 187-188, and table 1 | 1-36 |
| X | CN 107207582 A (LONZA LTD.) 26 September 2017 (2017-09-26)<br>claims 1-57 | 1-36 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 October 2024** | **12 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/108728**

**C. DOCUMENTS CONSIDERED TO BE RELEVANT**

| Category* | Citation of document, with indication, where appropriate, of the relevant passages | Relevant to claim No. |
|---|---|---|
| A | CN 107922479 A (MERCK PATENT GMBH) 17 April 2018 (2018-04-17) description, paragraphs 24-40 | 1-36 |

Form PCT/ISA/210 (second sheet) (July 2022)

**INTERNATIONAL SEARCH REPORT**

International application No.
**PCT/CN2024/108728**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/108728**

| Patent document cited in search report | Publication date (day/month/year) | Patent family member(s) | Publication date (day/month/year) |
|---|---|---|---|
| WO 2016030791 A1 | 03 March 2016 | BR 112017002912 A2 | 30 January 2018 |
| | | JP 2016050204 A | 11 April 2016 |
| | | JP 6832621 B2 | 24 February 2021 |
| | | IL 250319 A0 | 30 March 2017 |
| | | IL 250319 B | 30 April 2020 |
| | | US 2021205463 A1 | 08 July 2021 |
| | | EP 3185908 A1 | 05 July 2017 |
| | | EP 3185908 B1 | 15 April 2020 |
| | | CA 2901684 A1 | 28 February 2016 |
| | | CA 2901684 C | 09 June 2020 |
| | | JP 2021073274 A | 13 May 2021 |
| | | US 2018140714 A1 | 24 May 2018 |
| | | US 10967068 B2 | 06 April 2021 |
| | | RU 2017104511 A3 | 28 September 2018 |
| | | RU 2017104511 A | 18 February 2019 |
| | | ES 2797754 T3 | 03 December 2020 |
| | | HUE 049325 T2 | 28 September 2020 |
| | | AU 2015308179 A1 | 02 February 2017 |
| | | AU 2015308179 B2 | 02 July 2020 |
| | | MX 2021008334 A | 05 August 2021 |
| | | SI 3185908 T1 | 31 August 2020 |
| | | MX 2017002600 A | 19 May 2017 |
| | | PT 3185908 T | 18 June 2020 |
| | | KR 20170036048 A | 31 March 2017 |
| | | KR 102051503 B1 | 03 December 2019 |
| | | PL 3185908 T3 | 07 September 2020 |
| | | DK 3185908 T3 | 15 June 2020 |
| CN 113332449 A | 03 September 2021 | WO 2015191883 A1 | 17 December 2015 |
| | | RU 2020122624 A | 03 August 2020 |
| | | EP 4082564 A1 | 02 November 2022 |
| | | KR 20170031127 A | 20 March 2017 |
| | | KR 102451080 B1 | 06 October 2022 |
| | | JP 2020105182 A | 09 July 2020 |
| | | JP 6955042 B2 | 27 October 2021 |
| | | US 2020022942 A1 | 23 January 2020 |
| | | US 11285128 B2 | 29 March 2022 |
| | | AU 2015274506 A1 | 02 February 2017 |
| | | AU 2015274506 B2 | 28 January 2021 |
| | | AU 2015274506 C1 | 13 October 2022 |
| | | RU 2016149433 A | 18 July 2018 |
| | | RU 2016149433 A3 | 28 February 2019 |
| | | RU 2728235 C2 | 28 July 2020 |
| | | US 2018360793 A1 | 20 December 2018 |
| | | US 10639291 B2 | 05 May 2020 |
| | | EP 3154574 A1 | 19 April 2017 |
| | | EP 3154574 A4 | 13 December 2017 |
| | | EP 3154574 B1 | 11 May 2022 |
| | | ES 2914093 T3 | 07 June 2022 |
| | | MY 177148 A | 08 September 2020 |
| | | JP 2017519045 A | 13 July 2017 |

Form PCT/ISA/210 (patent family annex) (July 2022)

**INTERNATIONAL SEARCH REPORT**
**Information on patent family members**

International application No.
**PCT/CN2024/108728**

| Patent document cited in search report | | | Publication date (day/month/year) | Patent family member(s) | | | Publication date (day/month/year) |
|---|---|---|---|---|---|---|---|
| | | | | JP | 6666336 | B2 | 13 March 2020 |
| | | | | US | 2017106096 | A1 | 20 April 2017 |
| | | | | US | 10357472 | B2 | 23 July 2019 |
| | | | | CA | 2951887 | A1 | 17 December 2015 |
| | | | | US | 2018360794 | A1 | 20 December 2018 |
| | | | | US | 10471037 | B2 | 12 November 2019 |
| CN | 114901308 | A | 12 August 2022 | WO | 2021086981 | A1 | 06 May 2021 |
| WO | 2022179570 | A1 | 01 September 2022 | KR | 20230152069 | A | 02 November 2023 |
| | | | | JP | 2024512217 | A | 19 March 2024 |
| | | | | IL | 305335 | A | 01 October 2023 |
| | | | | MX | 2023009746 | A | 11 September 2023 |
| | | | | EP | 4297798 | A1 | 03 January 2024 |
| | | | | CA | 3209753 | A1 | 01 September 2022 |
| | | | | AU | 2022227708 | A1 | 12 October 2023 |
| | | | | US | 2024299576 | A1 | 12 September 2024 |
| CN | 106456798 | A | 22 February 2017 | KR | 20210135364 | A | 12 November 2021 |
| | | | | KR | 102430829 | B1 | 09 August 2022 |
| | | | | ES | 2783376 | T3 | 17 September 2020 |
| | | | | SI | 3134127 | T1 | 30 June 2020 |
| | | | | HUE | 049622 | T2 | 28 September 2020 |
| | | | | WO | 2015162563 | A1 | 29 October 2015 |
| | | | | JP | 2015209426 | A | 24 November 2015 |
| | | | | JP | 6837273 | B2 | 03 March 2021 |
| | | | | KR | 20160145790 | A | 20 December 2016 |
| | | | | PL | 3134127 | T3 | 27 July 2020 |
| | | | | PT | 3134127 | T | 09 April 2020 |
| | | | | DK | 3134127 | T3 | 16 March 2020 |
| | | | | MX | 2016013970 | A | 06 April 2017 |
| | | | | IL | 248221 | A0 | 30 November 2016 |
| | | | | IL | 248221 | B | 01 March 2022 |
| | | | | RU | 2016139340 | A3 | 25 May 2018 |
| | | | | RU | 2016139340 | A | 14 December 2018 |
| | | | | JP | 2021091692 | A | 17 June 2021 |
| | | | | JP | 7194207 | B2 | 21 December 2022 |
| | | | | US | 2017043033 | A1 | 16 February 2017 |
| | | | | US | 11602525 | B2 | 14 March 2023 |
| | | | | KR | 20190072663 | A | 25 June 2019 |
| | | | | BR | 112016024370 | A2 | 10 October 2017 |
| | | | | CA | 2946488 | A1 | 29 October 2015 |
| | | | | CA | 2946488 | C | 09 November 2021 |
| | | | | AU | 2015249452 | A1 | 27 October 2016 |
| | | | | AU | 2015249452 | B2 | 26 October 2017 |
| | | | | EP | 3134127 | A1 | 01 March 2017 |
| | | | | EP | 3134127 | B1 | 26 February 2020 |
| CN | 107207582 | A | 26 September 2017 | EP | 3878861 | A1 | 15 September 2021 |
| | | | | JP | 2021175762 | A | 04 November 2021 |
| | | | | JP | 7439024 | B2 | 27 February 2024 |
| | | | | WO | 2016131769 | A2 | 25 August 2016 |
| | | | | WO | 2016131769 | A3 | 22 December 2016 |
| | | | | KR | 20240091253 | A | 21 June 2024 |

Form PCT/ISA/210 (patent family annex) (July 2022)

**INTERNATIONAL SEARCH REPORT**
**Information on patent family members**

International application No.
**PCT/CN2024/108728**

| Patent document cited in search report | Publication date (day/month/year) | Patent family member(s) | Publication date (day/month/year) |
|---|---|---|---|
| | | SG 10202006863 YA | 28 August 2020 |
| | | SG 11201706292 SA | 28 September 2017 |
| | | US 2021100914 A1 | 08 April 2021 |
| | | US 11833222 B2 | 05 December 2023 |
| | | JP 2018509933 A | 12 April 2018 |
| | | JP 6932649 B2 | 08 September 2021 |
| | | US 2018154017 A1 | 07 June 2018 |
| | | US 10953106 B2 | 23 March 2021 |
| | | KR 20170116144 A | 18 October 2017 |
| | | KR 102672113 B1 | 03 June 2024 |
| | | EP 3259285 A2 | 27 December 2017 |
| CN 107922479 A | 17 April 2018 | AU 2016306907 A1 | 29 March 2018 |
| | | AU 2016306907 A9 | 25 July 2019 |
| | | US 2019194344 A1 | 27 June 2019 |
| | | US 11130818 B2 | 28 September 2021 |
| | | EP 3331904 A1 | 13 June 2018 |
| | | EP 3331904 B1 | 23 December 2020 |
| | | ES 2855998 T3 | 27 September 2021 |
| | | JP 2018529321 A | 11 October 2018 |
| | | JP 7008014 B2 | 10 February 2022 |
| | | CA 2994741 A1 | 16 February 2017 |
| | | IL 256810 A | 29 March 2018 |
| | | IL 256810 B | 31 October 2021 |
| | | WO 2017025179 A1 | 16 February 2017 |
| | | WO 2017025179 A8 | 19 April 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310943337
- CN 2024108728 W
- CN 114901308 A
- EP 404097 A
- WO 9311161 A
- CN 111587124 A
- US 5663149 A


### Non-patent literature cited in the description


- Sequences of Proteins of Immunological Interest. **KABAT et al.** Public Health Service. National Institutes of Health, 1991
- **KABAT et al.** *J. Biol. Chem.*, 1977, vol. 252, 6609-6616
- **KABAT et al.** Sequences of proteins of immunological interest. *U.S. Dept. of Health and Human Services*, 1991
- **CHOTHIA et al.** *J. Mol. Biol.*, 1987, vol. 196, 901-917
- **AL-LAZIKANI B. et al.** *J. Mol. Biol.*, 1997, vol. 273, 927-948
- **MACCALLUM et al.** *J. Mol. Biol.*, 1996, vol. 262, 732-745
- **ABHINANDAN** ; **MARTIN**. *Mol. Immunol*, 2008, vol. 45, 3832-3839
- **LEFRANC M. P. et al.** *Dev. Comp. Immunol.*, 2003, vol. 27, 55-77
- **HONEGGER** ; **PLÜCKTHUN**. *J. Mol. Biol.*, 2001, vol. 309, 657-670
- **ABHINANDAN** ; **MARTIN**. *Mol. Immunol.*, 2008, vol. 45, 3832-3839
- **EHRENMANN F. et al.** *Nucleic Acids Res.*, 2010, vol. 38, D301-D307
- **ADOLF-BRYFOGLE J et al.** *Nucleic Acids Res.*, 2015, vol. 43, D432-D438
- **WARD et al.** *Nature*, 1989, vol. 341, 544-546
- **BIRD et al.** *Science*, 1988, vol. 242, 423-426
- **HUSTON et al.** *Proc. Natl. Acad. Sci.*, 1988, vol. 85, 5879-5883
- **HOLLINGER et al.** *Proceedings of the National Academy of Sciences*, 1993, vol. 90, 6444-6448
- **KABAT EA** ; **WU TT**. *Ann NY Acad Sci*, 1971, vol. 190, 382-391
- U.S. Department of Health and Human Services. **KABAT EA et al.** Sequences of Proteins of Immunological Interest. 1991, 3242
- **LEFRANC M-P**. *The Immunologist*, 1999, vol. 7, 132-136
- **LEFRANC M-P et al.** *Nucleic Acids Res*, 1999, vol. 27, 209-212
- **PETTET et al.** *Antimicrob. Agents Chemother.*, 1998, vol. 42, 2961-2965
- **BOGER** ; **JOHNSON**. *PNAS*, 1995, vol. 92, 3642-3649
- **REMILLARD et al.** *Science*, 1975, vol. 189, 1002-1005
- *CHEMICAL ABSTRACTS*, 646502-53-6